# EUROPÄISCHE PATENTANMELDUNG

(11) **EP 2 206 534 A1**
(43) Veröffentlichungstag der Anmeldung: **14.07.2010**
(21) Anmeldenummer: 08166223.1
(22) Anmeldetag: 09.10.2008
(51) Int. Cl.: A61P 35/00, A61P 37/02, A61P 37/06, A61P 37/08, A61P 43/00, A61P 7/00, A61P 9/04, A61P 9/10, A61K 31/41, A61K 31/35, A61K 31/5375, A61K 31/55, A61K 31/222, A61K 31/136, A61K 31/122

(54) **Dibenzocycloheptanonderivate und pharmazeutische Mittel, welche diese Verbindungen enthalten**

(71) Anmelder: c-a-i-r biosciences GmbH, 72076 Tübingen (DE)
(72) Erfinder: Die Erfindernennung liegt noch nicht vor
(74) Vertreter: Reitstötter - Kinzebach

(57) **Zusammenfassung**

Die vorliegende Erfindung betrifft Verbindungen der Formel I 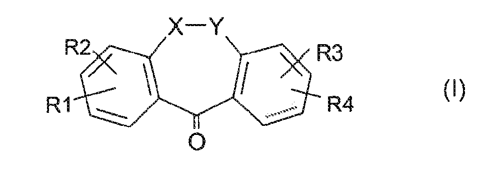 worin R1, R2, R3, R4, X und Y die in der Beschreibung angegebenen Bedeutungen besitzen. Die Verbindungen besitzen immunmodulierende und die Freisetzung von IL-1β und/oder TNF-α inhibierende bzw. regulierende Wirkung. Sie sind daher zur Behandlung von Erkrankungen brauchbar, die im Zusammenhang mit einer Störung des Immunsystems stehen.

## Beschreibung

Die vorliegende Erfindung betrifft Verbindungen der Formel I 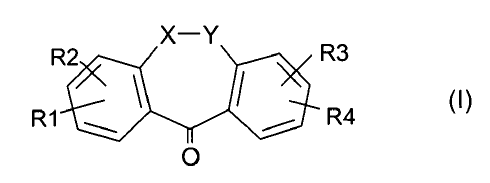 worin X, Y und R1 bis R4 die unten angegebenen Bedeutungen besitzen, sowie pharmazeutische Mittel, welche die Verbindungen der Formel I enthalten. Bei den Verbindungen handelt es sich um Interleukin-1β (IL-1β)- und Tumor-Nekrose-Faktor-α (TNF-α)-Inhibitoren, die zur Behandlung von inflammatorischen Erkrankungen brauchbar sind.

IL-1β und TNF-α schützen den Körper vor infektiösen Agenzien, Tumoren oder Gewebeschädigung. Bei Autoimmunerkrankungen kommt es jedoch zu einer erhöhten Produktion von IL-1β und TNF-α, was beispielsweise Knochen- und Knorpelabbau zur Folge haben kann. Arzneimittel, welche die Freisetzung von IL-1β und TNF-α regulieren, sind daher zur Behandlung von inflammatorischen Erkrankungen brauchbar.

Eine Gruppe von Verbindungen, welche die Freisetzung von IL-1β und TNF-α inhibieren, sind aus der WO 98/32730 bekannt. Sie entsprechen der allgemeinen Formel 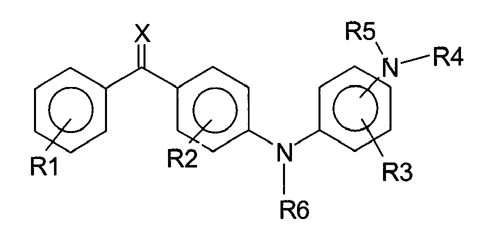 und sind zur Behandlung und Prophylaxe von Asthma, Allergien, rheumatoider Arthritis, Spondyloarthritis, Gicht, Atherosklerose, chronische entzündliche Darmerkrankung (inflammatory bowel disease), proliferative und inflammatorische Hauterkrankungen, wie Psoriasis und atopische Dermatitis brauchbar.

Weitere Verbindungen dieser Gruppe sind beschrieben in WO 01/05744, WO 01/05745, WO 01/05746, WO 01/05749, WO 01/05751, WO 01/42189, WO 02/45752, WO 02/076447 und WO 03/018535 sowie in J. Med. Chem. 2003, 46, 5651-5662 und Bioorganic & Medicinal Chemistry Letters 14 (2004) 3601-3605.

Die WO 2006/120010 beschreibt Dibenzocycloheptanonderivate der Formel I 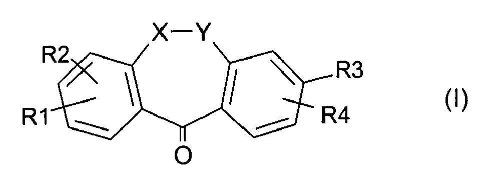 worin eines der Ringatome X und Y für CH₂ und das andere für O, S, SO, SO₂ oder NR5 steht; oder -X-Y- für -CH₂-CH₂- oder -CH=CH- steht; R1 für H oder C₁-C₆-Alkyl steht; und R2 für H, Halogen oder C₁-C₄-Alkyl-C≡C-, das ggf. mit einer Aminogruppe substituiert ist, steht. R3 steht für Halogen oder einen gegebenenfalls substituierten Aminorest. Die Verbindungen sind Interleukin-1β (IL-1β)- und Tumor-Nekrose-Faktor-α (TNF-α)-Inhibitoren, die zur Behandlung von inflammatorischen Erkrankungen brauchbar sind.

Die Wirkung der Verbindungen ist jedoch nicht zufriedenstellend.

Der vorliegenden Erfindung liegt daher die Aufgabe zugrunde, anti-inflammatorisch wirkende Verbindungen zur Verfügung zu stellen, die verbesserte Wirkung besitzen.

Diese Aufgabe wird durch die Verbindungen der Formel I gelöst. Die Erfindung betrifft somit Verbindungen der Formel I 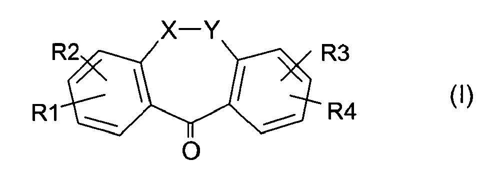 worin
eines der Ringatome X und Y für CH₂ und das andere für O, S, SO, SO₂ oder NR5 steht; oder -X-Y- für -CH₂-CH₂- oder -CH=CH- steht;
- R1: ausgewählt ist unter:
a) Hydroxy;
b) C₁-C₆-Alkoxy;
c) C₁-C₆-Alkoxy, das mit 1, 2 oder 3 Hydroxy- oder C₁-C₆-Alkoxygruppen substituiert ist;
d) NR6R7;
e) C₁-C₆-Alkoxy, das substituiert ist mit einem gesättigten oder ungesättigten, aromatischen oder nicht-aromatischen Heterocyclus mit 5 oder 6 Ringatomen, der 1, 2 oder 3 Heteroatome aufweist, die unabhängig voneinander ausgewählt sind unter O, N und S, wobei der Heterocyclus gegebenenfalls 1 oder 2 Hydroxy-, C₁-C₆-Alkoxy- oder C₁-C₆-Alkylsubstituenten aufweisen und mit einem Phenylring oder einem gesättigten oder ungesättigtenCarbocyclus mit 5 oder 6 Ringatomen kondensiert sein kann; und
f) Tetrazolo;
g) NR8CONR13R14;
g) CF₃SO₂O-;
h) OR16;
i) C₁-C₆-Alkylcarbonyloxy-C₁-C₆-alkoxy; und
j) NO₂;
- R2: für H oder C₁-C₆-Alkyl steht;
- R3: ausgewählt ist unter:
a) -NR6R7;
b) 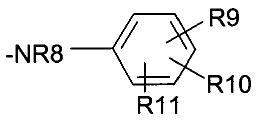
c) 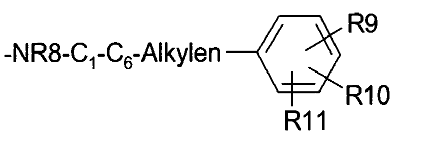
d) 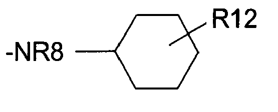
e) 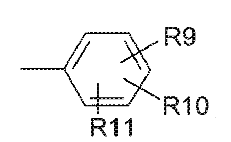
f) -NH-C₁-C₆-Alkylen-NR6R7
g) Tetrazolo;
h) Halogen;
i) OR15; und
j) NO₂; und
k) NR8COC₁-C₆-Alkyl;
- R4: für H, Halogen oder C₁-C₆-Alkyl steht;
- R5: für H oder C₁-C₆-Alkyl, das mit 1, 2 oder 3 Hydroxy- oder C₁-C₆-Alkoxygruppen substituiert ist, steht;
- R6 und R7,: die gleich oder verschieden sein können, für H oder C₁-C₆-Alkyl, das mit 1, 2 oder 3 Hydroxy- oder C₁-C₆-Alkoxygruppen substituiert ist, stehen;
- R8: für H oder C₁-C₆-Alkyl steht;
- R9, R10 und R11,: die gleich oder verschieden sein können, ausgewählt sind unter:
a) H,
b) NH₂,
c) mono-C₁-C₆-Alkylamino,
d) di-C₁-C₆-Alkylamino,
e) C₁-C₆-Alkyl,
f) C₁-C₆-Alkoxy,
g) Hydroxy,
h) Halogen,
i) C₁-C₆-Alkyl, das mit 1, 2 oder 3 Halogenatomen substituiert ist;
j) CONR6R7; und
k) NO₂;
- R12: für H oder NH₂ steht;
R13 und R14, die gleich oder verschieden sein können, für H oder C₁-C₆-Alkyl stehen oder zusammen mit dem Stickstoffatom an das sie gebunden sind, für einen nicht-aromatischen Heterocyclus mit 5 oder 6 Ringatomen, der 1 oder 2 Heteroatome aufweist, die unabhängig voneinander ausgewählt sind unter O und N;
- R15: für H oder C₁-C₆-Alkyl, das mit 1, 2 oder 3 Hydroxy- oder C₁-C₆-Alkoxygruppen substituiert ist, steht;
- R16: für einen nicht-aromatischen Heterocyclus mit 5 oder 6 Ringatomen, der 1 oder 2 Heteroatome aufweist, die unabhängig voneinander ausgewählt sind unter O und N;
und die optischen Isomeren, physiologisch verträglichen Salze und Solvate davon.

Der Ausdruck "Alkyl" (auch in Verbindung mit anderen Gruppen, wie Alkoxy, Halogenalkyl etc.) umfasst geradkettige und verzweigte Alkylgruppen mit vorzugsweise 1 bis 6 bzw. 1 bis 4 Kohlenstoffatomen, wie Methyl, Ethyl, n- und i-Propyl, n-, i- und t-Butyl, sec-Butyl, n-Pentyl und n-Hexyl.

Der Ausdruck "Halogen" steht für ein Fluor-, Chlor-, Brom- oder Jodatom, insbesondere für ein Fluor- oder Chloratom.

Bei C₁-C₆-Alkoxy, das mit 1, 2 oder 3 Hydroxy- oder C₁-C₆-Alkoxygruppen substituiert ist, handelt es sich insbesondere um 2-Hydroxyethoxy, 3-Hydroxypropoxy, 2-Hydroxypropoxy, 1,2-Dihydroxyethoxy, 2,3-Dihydroxypropoxy oder 2,3-Dimethoxypropoxy.

Bei einem gesättigten nicht-aromatischen Heterocyclus handelt es sich insbesondere um Pyrrolidinyl, Piperidinyl, Hydroxypiperidinyl, Piperazinyl, Tetrahydropyranyl, Tetrahydrofuranyl, Dioxolanyl, 2,2-Dimethyldioxolanyl, Dioxanyl, Morpholinyl oder Thiomorpholinyl. Der Piperidinylrest kann durch 1, 2, 3 oder 4 C₁-C₄-Alkylgruppen, insbesondere Methylgruppen substituiert sein. Ein bevorzugter Piperidinylrest ist 2,2,6,6-Tetramethylpiperidinyl. Die Stickstoffheterocyclen können über ein Stickstoffatom oder ein Kohlenstoffatom gebunden sein.

Bei einem ungesättigten nicht-aromatischen Heterocyclus handelt es sich insbesondere um Pyrrolinyl, Di- oder Tetrahydropyridinyl.

Bei einem aromatischen Heterocyclus handelt es sich insbesondere um Pyridyl, bevorzugt 3- oder 4-Pyridyl, Pyrimidinyl, Pyrrolyl, Imidazolyl, Pyrazolyl, Oxazolyl, Isoxazolyl, Furyl, Thienyl, Thiazolyl, Thiadiazolyl, Isothiazolyl oder die entsprechenden Benzoderivate davon.

R1 steht vorzugsweise für die oben genannten Bedeutungen a), b), c) und d). Gemäß einer Ausführungsform steht R1 steht in Position 7. Gemäß einer weiteren Ausführungsform steht R1 in Position 8. Gemäß einer weiteren Ausführungsform steht R1 in Position 9.

R2 steht vorzugsweise für H.

Der Rest R3 steht vorzugsweise in Position 3 (bezogen auf Y). Gemäß einer Ausführungsform steht R3 für die oben genannten Bedeutungen a), b), e), g), h), j) und k). R3 steht gemäß einer weiteren Ausführungsform für die oben genannte Formel (b). Gemäß einer weiteren Ausführungsform steht R3 für 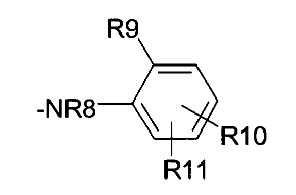 wobei R8, R9, R10 und R11 die oben angegebenen Bedeutungen besitzen. R10 und R11 stehen vorzugsweise in 3- und 5-Position.

Gemäß einer weiteren Ausführungsform steht R3 für 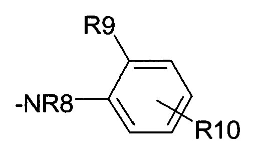 worin R8, R9 und R10 die oben angegebenen Bedeutungen besitzen. R10 steht vorzugsweise in 4-Position.

R4, R5, R6, R7 und R8 stehen vorzugsweise für H.

R9, R10 und R11 stehen vorzugsweise für die oben genannten Bedeutungen a), b), e), f),g) und h) und insbesondere für H, NH₂, C₁-C₆-Alkoxy oder Halogen.

R12 steht vorzugsweise für H.

R16 steht vorzugsweise für einen nicht-aromatischen Heterocyclus mit 5 oder 6 Ringatomen, der 1 oder 2 Sauerstoffheteroatome aufweist, und insbesondere für Tetrahydropyranyloxy oder 2,2-Dimethyldioxolanyl.

Eine Ausführungsform der Erfindung sind die Verbindungen der Formel Ia: 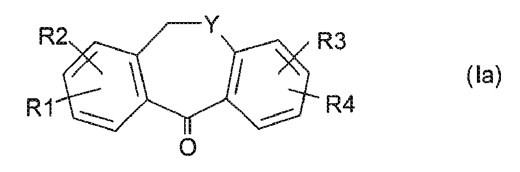 worin Y für O oder S steht und R1, R2, R3 und R4 die oben angegebenen Bedeutungen besitzen.

Eine weitere Ausführungsform sind die Verbindungen der Formel Iaa 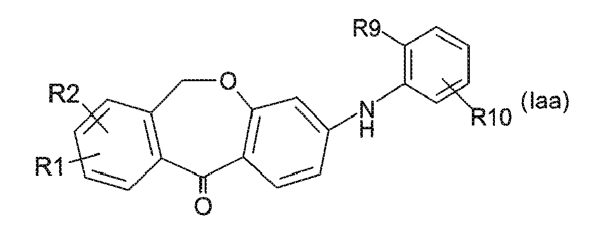 worin Y für O oder S steht und R1, R2, R9 und R10 die oben angegebenen Bedeutungen besitzen.

Eine weitere Ausführungsform der Erfindung sind die Verbindungen der Formel Ib 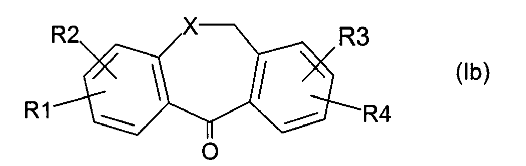 worin X für O oder S steht und R1, R2, R3 und R4 die oben angegebenen Bedeutungen besitzen.

Eine weitere Ausführungsform sind die Verbindungen der Formel Ic 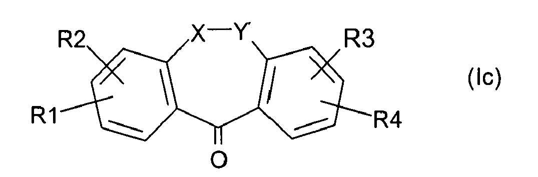 worin -X-Y- für -CH₂-CH₂- oder -CH=CH- steht und R1, R2, R3 und R4 die oben angegebenen Bedeutungen besitzen.

Eine weitere Ausführungsform sind die Verbindungen der Formel Ica: 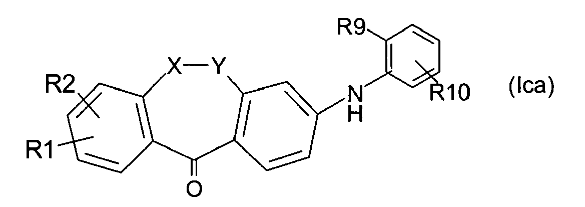 worin -X-Y- für -CH₂-CH₂- oder -CH=CH- steht und R1, R2, R9 und R10 die oben angegebenen Bedeutungen besitzen.

Die Erfindung umfasst auch die physiologisch verträglichen Salze der Verbindungen der Formel I. Im vorliegenden Fall handelt es sich insbesondere um die Säureadditionssalze. Zur Bildung der Säureadditionssalze werden anorganische Säuren, wie Salzsäure, Schwefelsäure oder Phosphorsäure oder organische Säuren, wie Weinsäure, Zitronensäure, Maleinsäure, Fumarsäure, Äpfelsäure, Mandelsäure, Ascorbinsäure, Gluconsäure, Methansulfonsäure, Benzolsulfonsäure oder Toluensulfonsäure und dergleichen, eingesetzt.

Soweit die erfindungsgemäßen Verbindungen Asymmetriezentren aufweisen, sind die Racemate sowie die einzelnen optischen Isomere (Enantiomere, Diastereomere) ebenfalls Gegenstand der Erfindung.
Gegenstand der Erfindung sind auch die Solvate der Verbindungen der Formel I oder der Salze davon, insbesondere die Hydrate.

Die Herstellung der Verbindungen der Formel I , die an der Position 7 substituiert sind, wobei X-Y für CH₂-CH₂ oder CH = CH steht, erfolgt nach den in den Schemata I bis III beispielhaft erläuterten Verfahren.
Schema I: Synthese des Grundgerüsts mit Substitution in Position 7

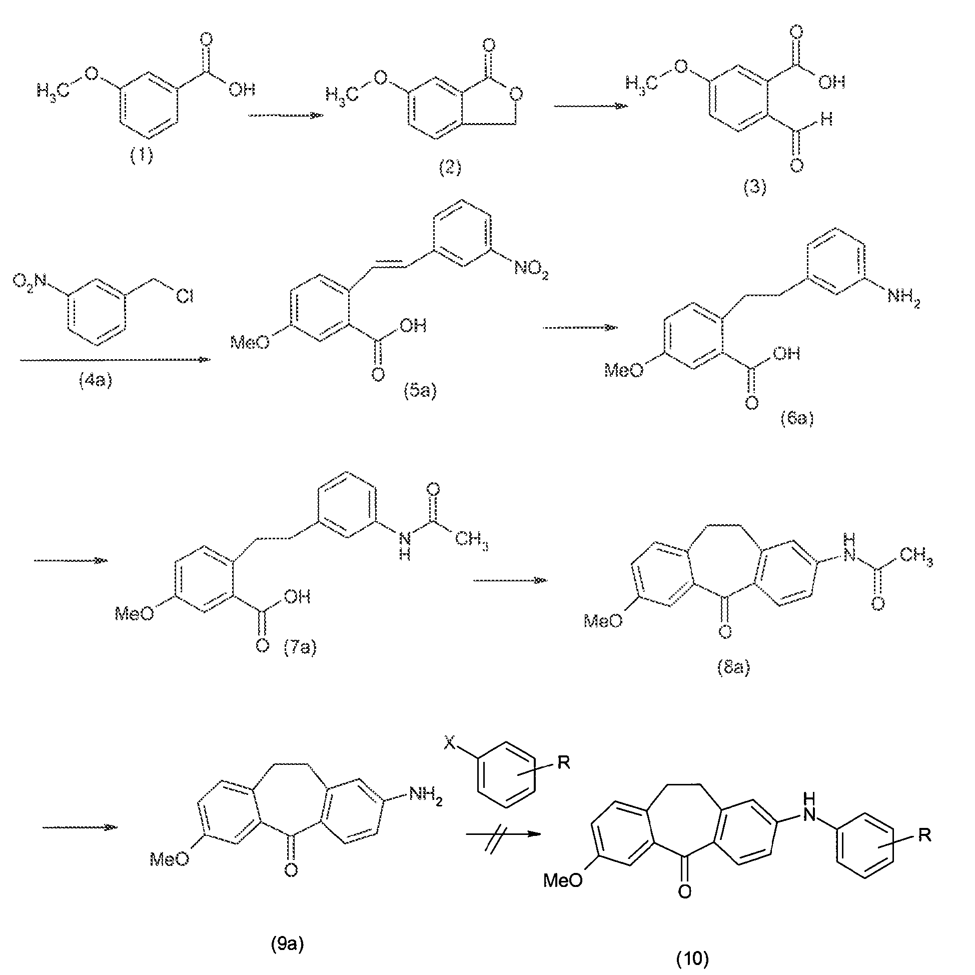

Ausgehend von 3-Methoxybenzoesäure (1) wird über das Phthalid (2) eine Formylgruppe eingeführt. Mittels einer Wittig-Reaktion erhält man die ungesättigte Verbindung (5a), die in üblicher Weise, beispielsweise unter Verwendung eines Edelmetallkatalysators, wie Pt oder Pd, hydriert werden kann. Vor dem Ringschluss durch Friedel-Crafts-Acylierung wird die Aminogruppe durch eine geeignete Schutzgruppe geschützt, die nach der Friedel-Crafts-Acylierung entfernt wird. Nach Umsetzung mit einem Arylhalogenid erhält man Verbindung (10) zu erhalten.

Schema II zeigt ein alternatives Verfahren zu Herstellung von (10), mit dem sich die Verwendung der Schutzgruppe vermeiden lässt:

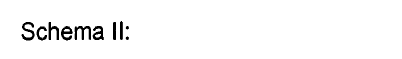

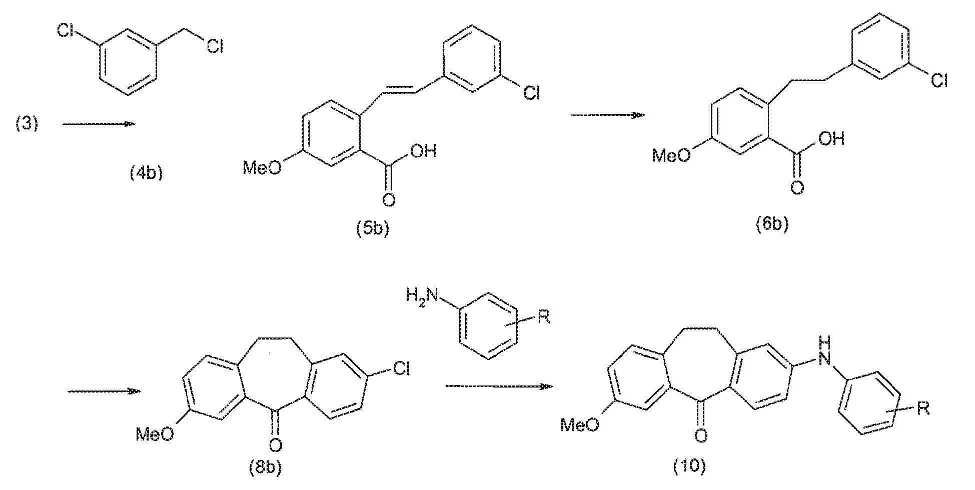

Die an der Position 7 methoxy-substituierten Verbindungen (10) lassen sich durch Etherspaltung, etwa mittels wässriger HBr, in die Hydroxyverbindungen (11) überführen, siehe Schema III.

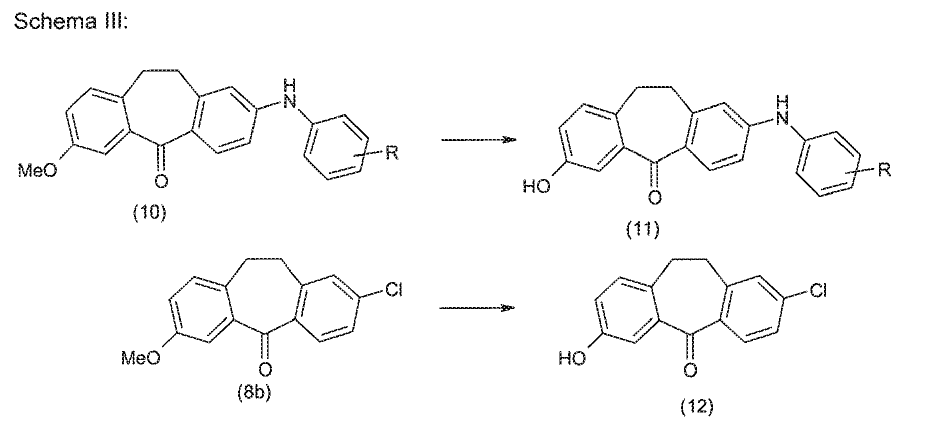

Die Hydroxygruppe an Position 7 erlaubt weitere Substitutionen, etwa über die Ethersynthese nach Williams oder die Mitsunobu-Reaktion, siehe Schema IV:

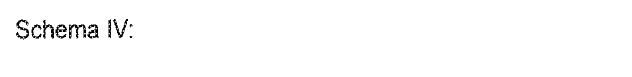

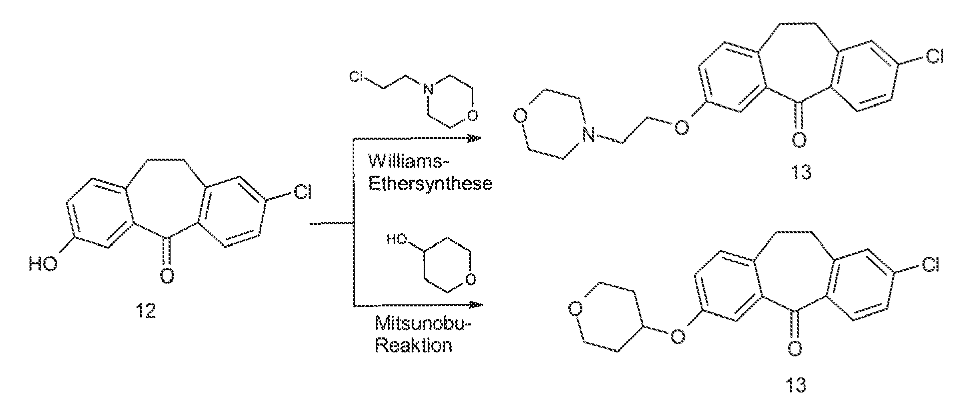

Die Herstellung der Verbindungen der Formel I , die an der Position 8 oder 9 substituiert sind, wobei X-Y für CH₂-CH₂ oder CH = CH steht, erfolgt nach den in den Schemata V bis X beispielhaft erläuterten Verfahren.

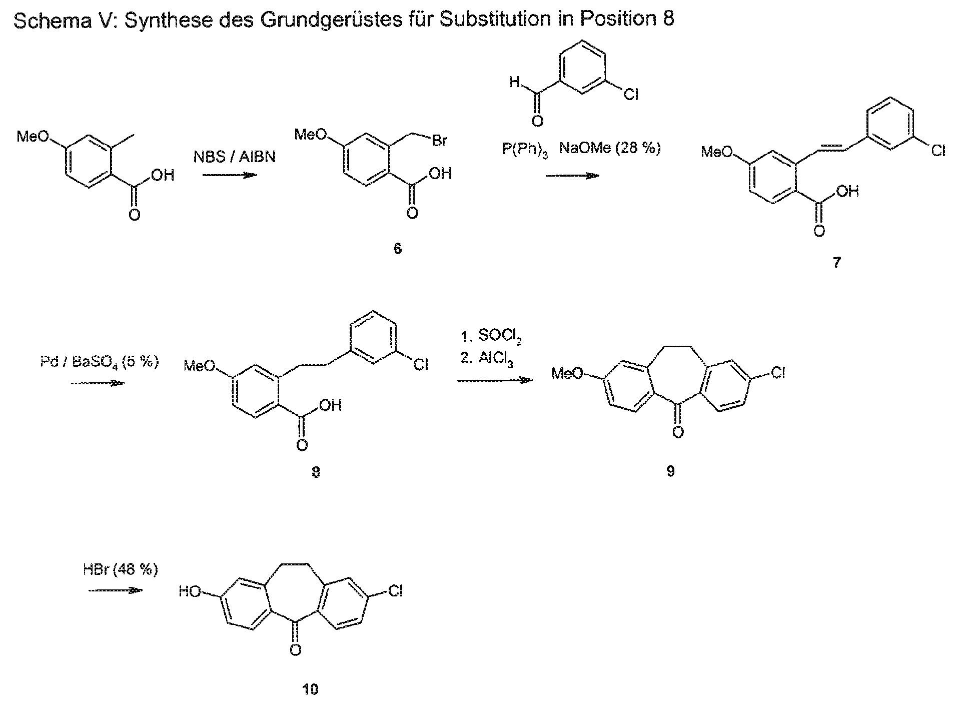

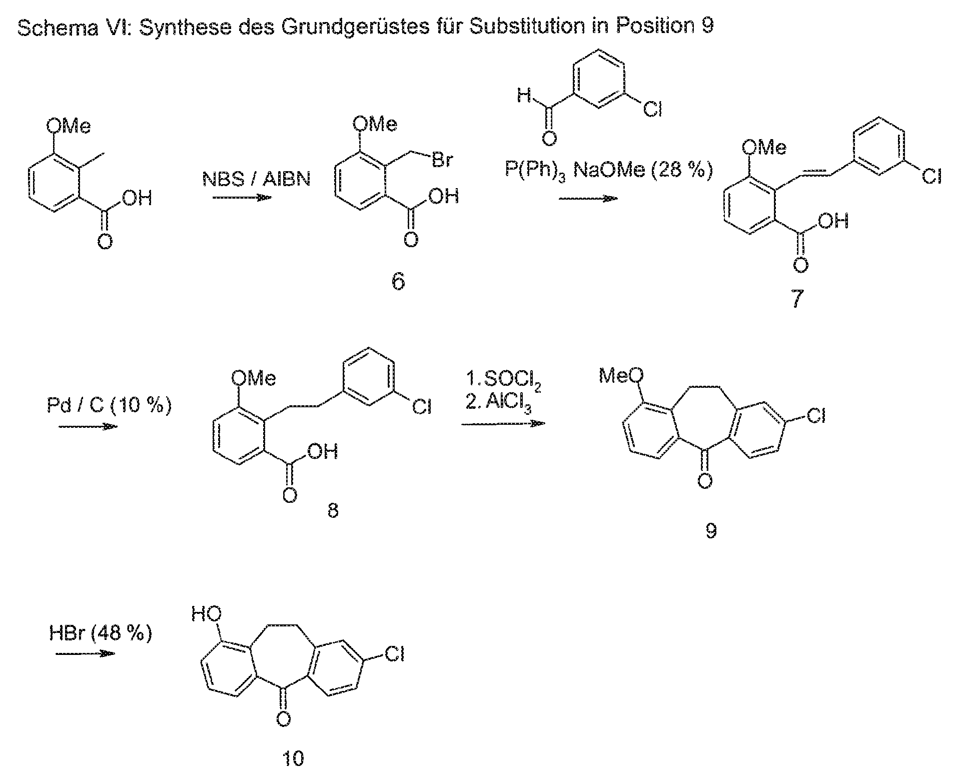

Ausgehend von Methoxymethylbenzoesäure erfolgt zunächst eine Bromierung mit N-Bromsuccinimid (NBS). Die entstandene Brommethylmethoxybenzoesäure (6) wird in einer Wittig-Reaktion mit 3-Chlorbenzaldehyd zu Chlorphenylvinylmethoxybenzoesäure (7) umgesetzt. Die nachfolgende Hydrierung der Chlorphenylvinylmethoxy-benzoesäure erfolgt in üblicher Weise unter Verwendung von Edelmetalkatalysatoren, wie Pd/BaSO₄ (Substitution in Position 8) bzw. Pd/C (Substitution in Position 9). Die Carbonsäure (8) wird in das Säurechlorid überführt, zum Beispiel durch Umsetzung mit Thionylchlorid. Der anschließende Ringschluss zu (9) erfolgt durch Friedel-Crafts-Acylierung mit geeigneten Lewis-Säuren, wie AlCl₃. Verbindung (10) wird dann durch Etherspaltung, beispielsweise mit wässrigem Bromwasserstoff, erhalten.

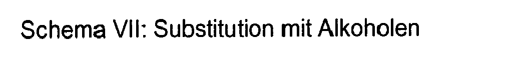

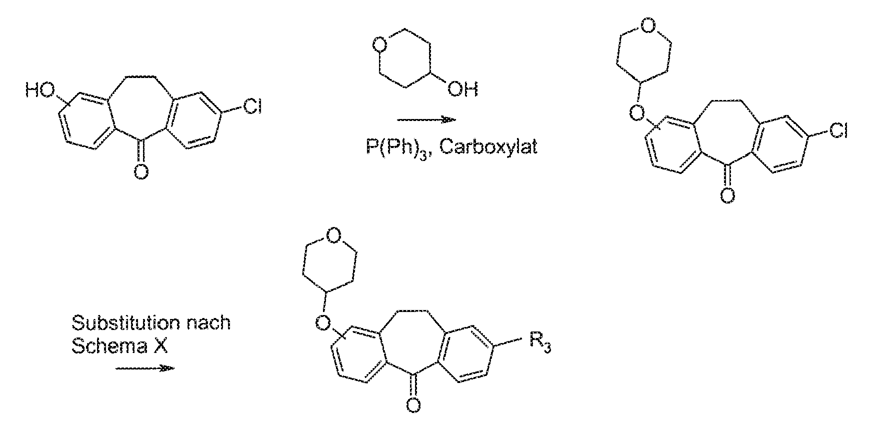

Zu einer gekühlten Lösung aus Chlorhydroxydibenzodihydrodibenzosuberon, 4-Hydroxytetrahydropyran, Triphenylphosphin und THF wird Carboxylat gegeben. Die Reaktion wird bis zur Beendigung bei Raumtemperatur fortgeführt. Das Chlor an Position 2 kann entsprechend Schema X substituiert werden.

Auf diese Weise wurden folgende Verbindungen erhalten:

| | | |
|---|---|---|
| 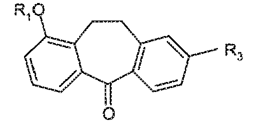 | | |

| Verbindung des Beispiels Nr. | R₁ | R₃ |
|---|---|---|
| 71 (11h) | 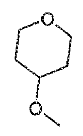 | 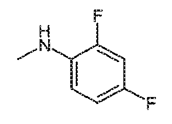 |
| 70 (15) | 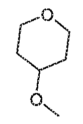 | -Cl |

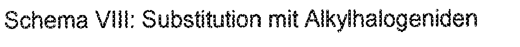

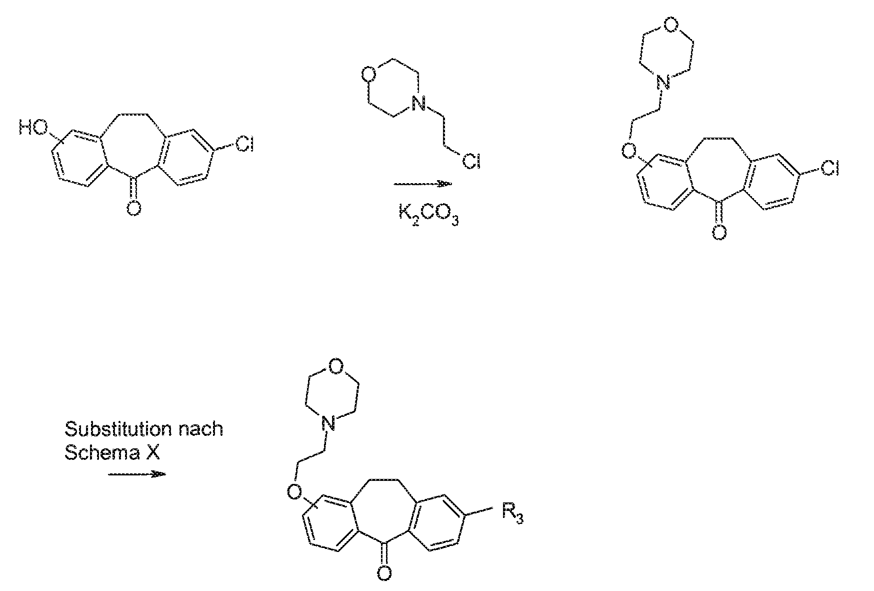

Eine Mischung aus der phenolischen Verbindung, N-2-Chlorethylmorpholin und K₂CO₃ wird mehrere Stunden unter Erwärmen gerührt. Nach dem Abkühlen wird die gewünschte Verbindung in üblicher Weise gewonnen, die organische Phase abgetrennt, gewaschen und eingeengt. Das Chlor an Position 2 kann entsprechend Schema X substituiert werden.

Auf diese Weise wurden folgende Verbindungen erhalten:

| Verbindungen in Position 9 | | | Verbindungen in Position 8 | | |
|---|---|---|---|---|---|
| 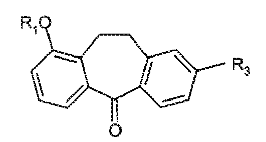 | | | 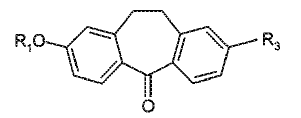 | | |

| Verbindung des Beispiels Nr. | R₁ | R₃ | Verbindung des Beispiels Nr. | R₁ | R₃ |
|---|---|---|---|---|---|
| 73 (11j) | 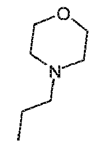 | 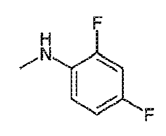 | 56 (12e) | 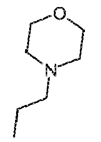 | 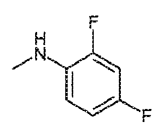 |
| 89 (16) | 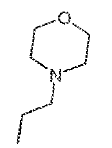 | -Cl | 90 (17) | 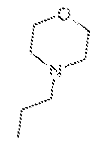 | -Cl |

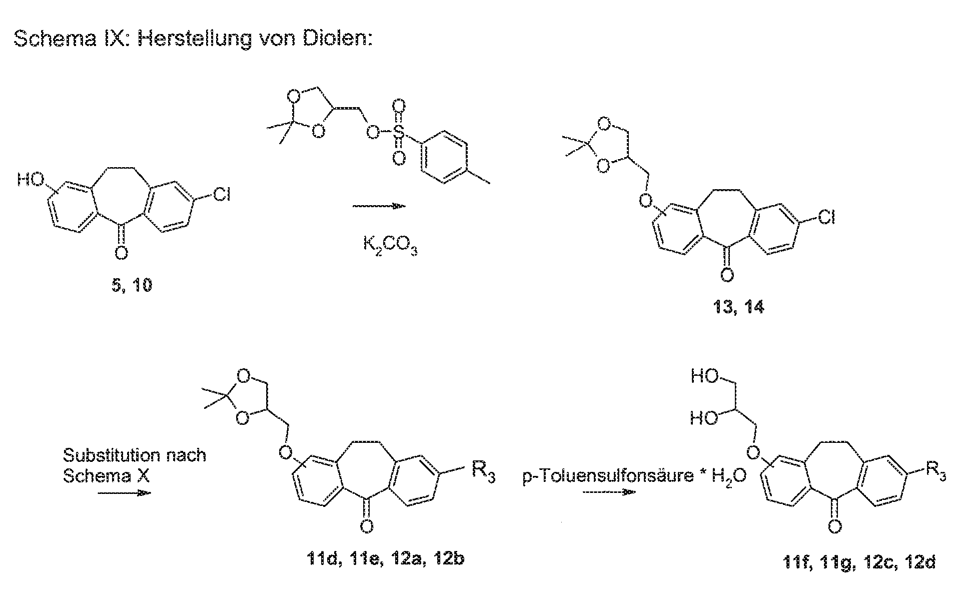

Auf diese Weise wurden folgende Verbindungen erhalten:

| Verbindungen in Position 9 | | | Verbindungen in Position 8 | | |
|---|---|---|---|---|---|
| 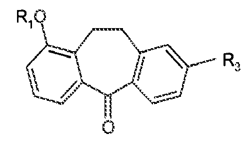 | | | 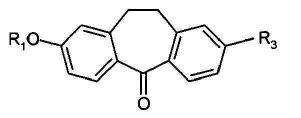 | | |

| Verbindung des Beispiels Nr. | R₁ | R₃ | Verbindung des Beispiels Nr. | R₁ | R₃ |
|---|---|---|---|---|---|
| 66 (11d) | 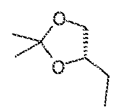 | 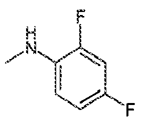 | 51 (12a) | 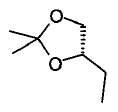 | 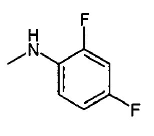 |
| 68 (11e) | 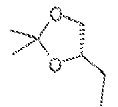 | 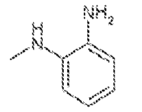 | 53 (12b) | 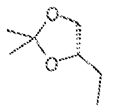 | 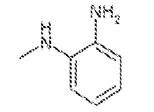 |
| 67 (11f) | 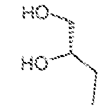 | 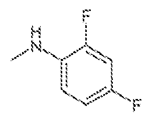 | 52 (12c) | 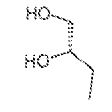 | 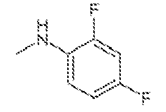 |
| 69 (11g) | 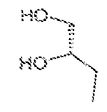 | 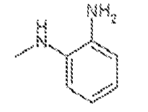 | 54 (12d) | 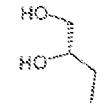 | 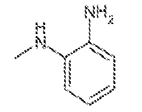 |
| 65 (13) | 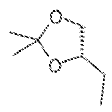 | -Cl | 87 (14) | 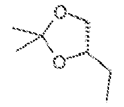 | -Cl |

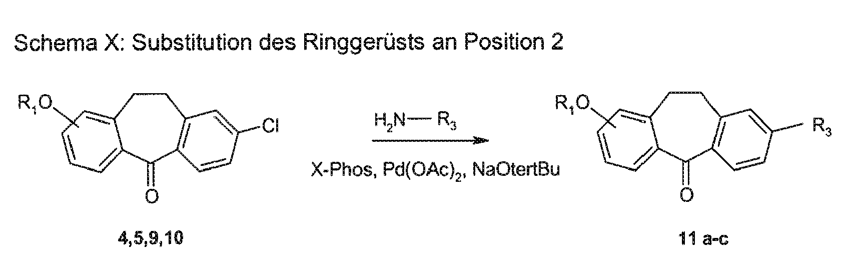

Die Mischung aus Chlordihydrodibenzosuberenon, Pd(OAc)₂, Phosphinligand (2-(Dicyclohexylphosphino)-2',4',6'-triisopropylbiphenyl), NaOtert-Bu, Toluol, tert-BuOH und dem Amin R₃NH₂ wird bei erhöhten Temperaturen mehrere Stunden gerührt. Nach dem Abkühlen wird das gewünschte Produkt in üblicher Weise gewonnen.

Auf diese Weise wurden folgende Verbindungen erhalten:

| | | |
|---|---|---|
| 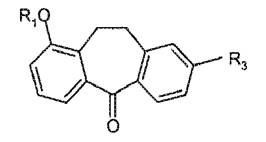 | | |

| Verbindung des Beispiels Nr. | R₁ | R₃ |
|---|---|---|
| 63 (11a) | -CH₃ | 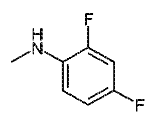 |
| 64 (11b) | -CH₃ | 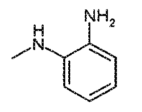 |
| 62 (11c) | -OH | 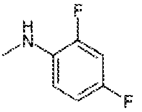 |

Die Herstellung der Verbindungen der Formel I , die an der Position 7 substituiert sind, wobei eines der Ringatome X oder Y für CH₂ steht, erfolgt nach dem in Schema XI erläuterten Verfahren.

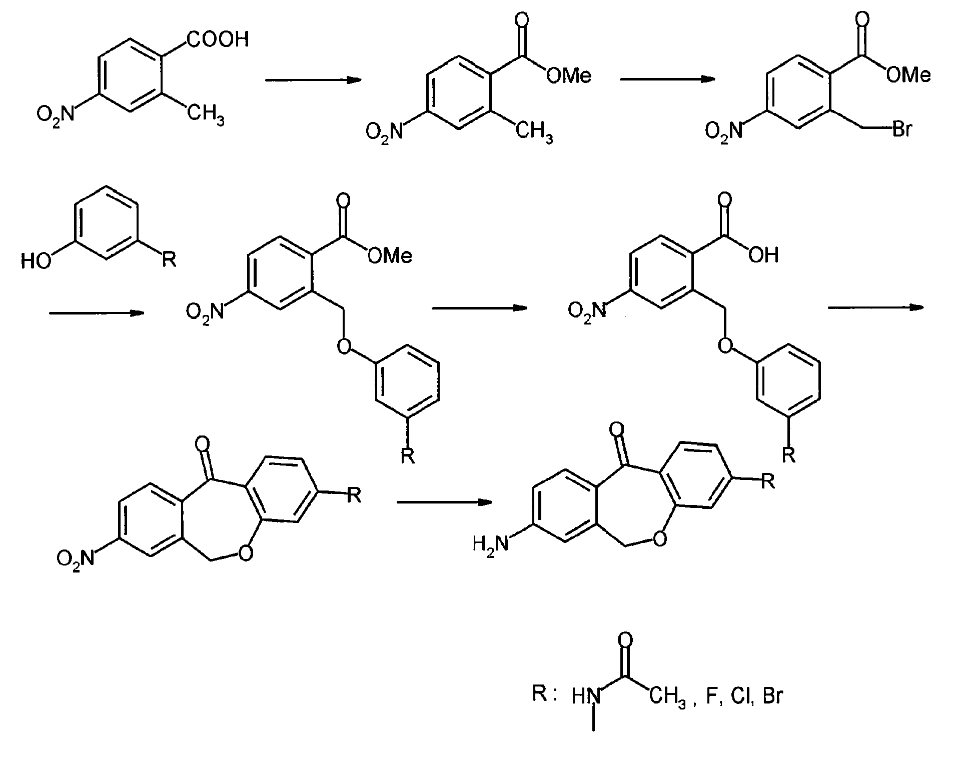

Nach Veresterung der Ausgangsverbindung 2-Methyl-4-nitrobenzoesäure wird in üblicher Weise mit N-Bromsuccinimid (NBS) bromiert. Das bromierte Edukt wird in Gegenwart von Kaliumcarbonat mit dem Phenol umgesetzt. Nach Verseifung des Esters, bspw. mit KOH, erfolgt Ringschluss, beispielsweise mit Polyphosphorsäure in einem organischen Lösungsmittel, wie Sulfolan. Die Nitrogruppe wird anschließend in üblicher Weise, beispielsweise mit Sn/HCl, zur Aminogruppe reduziert. Es empfiehlt sich, bei erhöhten Temperaturen zu arbeiten, zweckmäßigerweise bei der Siedetemperatur des Lösungsmittels bzw. am Rückfluss.

Die Herstellung der Verbindungen der Formel I, worin eines der Ringatome X oder Y für SO oder SO₂ steht, erfolgt durch Oxidation der Verbindungen, worin Y für S steht, in üblicher Weise, beispielsweise mit Perverbindungen, wie m-Chlorperbenzoesäure.

Die erfindungsgemäßen Verbindungen zeigen in vitro und in vivo immunmodulierende und die Freisetzung von TNF-α und IL-1β hemmende Wirkung. Die erfindungsgemäßen Verbindungen eignen sich daher zur Behandlung von Erkrankungen, die im Zusammenhang mit einer Störung des Immunsystems stehen. Sie eignen sich beispielsweise zur Behandlung von Autoimmunerkrankungen, Krebs, rheumatischer Arthritis, Gicht, septischem Schock, Osteoporose, neuropathischem Schmerz, HIV-Ausbreitung, HIV-Demenz, viraler Myokarditis, insulinabhängiger Diabetes, Periodontalerkrankungen, Restenose, Alopezie, T-Zell-Depletion bei HIV-Infektionen oder AIDS, Psoriasis, akuter Pankreatitis, Abstoßungsreaktionen bei allogenen Transplantaten, allergisch bedingter Lungenentzündung, Arteriosklerose, Multipler Sklerose, Kachexie, Alzheimer Erkrankung, Schlaganfall, Ikterus, Colitis ulcerosa, Morbus Crohn, Inflammatory-Bowel-Disease (IBD), Ischämie, kongestive Herzinsuffizienz, Lungen-Fibrose, Hepatitis, Glioblastom, Guillain-Barré-Syndrom, systemischer Lupus erythematodes, Adult-respiratory-distress-Syndrom (ARDS) und Atemnotsyndrom.

Die erfindungsgemäßen Verbindungen können entweder als einzelne therapeutische Wirkstoffe oder als Mischungen mit anderen therapeutischen Wirkstoffen verabreicht werden. Die Verbindungen können alleine verabreicht werden, im Allgemeinen werden sie jedoch in Form pharmazeutischer Mittel dosiert und verabreicht, d.h. als Mischungen der Wirkstoffe mit geeigneten pharmazeutischen Trägern oder Verdünnungsmittel. Die Verbindungen oder Mittel können oral oder parenteral verabreicht werden, vorzugsweise werden sie in oralen Dosierungsformen gegeben.

Die Art des pharmazeutischen Mittels oder Trägers bzw. des Verdünnungsmittels hängt von der gewünschten Verabreichungsform ab. Orale Mittel können bspw. als Tabletten oder Kapseln vorliegen und können übliche Exzipienten enthalten wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglycol oder Siliciumdioxid), desintegrierende Mittel (z. B. Stärke) oder Netzmittel (z. B. Natriumlaurylsulfat). Flüssige Oralpräparate können in Form wässriger oder öliger Suspensionen, Lösungen, Emulsionen, Sirupe, Elixiere oder Sprays und dergleichen sein. Sie können auch als Trockenpulver vorliegen, das zur Rekonstitution mit Wasser oder einem anderen geeigneten Träger aufbereitet wird. Derartige flüssige Präparate können übliche Additive, beispielsweise Suspendiermittel, Geschmacksstoffe, Verdünnungsmittel oder Emulgatoren enthalten. Für die parenterale Verabreichung kann man Lösungen oder Suspensionen mit üblichen pharmazeutischen Trägern einsetzen.

Die erfindungsgemäßen Verbindungen oder Mittel können an Säuger (Mensch oder Tier) in einer Dosis von etwa 0,5 mg bis 100 mg pro kg Körpergewicht pro Tag verabreicht werden. Sie können in einer Einzeldosis oder in mehreren Dosen gegeben werden. Das Wirkungsspektrum der Verbindungen als Inhibitoren der TNF-α und IL-1β Freisetzung wurde anhand nachstehender Testsysteme wie beschrieben von Donat C. und Laufer S. in Arch. Pharm. Pharm. Med. Chem. 333, Suppl. 1, 1-40, 2000, untersucht.

### Experimenteller Teil

Die nachfolgenden Beispiele erläutern die Erfindung ohne sie zu begrenzen.

### Geräte und Methoden

| | |
|---|---|
| Schmelzpunkte | Büchi Melting Point B-545 (thermodynamische Korrektur) |
| NMR-Spektroskopie | Bruker Avance 200 (200 MHz) |
| IR-Spektroskopie | Perkin-Elmer Spectrum One (ATR) |
| GC-MS | Hewlett Packard HP 6890 Series GC-System |
| | Hewlett Packard HP 5973 Mass Selective Detector |
| | GC-Säule: HP-5MS 5% Phenylmethylsiloxan |
| | ESI-MS: 70eV |

### GC-Methode

| Temperatur [°C] | Laufzeit [min] |
|---|---|
| 160 | 1 |
| 160 → 240 | 10 |
| 240 | 5 |
| 240 → 270 | 10 |
| 270 | 35 |

### Abkürzungen:

| | |
|---|---|
| MeOH | Methanol |
| NaOMe | Natriummethylat |
| KOtert-Bu | Kalium-tert.-butylat |
| Tert-BuOH | tert.-Butanol |
| EtOH | Ethanol |
| DMF | Dimethylformamid |
| THF | Tetrahydrofuran |
| DCM | Dichlormethan |
| i.Vak. | im Vakuum |
| EA | Ethylacetat |

### Allgemeine Methode A

Zur Synthese der Stilbene mittels Wittig-Reaktion wird die angegebene Menge Triphenylphosphin in MeOH gelöst. Unter Rühren wird das entsprechende Benzylchlorid zugetropft und 2 Stunden unter Rückfluss erhitzt. Die Reaktionsmischung wird auf 0°C abgekühlt und mit dem entsprechenden Aldehyd versetzt. Anschließend wird bei 0°C innerhalb ca. 45 min die angegebene Menge NaOMe-Lösung zugetropft. Nach beendigtem Zutropfen wird 3 h bei 0°C nachgerührt und das Reaktionsgemisch auf ein gerührtes Gemisch aus 75 g Eis und 175 ml Wasser gegossen. Es wird abgesaugt, der Filterrückstand wird mit ca. 50 ml Wasser gewaschen und die vereinigten wässrigen Phasen werden im Vakuum mehrmals mit Dichlormethan gewaschen. Das Produkt erhält man durch Ansäuern der wässrigen Phase in Form eines Niederschlags, der abfiltriert wird. Die Aufreinigung erfolgt durch Umkristallisieren aus MeOH/H₂O.

### Allgemeine Methode B

Zur Synthese der Ketone mittels Friedel-Crafts-Acylierung wird die angegebene Menge Säure in Dichlormethan suspendiert und der Ansatz mit Argon gespült. Nach Erhitzen zum Rückfluss wird unter Rühren innerhalb 1 Stunde eine Lösung aus Thionylchlorid in 50 ml Dichlormethan zugetropft und eine weitere Stunde gerührt. Nach dem Abkühlen auf Raumtemperatur wird innerhalb von 45 min eine Suspension von AlCl₃ in Dichlormethan zugetropft, 30 min bei Raumtemperatur nachgerührt und das Reaktionsgemisch unter Rühren auf ein Gemisch aus 30 g Eis und 50 ml Wasser gegossen. Das Hydrolysegemisch wird ca. 30 min gerührt, der Niederschlag abfiltriert und die beiden Phasen des Filtrats werden getrennt. Das Produkt erhält man durch Einengen der organischen Phase im Vakuum. Die Aufreinigung erfolgt durch Umkristallisieren mit MeOH.

### Allgemeine Methode C

Zur Herstellung der Testverbindungen wird eine Mischung aus Arylhalogenid, Amin, Pd(OAc)₂, Phosphinligand, KOtert-Bu, Toluol und tert-BuOH unter Argon auf 100 °C erhitzt und die angegebene Zeit bei dieser Temperatur gerührt. Danach wird auf Raumtemperatur abgekühlt, mit 150 ml H₂O hydrolysiert, mit je 3x 200 ml Diethylether extrahiert, die organische Phase filtriert und im Vakuum eingeengt. Die Aufreinigung erfolgt über Flash-Chromatographie.

### Allgemeine Methode D

Zu einer Lösung der Nitroverbindung in EtOH wird SnCl₂ x 5 H₂O hinzugefügt. Die Mischung wird 5 h refluxiert. Dann wird abgekühlt und mit 20%iger NaOH alkalisiert. Es wird mit je 3x 200 ml Ethylacetat extrahiert und die organische Phase im Vakuum eingeengt.

### Allgemeine Methode E

Zur Herstellung 2-Chlor-7-(S-1,2-isopropylidenglycer-3-yl)-10,11-dihydrodibenzo[a,d]cyclohepten-5-on werden 0,57 mmol 2-Chlor-7-hydroxy-10,11-dihydrodibenzo[a,d]cyclohepten-5-on in 10 ml wasserfreiem DMF gelöst, 0,82 mmol des entsprechenden α,β-isopropylideneglycerol-γ-tosylates und 1,65 mmol K₂CO₃ hinzugefügt. Das Reaktionsgemisch wird 24 h unter Argon-Atmosphäre bei 80°C gerührt. Die Reaktionsmischung wird auf Raumtemperatur abgekühlt in 50 ml Wasser gelöst, mit Diethylether extrahiert und die organische Phase wird im Vakuum eingeengt. Das Produkt fällt in Form eines hellgelben Öls an.

### Allgemeine Methode F

Zur Lösung von 2 mmol Diisopropylazodicarboxylat in 20 ml THF wird eine Lösung von 2 mmol P(Ph)₃ in 10 ml THF bei 0°C zugetropft und 1 h gerührt. Anschließend wird eine Lösung aus 2 mmol 2-Chlor-7-hydroxy-10,11-dihydro-dibenzo[a,d]cyclohepten-5-on und 2 ml des entsprechenden Alkohols in THF zugetropft und 2 h bei Raumtemperatur gerührt. Es wird mit Wasser hydrolysiert, mit Ethylacetat extrahiert und die organische Phase im Vakuum eingeengt. Die Aufreinigung erfolgt an SiO₂ (Hexan/Ethylacetat 7+3).

### Allgemeine Methode G

1,7 mmol 2-Chlor-7-hydroxy-10,11-dihydro-dibenzo[a,d]cyclohepten-5-on, 1,86 mol Alkylhalogenid und 6,75 mmol K₂CO₃ werden entweder in DMF oder Acetonitril gelöst, für die angegebene Zeit bei 80°C gerührt, auf Raumtemperatur abgekühlt und in Diethylether und Wasser gelöst. Die organische Phase wird mit Wasser und Lauge gewaschen, über MgSO₄ getrocknet und im Vakuum eingeengt.

### Beispiel 1

### 6-Methoxyphtalid (2)

Die Mischung aus 20 g (131 mmol) 3-Methoxybenzoesäure, 13 ml (160 mmol) 37%ige Formalin-Lsg., 16 ml (162 mmol) 37%ige HCl und 150 ml 150 ml 100%ige Essigsäure wird unter Rühren auf 90°C erhitzt. Nachdem eine klare Lösung entstanden ist, wird der Rührer abgeschaltet und der Ansatz 14 Stunden bei dieser Temperatur belassen. Die Essigsäure wird bei 80°C im Vakuum abgezogen, der Rückstand in 150 ml Toluol aufgenommen und auf 80 ml eingeengt. Die 80°C heiße Lösung wird mit je 40 ml 20%iger Na₂SO₄-Lösung gewaschen, bis der pH der wässrigen Lösung alkalisch ist, dann wird mit 40 ml H₂O gewaschen. Die organische Phase wird nach Zugabe von 6 ml Morpholin 2 h bei 80°C gerührt, dann mit je 50 ml 10%iger H₂SO₄, bis die wässrige Phase sauer ist, sowie mit je zwei mal 50 ml H₂O gewaschen. Zum Kristallisieren des Produkts wird auf 50 ml eingeengt, ggf. ein Impfkristall zugefügt und bis zum Abkühlen auf Raumtemperatur gerührt. Man erhält das Produkt durch Abfiltrieren in Form von weißen Kristallen.
C₉H₈O₃ (Mr 164,16)

| | |
|---|---|
| Ausbeute: | 13,8 g (64%) |
| Smp.: | 107,6 °C |
| GC | 4,3 min |

| | |
|---|---|
| MS m/z (%): | 164 (56, M⁺), 135 (100, M⁺-OCH₃), 107 (25) 92 (11), 77 (19), 63 (10), 51 (6). |
| IR (ATR) | 2945(C-H)ₐₗᵢₚₕ, 1745 (C=O), 1489, 1320, 1278, 1247, 1054, 1017, 990, 9056, 769. |
| ¹H-NMR (DMSO-d6) δ in ppm: | 3.84 (s, 3 H, -OCH₃), 5.33 (s, 2 H, C³-H), 7.28 (s, 1 H, C⁷-H), 7.31 (d, J = 8.3 Hz, C⁴-H), 7.56 (d, 1 H, J = 8.3 Hz, C⁵-H). |

### Beispiel 2

### 2-Formyl-5-methoxy-benzoesäure (3)

10 g (61 mmol) 6-Methoxyphtalid, 11,4 g (64,0 mmol) NBS und 200 ml Chlorbenzol werden unter Rühren gemischt und die Suspension auf 85°C erhitzt und mit 2 ml der Lösung von 100 mg AIBN in 10 ml Chlorbenzol versetzt. Innerhalb weniger Minuten steigt die Temperatur auf 110°C und es entsteht eine rote Lösung. Nach dem Abflauen des Temperaturanstiegs werden die restlichen 8 ml zugegeben und 40 min bei 85 °C gerührt. Nach dem Abkühlen auf 0°C wird vom ausgefallenen Succinimid abfiltriert und mit Chlorbenzen nachgewaschen. Das Filtrat wird eingeengt, bis ein öliger Rückstand entsteht, der mit 10%iger NaOH-Lösung aufgenommen und mit je drei mal 300 ml Dichlormethan gewaschen wird. Nach dem Ansäuern der wässrigen Phase mit konzentrierter HCl wird 1 Stunde bei 0°C gerührt. Dabei fällt die Säure in Form eines weißen Niederschlags aus, von dem abfiltriert wird
C₉H₈O₄ (Mr = 180,16)

| | |
|---|---|
| Ausbeute | 9,6 g (87%) |
| Schmelzpunkt | 166,2 °C |
| IR (ATR) | 2903 (COOH), 2601 (C-H)ₐₗᵢₚₕ, 1703, 1586 (C=O), 1497, 1278, 1210, 1187, 1142, 1072, 1025, 897, 823, 736. |
| H₁-NMR (DMSO-d6) δ in ppm: | 3.64 (s, 3 H, -OCH₃), 6.59 (s, 1 H, -CHO), 7.23-7.86 (m, 3 H, C³-H, C⁴-H, C⁶-H). 10.3 (s, 1 H, -COOH). |

### Beispiel 3

### 2-(3-Nitrophenethenyl)-5-methoxybenzoesäure (5a)

Zur Synthese der Verbindung 5a nach Methode A werden 15,3 g (0,058 mol) Triphenylphosphin, in 100 ml MeOH, 10,0 g (0,058 mol) Nitrobenzylchlorid (gelöst in 50 ml MeOH), 9,6 g (0,058 mol) 2-Formyl-5-methoxybenzoesäure und 28,0 g (0,145 mol) 28%iger NaOMe-Lösung verwendet.
C₁₆H₁₃NO₅ (Mr = 299,29)

| | |
|---|---|
| Ausbeute | 13.5 g (78 %) |
| Schmelzpunkt | 174,7 °C |
| IR (ATR) | 2838 (COOH), 2627 (C-H)ₐₐₗᵢₚₕ, 1687 (C=O), 1530, 1348, 1266, 1237, 810, 734, 671. |
| H₁-NMR (DMSO-d6) δ in ppm: | 3.80 (s, 3 H, -OCH₃), 6.67 (d, 1 H, J = 12.1 Hz, CH=CH), 6.98-7.00 (m, 2 H, C³H, C⁴-H, 7.12 (d, 1 H, J = 12.0 Hz, CH=CH), 7.43-7.47 (m, 3 H, C⁶-H, C⁵'-H, C⁶'-H), 7.88 (s, 1 H, C²'-H), 7.98 (m, 1 H, C⁴'-H). |

### Beispiel 4

### 2-(3-Chlorphenethenyl)-5-methoxybenzoesäure (5b)

Zur Synthese der Verbindung 5b nach Methode A werden 15,3 g (0,058 mol) Triphenylphosphin, in 100 ml MeOH, 9,4 g (0,058 mol) Chlorbenzylchlorid (gelöst in 50 ml MeOH), 9,6 g (0,058 mol) 2-Formyl-5-methoxybenzoesäure und 28,0 g (0,145 mol) 28%iger NaOMe-Lösung verwendet.
C₁₆H₁₃ClO₃ (Mr = 288,73)

| | |
|---|---|
| Ausbeute | 6,7 g (40 %) |
| Schmelzpunkt | 142,3 °C |
| IR (ATR) | 2835 (COOH), 2563 (C-H)ₐₗᵢₚₕ, 1681 (C=O), 1604, 1594, 1419, 1265, 1251, 1218,840,779,756. |
| H₁-NMR (DMSO-d6) δ in ppm: | 3.79 (s, 3 H, -OCH₃), 7.04 (d, 1 H, J = 16.3 Hz, CH=CH), 7.16 (dd, 1 H, J₁ = 2.7 Hz, J₂ = 7.9 Hz), 7.31-7.36 (m, 2 H, C²'-H, C⁴'-H), 7.41 (d, 1 H, J = 7.6, C⁴-H), 7.46-7.55 (m, 2 H, C⁶-H, C⁶'-H), 7.76 (d, 1 H, J = 8.8 Hz, C³-H), 7.86 (d, 1 H, J = 16.4 Hz, CH=CH). |

### Beispiel 5

### 2-(3-Aminophenethyl)-5-methoxybenzoesäure (6a)

10,0 g (0,033 mol) 2-(3-Nitrophenethenyl)-5-methoxybenzoesäure werden in 150 ml EtOH suspendiert und 100 mg Pd/Kohle zugefügt. Der Ansatz wird mehrmals mit H₂ gespült und 72 Stunden bei Raumtemperatur gerührt. Nach dem Abfiltrieren der Pd/Kohle wird im Vakuum eingeengt und der Rückstand mit MeOH/H₂O umkristallisiert.
C₁₆H₁₇NO₃ (Mr = 271,32)

| | |
|---|---|
| Ausbeute | 8,5 g (95 %) |
| Schmelzpunkt | 108,5 °C |
| H₁-NMR (DMSO-d6) δ in ppm: | 2.61 (2 H, m, -CH₂-CH₂-), 3.05 (m, 2 H, -CH₂-CH₂), 3.75 (s, 3 H, -OCH₃), 6.36-6.44 (m, 3 H, C²'-H, C⁴'-H, C⁶'-H), 6.90 (t, 1 H, J₁ = 7.6 Hz, J₂ = 7.7 Hz, C⁵'-H), 7.01 (d, 1 H, J = 8.5 Hz, C⁴-H), 7.20 (d, 1 H, J = 8.5 Hz, C³-H), 7.3 (s, 1 H, C⁶-H). |

### Beispiel 6

### 2-(3-Chlorphenethyl)-5-methoxybenzoesäure (6b)

5,0 g (0,017 mol) 2-(3-Chlorphenethenyl)-5-methoxybenzoesäure werden in einem Gemisch aus 75 ml Ethylacetat und 75 m1 Acetonitril gelöst und 100 mg Pd/Kohle zugefügt. Der Ansatz wird mehrmals mit H₂ gespült und 5 Stunden bei Raumtemperatur gerührt. Nach dem Abfiltrieren der Pd/Kohle wird im Vakuum eingeengt und der Rückstand mit MeOH/H₂O umkristallisiert.
C₁₆H₁₅ClO₃ (Mr = 290,75)

| | |
|---|---|
| Ausbeute | 4,7 g (95 %) |
| H₁-NMR (DMSO-d6) δ in ppm: | 2.73-2,81 (m, 2 H, -CH₂-CH₂-), 3.05-3,13 (m, 2 H, -CH₂-CH₂-), 3,75 (s, 3 H, OCH₃), 6.99-7.35 (m, 6 H, C³-H, C⁴-H, C⁶-H, C²'-H, C⁴'-H, C⁵'-H, C⁶'-H). |

### Beispiel 7

### 2-(3-Acetamidophenethyl)-5-methoxybenzoesäure (7a)

20 g (0,074 mol) 2-Aminophenethyl-5-methoxybenzoesäure werden in 50 ml Acetanhydrid gelöst und 15 Stunden bei Raumtemperatur gerührt. Es wird mit 200 ml Eiswasser hydrolysiert mehrmals mit je 3x 200 ml Ethylacetat extrahiert. Nach dem Einengen der organischen Phase im Vakuum erhält man ein gelbes Öl, aus dem das Produkt durch Umkristallisieren mit MeOH/H₂O erhalten wird.
C₁₇H₁₇NO₂ (Mr = 283,33)

| | |
|---|---|
| Ausbeute | 16.4 g (0,058 mol) (79,0%) |
| Schmelzpunkt | 145,6 °C |
| IR (ATR) | 3265-2933, 2562 (C-H)ₐₗᵢₚₕ, 1622, 1693, 1593, 1567, 1437, 1420, 1289, 1235,789,747. |
| H₁-NMR (DMSO-d6) δ in ppm: | 2.01 (s, 3 H, -CO-CH₃), 2.74 (m, 2 H, -CH₂-CH₂), 3.08 (m, 2 H, -CH₂-CH₂), 3.74 (s, 3 H, -OCH₃), 6.89 (d, 1 H, J= 7.6 Hz, C⁴'-H), 6.95 (d, 1 H, J = 8.5 Hz, C⁶'-H), 7.12-7.20 (m, 2 H, C²'-H, C⁶-H), 7.28 (d, 1 H, J = 2.8 Hz, C⁴-H), 7.44-7.46 (m, 2 H, C³-H, C⁶-H), 9.89 (s, 1 H, -NH-). |

### Beispiel 8

### 2-Nitro-7-methoxydibenzosuberenon (8d)

Zur Herstellung des 2-Nitro-7-methoxydibenzosuberenons nach Methode B werden 5,1 g (0,019 mol) 2-(3-Nitrophenylethenyl)-5-methoxybenzoesäure, 150 ml Dichlormethan, 2,42 g (0,02 mol) Thionylchlorid und 3,33 g (0,025 mol) AlCl₃ verwendet.
C₁₆H₁₁NO₄ (Mr = 281,27)

| | |
|---|---|
| Ausbeute: | 58% |
| Schmelzpunkt | 163,7 °C |
| GC | 17,7 min |
| MS m/z (%): | 281 (100, M⁺), 266 (10, M⁺-CH₃), 235 (10, M⁺-NO₂), 220 (34), 192 (45), 163 (47). |
| IR (ATR) | 3093-3005 (C-H)ₐᵣₒₘ, 2921-2838, 1703 (C=O), 1521, 1482, 1431, 1347, 1290, 1223, 1257, 1019, 872, 806, 730, 674. |
| H₁-NMR (DMSO-d6) δ in ppm: | 3.81 (s, 3 H, -OCH₃), 6.92 (dd, 1 H, J₁ = 2.4 Hz, J₂ = 8.0 Hz), 7.04 (d. 1 H, J = 2.5, C⁸-H), 7.21 (d, 1 H, J = 7.9, -CH=CH-), 7.70 (d, 1 H, J = 8.0, C³-H), 8.13-8.24 (m, 2 H, C²-H, C⁴-H), 8.34 (s, 1 H, C¹⁰-H), 8.67 (t, 1 H, J = 2.0 Hz, C⁷-H). |

### Beispiel 9

### 2-Chlor-7-methoxysuberenon (8c)

Zur Herstellung der Verbindung 8c nach Methode B werden 5,5 g (0,019 mol) 2-(3-Chlorphenylethenyl)-5-methoxybenzoesäure, 150 ml Dichlormethan, 2,42 g (0,02 mol) Thionylchlorid und 3,33 g ( 0,025 mol) AlCl₃ verwendet.
C₁₆H₁₁ClO₂ (Mr = 270,72)

| | |
|---|---|
| Schmelzpunkt | 133,0 °C |
| Ausbeute | 4,8 g (93 %) |
| GC | 14,9 min |
| MS m/z (%): | 272/270 (52/100, M⁺), 244/242 (16/49), 229/227 (12/35), 201/199 ((53/100), 176 (19), 163 (78), 137 (8), 113 (9), 97 (11), 63 (10). |
| IR (ATR) | 2923-2843 (C-H)ₐₗᵢₚₕ, 1636, 1605, 1583 (C=O), 1556, 1503, 1298, 1245,1225, 972, 878, 863, 835, 786. |
| H₁-NMR (DMSO-d6) δ in ppm: | 3.89 (s, 3 H, -OCH₃), 7.06 (d, 1 H, J = 12.1 Hz, -CH=CH-), 7.25 (d, 1 H, J = 12.1 Hz, -CH=CH-), 7.37 (dd, 1 H, J₁ = 2.9 Hz, J₂ = 8.5 Hz, C³-H),7.35-7.40 (m, 2 H, C²-H, C¹⁰-H, 7.72 (d, 1 H, J = 8.7 Hz, C⁸-H), 7.85 (d, 1 H, J = 2.2 Hz, C⁴-H), 8.11 (d, 1 H, J = 8.6 Hz, C⁷-H). |

### Beispiel 10

### 2-Acetamido-7-methoxydibenzosuberon (8a)

Zur Herstellung der Verbindung 8a nach Methode B werden 6,0 g (0,019 mol) 2-(3-Acetamidophenethyl)-5-methoxybenzoesäure, 150 ml Dichlormethan, 2,42 g (0,02 mol) Thionylchlorid und 6,66 g ( 0,05 mol) AlCl₃ verwendet.
C₁₈H₁₇NO₃ (Mr = 295,34)

| | |
|---|---|
| Ausbeute | 4,66 g (83% |
| GC | 27,0 min |
| MS m/z (%): | 295 (100, M⁺), 253 (60, M⁺-COCH₃), 238 (12, 253-CH₃), 224 (36, 238-NH), 210 (10), 194 (10), 180 (9), 165 (14), 152 (8). |
| H₁-NMR | 2.07 (s, 3 H, -COCH₃), 3.05 (s, 4 H, -CH₂-CH₂), 3.75 (s, 3 H, -OCH₃), 7.05 (d, 1 H, J = 8.4 Hz, C⁸-H), 7.22 (d, 1 H, J = 8.4 Hz, C⁹-H), 7.38 (s, 1 H, C⁵-H), 7.52-7.56 (m, 2 H, C¹-H, C³-H), 7.92 (d, 1 H, J = 9.3 Hz, C⁴-H), 10.22 (s, 1 H, -NH-). |

### Beispiel 11

### 2-Chlor-7-methoxydibenzodibenzosuberon (8b)

Zur Herstellung der Verbindung 8b nach Methode B werden 5,5 g (0,019 mol) 2-(3-Chlorphenylethyl)-5-methoxybenzoesäure, 150 ml Dichlormethan, 2,42 g (0,02 mol) Thionylchlorid und 3,33 g (0,025 mol) AlCl₃ verwendet.
C₁₆H₁₃ClO₂ (Mr = 272,73)

| | |
|---|---|
| Ausbeute | 58% |
| GC | 12,8 min |
| MS m/z (%): | 274/272 (35/100, M⁺), 259/257 ((5/17, M⁺-CH₃), 243, 241 (19/21, M⁺-OCH₃), 208 (29), 194 (17),178 (23, 208-CO), 165 (49). |
| H₁-NMR (DMSO-d6) δ in ppm: | 3.09 (s, 4 H, -CH₂-CH₂), 3.77 (s, 3 H, -OCH₃), 7.06 (dd, 1 H, J₁ = 2.8Hz, J₂ = 8.4 Hz, C³-H), 7.25 (d, 1 H, J = 8.4 Hz, C³-H), 7.38-7.45 (m, 3 H, C¹-H, C⁶-H, C⁹-H), 7.85 (d, 1 H, J = 8.3, C⁴-H). |

### Beispiel 12

### 2-Amino-7-methoxydibenzosuberon (9a)

4,0 g ( 0,0135 mol) 2-Acetamido-7-methoxydibenzosuberon werden in 100 ml 20%iger HCl suspendiert und das Reaktionsgemisch 5 Stunden unter Rückfluss erhitzt.
Beim Abkühlen tritt ein Niederschlag auf, von dem abfiltriert wird. Nach dem Trocknen im Vakuum erhält man ein sandfarbenes Pulver.
C₁₆H₁₅NO₂ (Mr = 253,30)

| | |
|---|---|
| Ausbeute | 1,85 g (0,0073 mol) (54,0 %) |
| Schmelzpunkt | 185,3 °C |
| GC | 18,4 min |
| MS m/z (%): | 253 (100, M⁺), 238 (51, M⁺-CH₃), 224 (41, 238-NH), 210 (14), 194 (12), 180 (13), 165 (13), 152 (6). |
| H₁-NMR | 2.96 (s, 4 H, -CH₂-CH₂), 3.75 (s, 3 H, -OCH₃), 6.06 (s, 2 H, -NH₂), 6.34 (s, 1 H, C¹-H), 6.49 (d, 1 H, J = 8.7 Hz, C³-H), 7.00 (d, 1 H, J = 8.3 Hz, C⁸-H), 7.19 (d, 1 H, J = 8.3 Hz, C⁹-H), 7.36 (s, 1 H, C⁶-H), 7.87 (d, 1 H, J = 8.7 Hz, C⁴-H). |

### Beispiel 13

### 2-Chlor-7-hydroxydibenzosuberon (12)

Zu einer Lösung von 1,0 g (2,67 mmol) 2-Chlor-7-methoxydibenzosuberon in 10 ml DCM wird unter Argon-Schutzgasatmosphäre bei Raumtemperatur 0,13 ml (1,34 mmol) Bortribromid zugetropft. Die Reaktionsmischung wird bei Raumtemperatur 6 h gerührt, anschließend mit 20 ml Wasser versetzt und die org. Phase im Vakuum eingeengt. Man erhält das Produkt in Form eines orangenen Feststoffes.
C₁₅H₁₁ClO₂ (Mr = 258,71)

| | |
|---|---|
| Ausbeute | 0,66 g (95%) |
| Schmelzpunkt | 191,6 °C |
| IR (ATR) | 2926, 1589 (C=O), 1561, 1459, 1422, 1363, 1312, 1160, 1080, 1006,837,798. |
| H₁-NMR (DMSO-d6) δ in ppm: | 3.05 (dd, 4 H, J₁ = 9.0, J₂ = 13.5, -CH₂-CH₂-), 6.91 (d, 1 H, J = 8.2, C⁸-H), 7.13 (d, 1 H, J = 8.3, C⁹-H), 7.30-7.80 (m, 3 H, C¹-H, C³-H, C⁶-H), 7.82 (d, 1 H, J = 9.1, C⁴-H). |
| ¹³C-NMR (DMSO-d6) δ in ppm: | 33.4 (C¹¹), 34.4 (C¹⁰), 116.2 (C⁶), 120.7 (C⁸), 126.9 (C³), 129.3 (C¹), 131.5 (C⁹), 132.5 (C⁴), 133.1 (C^{4a}), 137.1 (C^{5a}), 137.3 (C^{9a}), 138.5 (C²), 144.7 (C^{11a}), 156.1 (C⁷), 193.5 (C⁵). |

### Beispiel 14

### 2-Chlor-7-(S-1,2-isopropylidenglycer-3-yl)-10,11-dihydro-dibenzo[a,d]cyclohepten-5-on (13a)

Die Herstellung der Titelverbindung erfolgt nach Methode E.
C₂₁H₂₁ClO₄ (Mr = 372,85)

| | |
|---|---|
| Ausbeute: | 89 % |
| GC | 22,7 min |
| MS m/z (%): | 374/372 (15/43, M⁺), 359/357 (6/17), 299/297 (26/76), 273/271 (5/13), 260/258 (4/10), 194 (11), 178 (24), 165 (37), 145 (24), 115 (46), 101 (100), 73 (16), 59 (16). |
| ¹H-NMR (DMSO-d6) δ in ppm: | 1.29 (s, 3 H, -CH₃), 1.34 (s, 3 H, -CH₃), 3.06 (s, 4 H, -CH₂-CH₂-), 3.73-3.80 (m, 1 H, -OCH₂-), 4.03-4.12 (m, 3 H, -CH₂O-, -OCH₂-), 4.37-4.42 (m, 1 H, -CH-), 7.13 (d, 1 H, J = 8.3, C⁸-H), 7.27 (d, 1 H, J = 8.4, C⁹-H), 7.39-7.47 (m, 3 H, C¹-H, C³-H, C⁶-H), 7.85 (d, 1 H, J = 8.3, C⁴-H). |
| ¹³C-NMR (DMSO-d6) δ in ppm: | 25.8 (-CH₃), 26.9 (-CH₃), 33.3 (C¹¹), 34.4 (C¹⁰), 66.0 (-OCH₂-), 69.3 (-CH₂O-), 74.0 (-CH-), 115.0 (C⁶), 120.3 (C⁸), 127.0 (C³), 129.5 (C¹), 131.6 (C⁹), 132.6 (C⁴), 135.1 (C^{4a}), 136.9 (C^{5a}), 137.5 (C^{9a}), 138.6 (C²), 144.8 (C^{11a}), 157.3 (C⁷), 193.2 (C⁵). |

### Beispiel 15

### 2-Chlor-7-(R-1,2-isopropylidenglycer-3-yl) -10,11-dihydro-dibenzo[a,d]cyclohepten-5-on (13b)

Die Herstellung der Titelverbindung erfolgt nach Methode E.
C₂₁H₂₁ClO₄ (Mr = 372,85)

| | |
|---|---|
| Ausbeute: | 91 % |
| GC | 24,6 min |
| MS m/z (%): | 374/372 (16/45, M⁺), 359/357 (6/17), 299/297 (26/76), 273/271 (5/13), 260/258 (4/11), 194 (12), 178 (26), 165 (39), 145 (25), 115 (46), 101 (100), 73 (14), 59 (14). |
| ¹H-NMR (DMSO-d6) δ in ppm: | 1.29 (s, 3 H, -CH₃), 1.34 (s, 3 H, -CH₃), 3.10 (s, 4 H, -CH₂-CH₂-), 3.72-3.80 (m, 1 H, -CH₂O-), 4.03-4.09 (m, 3 H, -CH₂O-, -OCH₂-), 4.31-4.46 (m, 1 H, -CH-), 7.13 (d, 1 H, J = 8.6 C⁸-H), 7.27 (d, 1 H, J = 8.5, C⁹-H), 7.39-7.49 (m, 3 H, C¹-H, C³-H, C⁶-H), 7.85 (d, 1 H, J = 7.4, C⁴-H). |
| ¹³C-NMR (DMSO-d6) δ in ppm: | 25.7 (-CH₃), 26.9 (-CH₃), 33.3 (C¹¹), 34.3 (C¹⁰), 660 (-CH₂O-), 69.3 (-OCH₂-), 74.0 (-CH-), 115.0 (C⁶), 120.2 (C⁸), 127.0 (C³), 129.5 (C¹), 131.6 (C⁹), 132.6 (C⁴), 135.1 (C^{4a}), 136.9 (C^{5a}), 137.5 (C^{9a}), 138.6 (C²), 144.7 (C^{11a}), 157.3 (C⁷), 193.1 (C⁵). |

### Beispiel 16

### 2-Chlor-7-(3-acetoxypropoxy) -10,11-dihydro-dibenzo[a,d]-cyclohepten-5-on (13h)

Die Herstellung der Titelverbindung erfolgt nach Methode G unter Verwendung von 3-lodpropyl-acetat als Edukt in DMF als Solvens.
C₂₀H₁₉ClO₄(Mr = 358,83)

| | |
|---|---|
| Ausbeute: | 87 % |
| GC | 24,2 min |
| MS m/z | (%):360/358 (2/6, M⁺), 259/257 (2/5, M⁺-Acetoxypropyl), 243/241 (2/2, M⁺- Acetoxypropoxy), 194 (9, 243/241-Cl), 178 (6), 165 (17), 101 (100, Acetoxypropyl), 73 (11). |
| ¹H-NMR (DMSO-d6) δ in ppm: | 1.96-2.08 (m, 5 H, CH₃-Ac, -CH₂-), 3-04-3.13 (m, 4 H, -CH₂-CH₂-), 4.01-4.18 (m, 4 H, -OCH₂-, -CH₂O-), 7.10 (d, 1 H, J = 8.3, C⁸-H), 7.25 (d, 1 H, J = 8.4, C⁹-H), 7.30-7.45 (m, 3 H, C¹-H, C³-H, C⁶-H), 7.57 (d, 1 H, J = 8.3, C⁴-H). |
| ¹³C-NMR (DMSO-d6) δ in ppm: | 21.0 (CH₃Ac-), 28.4 (-CH₂-), 33.6 (C¹¹), 34.3 (C¹⁰), 61.1 (AcO-CH₂-), 64.8 (-OCH₂), 114.9 (C⁶), 120.3 (C⁸), 127.6 (C³), 130.3 (C¹), 131.6 (C⁹), 132.6 (C⁴), 135.0 (C^{4a}), 136.9 (C^{5a}), 137.5 (C^{9a}), 138.5 (C²), 144.7 (C^{11a}), 157.3 (C⁷), 170.7 (C=O), 193.1 (C⁵). |

### Beispiel 17

### 2-Chlor-7-(3-acetoxyethoxy)-10,11-dihydro-dibenzo[a,d]cyclohepten-5-on (13g)

Die Herstellung der Titelverbindung erfolgt nach Methode G unter Verwendung von 3-lodethyl-acetat als Edukt und DMF als Solvens.
C₁₉H₁₇ClO₄ (Mr = 344,80)

| | |
|---|---|
| Ausbeute: | 78 % |
| GC | 21,1 min |
| MS m/z (%): | 346/344 (2/4, M⁺), 194 (5, 241/243-Cl), 178 (8), 165 (11), 87 (100, Acetoxyethyl). |
| ¹H-NMR (DMSO-d6) δ in ppm: | 2.03 (s, 3 H, -COCH₃), 3.10 (s, 4 H, -CH₂-CH₂-), 4.19-4.23 (m, 2 H, -OCH₂-,), 4.30-4.35 (m, 2 H, -CH₂-O-); 7.11-7.48 (m, 5 H, C¹-H, C³-H, C⁶-H, C⁸-H, C⁹-H), 7.86 (d, 1 H, J = 8.3, C⁴-H). |
| ¹³C-NMR (DMSO-d6) δ in ppm: | 21.0 (CH₃-CO-), 33.3 (C¹¹), 34.3 (C¹⁰), 62.8 (AcO-CH₂-), 66.4 (-CH₂O-), 115.0 (C⁶), 120.3 (C⁸), 127.0 (C³), 129.5 (C¹), 131.6 (C⁹), 132.6 (C⁴), 135.2 (C^{11a}), 136.9 (C^{5a}), 137.5 (C^{9a}), 138.6 (C²), 144.8 (C^{4a}), 157.1 C⁷), 170.7 (C=O), 193.1 (C⁵). |

### Beispiel 18

### 2-Chloro-7-(2-morpholin-4-yl-ethoxy)-10,11-dihydro-dibenzo[a,d]-cyclohepten-5-on (13c)

Die Herstellung der Titelverbindung erfolgt nach Methode G unter Verwendung von 2-Chlorethylmorpholin-hydrochlorid als Edukt in Acetonitril als Solvens.
C₂₁H₂₂ClNO₃ (Mr = 371,87)

| Ausbeute: | |
|---|---|
| GC | 32,7 min |
| MS m/z (%): | 373/371 (1/2, M⁺), 330/328 (2/6), 286/284 (2/6, M⁺-Morpholin-4-yl), 178 (6), 165 (4) 100 (100, Morphin-4-yl-methyl). |
| ¹H-NMR (DMSO-d6) δ in ppm: | 3.11 (s, 4 H, -CH₂-CH₂), 3.20-3.53 (m, 6 H, -N-CH₂, -CH₂-N-CH₂-), 3.79-4.06 (m, 4 H, -CH₂-O-CH₂-), 4.47-4.50 (m, 2 H, (-OCH₂-), 7.19 (d, 1 H, J = 8.3, C⁸-H), 7.32 (d, 1 H, J = 8.4, C⁹-H), 7.40-7.47 (m, 3 H, C¹-H, C³-H, C⁶-H), 7.86 (d, 1 H, J = 8.4, C⁴-H), 11.57 (s, 1 H, -NH-). |
| ¹³C-NMR (DMSO-d6) δ in ppm: | 33.3 (C¹¹), 34.4 (C¹⁰), 57.0 (-CH₂-O-CH₂-), 60.1 (-N-CH₂-), 65.5 (-OCH₂-), 66.1 (-CH₂-O-CH₂), 115.1 (C³), 120.3 (C⁸), 127.0 (C³), 129.5 (C¹), 131.6 (C⁹), 132.6 (C⁴), 135.0 (C^{4a}), 136.9 (C^{5a}), 137.5 (C^{9a}), 138.6 (C²), 144.7 (C^{11a}), 157.2 (C⁷), 193.2 (C⁵). |

### Beispiel 19

### 2-Chloro-7-(2-tetrahydropyran-4-yl-oxy)-10,11-dihydro-dibenzo[a,d]-cyclohepten-5-on (13i)

Die Herstellung der Titelverbindung erfolgt nach Methode F.
C₂₀H₁₉ClN₃ (Mr = 342,83)

| | |
|---|---|
| Ausbeute: | 46 % |
| ¹H-NMR (DMSO-d6) δ in ppm: | 1.54-1.61 (m, 2 H, -CH₂-), 1.91-1.98 (m, 2 H, -CH₂-), 3.06-3.16 (m, 4 H, -CH₂-CH₂-), 3.42-3.54 (m, 2 H, -CH₂O-), 3.78-3.86 (m, 2 H, -CH₂O-), 4.56-4.62 (m, 1 H, -CH-), 7.16 (d, 1 H, J = 8.4, C⁸-H), 7.27 (d, J = 8.4, C⁹-H), 7.38-7.48 (m, 3 H, C¹-H, C³-H, C⁶-H), 7.86 (d, 1 H, J = 8.4 (C⁴-H). |

### Beispiel 20

### 2-(2-Aminoanilino)-7-methoxydibenzosuberon (10a)

Zur Herstellung der Verbindung 10a nach Methode C werden 0,5 g (1,8 mmol) 2-Chlor-7-methoxydibenzosuberon, 1,0 (9,2 mmol) g 1,2-Phenylendiamin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₂H₂₀N₂O₂ (Mr = 344,42)

| | |
|---|---|
| Ausbeute: | 0,48 g (78 %) |
| Schmp.: | 106,5 °C |
| GC | 49,4 min |
| MS m/z (%): | 344 (100, M⁺), 329 (8), 315 (6), 301 (5), 195 (7), 165 (6), 107 (9). |
| IR | 3418-3343 (C-H)ₐᵣₐₒₘ, 3320 (N-H), 2976-2939 (C-H)ₐₗᵢₚₕ, 1546 (C=O), 1516, 1494, 1323, 1284, 1147, 1026, 828, 751, 740. |
| ¹H-NMR (DMSO-d6) δ in ppm: | 2.96 (s, 4 H, -CH₂-CH₂), 3.76 (s, 3 H, -OCH₃), 4.84 (s, 2 H, -NH₂), 6.46 (s, 1 H, C¹-H), 6.54-6.64 (m, 2 H, C³'-H, C⁶'-H), 4,78 (d, 1 H, J = 7.8 Hz, C⁴'-H), 6.91-7.04 (m, 3 H, C³-H, C⁵-H, C⁸-H), 7.20 (d, 1 H, J = 8.3 Hz, C⁹-H), 7.39 (s, 1 H, C⁶-H), 7.92-7.97 (m, 2 H, C⁴-H, -NH-). |
| ¹³C-NMR (DMSO-d6) δ in ppm: | 33.6 (C¹⁰), 36.6 (C¹¹), 55.5 (-OCH₃), 112.1 (C⁶), 113.1 (C³), 114.8 (C¹), 115.8 (C^{3'}), 116.8 (C⁴'), 118.3 (C⁸), 125.2 (C^{5'}), 126.2 (C^{6'}), 126.3 (C^{1'}), 126.4 (C^{4a}), 130.4 (C⁹), 133.9 (C^{5a}), 134.5 (C⁴), 140.3 (C^{9a}), 144.0 (C²'), 145.7 (C^{11a}), 151.1 (C²). 158.0 (C⁷), 190.3 (C⁵). |

### Beispiel 21

### 2-(2-Amino-4-fluoranilino)-7-methoxydibenzosuberon (10b)

Zur Herstellung der Verbindung 10b nach Methode C werden 0,5 g (1,8 mmol) 2-Chlor-7-methoxydibenzosuberon, 0,3 g (1,9 mmol) 2-Nitro-4-fluoranilin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet. Die Aufreinigung der Nitroverbindung erfolgt durch Umkristallisieren mit MeOH. Ohne weitere Aufreinigung werden 0,5 g der Nitroverbindung, 2,9 g ZnCl₂ x2 H₂O in 20 ml EtOH nach Methode D reduziert.
C₂₂H₁₉FN₂O₂ (Mr = 362,41 )

| | |
|---|---|
| Ausbeute: | 0,42 g (65%) |
| Schmp.: | 72.2 °C |
| GC | 54,1 min |
| MS m/z (%): | 362 (100, M⁺), 247 (7), 125 (14). |
| IR (ATR) | 3353 (N-H), 2941-2834 (C-H)ₐₗᵢₚₕ, 1622, 1567 (C=O), 1508, 1324, 1273, 1162, 839, 784. |
| ¹H-NMR (DMSO-d6) δ in ppm: | 2.49 (s, 4 H, -CH₂-CH₂), 3.76 (s, 3 H, -OCH₃), 5.25 (s, 2 H, -NH₂), 6.30-6.38 (m, 2 H, C³'-H, C⁵'-H), 6.51-6.58 (m, 2 H, C³-H, C⁶'-H), 7.94-7.05 (m, 2 H, C¹-H, C⁸-H), 7.21 (d, 1 H, J = 8.3 Hz, C⁹-H), 7.38 (s, 1 H, C⁶-H) 7.89 (s, 1 H, -NH-), 7.93 (d, 1 H, C⁴-H). |
| ¹³C-NMR (DMSO-d6) δ in ppm: | 33.6 (C¹⁰), 36.5 (C¹¹), 55.5 (-OCH₃), 101.4 (d, J = 25.5 Hz, C³'), 102.5 (d, J = 22.6 Hz, C⁵'), 111.9 (C⁶), 112.9 (C³), 114.8 (C¹), 118.4 (C⁸), 121.3 (d, J = 2.3 Hz, C¹'), 126.2 (C^{4a}), 128.4 (d, J = 20.8 Hz, C⁶'), 130.4 (C⁹), 134.0 (C^{5a}), 134.5 (C⁴), 140.3 (C^{9a}), 145.8 (C^{11a}), 146.5 (d, J = 12.0 Hz, C²'), 151.5 (C²), 158.0 (C⁷), 161.2 (d, J = 238.6 Hz, C⁴'), 190.2 (C⁵). |

### Beispiel 22

### 2-(2, 4-Difluoranilino)-7-methoxydibenzosuberon (10c)

Zur Herstellung der Verbindung 10c nach Methode C werden 0,5 g (1,8 mmol) 2-Chlor-7-methoxydibenzosuberon, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₂H₁₇F₂NO₂ (Mr = 365,38)

| | |
|---|---|
| Ausbeute: | 0,57 g (87%) |
| Schmp.: | 123-126 °C (Zersetzung). |
| GC | 31,4 min |
| MS m/z (%): | 365 (100, M⁺), 350 (7, M⁺-CH₃), 337 (11), 322 (6), 237 (4, M⁺-2-NH₂, 4-F-Anilin), 208 (15), 194 (5), 178 (6), 165 (15), 152 (5). |
| IR (ATR) | 3327 (N-H), 3074 (C-H)ₐᵣₒₘ, 2941-2842 (C-H)ₐₗᵢₚₕ, 1338, 1551 (C=O), 1525, 1498, 1325, 1281, 1238, 854, 832, 786. |
| ¹H-NMR (DMSO-d6) δ in ppm: | 2.99 (s, 4 H, -CH₂-CH₂), 3.76 8s, 3 H, -OCH₃), 6.61 (s, 1 H, C¹-H), 6.74 (d, 1 H, J = 8.7 Hz, C³-H), 7.00-7.47 (m, 6 H, C⁶-H, C⁸-H, C⁹-H, C³'-H, C⁵'-H, C⁶'-H), 7.95 (d, 1 H, J = 8.8 Hz, C⁴-H), 8.55 (s, 1 H, -NH-). |
| ¹³C-NMR (DMSO-d6) δ in ppm: | 36.2 (C¹⁰), 38.6 (C¹¹), 55.5 (-OCH₃), 105.3 (dd, J = 26.7 Hz, C³'), 112.2 (d, J = 22.1 Hz, C⁵'), 112.6 (C⁶), 113.9 (C³), 114.7 (C¹), 118.6 (C⁸), 125.3 (d, J = 12.2 Hz, C¹'), 126.4 (d, J = 9.7 Hz, C⁶'), 127.7 (C^{4a}), 130.6 (C⁹), 133.8 (C^{5a}), 134.6 (C⁴), 140.0 (C^{9a}), 145.7 (C^{11a}), 149.4 (C²), 154.9 (d, J = 143.5 Hz, C⁴'), 158.1 (C⁷), 159.8 (d, J = 138.2 Hz, C⁷'), 190.8 (C⁵). |

### Beispiel 23

### 2-(2-Chlor-4-fluoranilino)-7-methoxydibenzosuberon (10d)

C₂₂H₁₇ClFNO₂ (Mr = 381,84)

Zur Herstellung der Verbindung 10d nach Methode C werden 0,5 g (1,8 mmol) 2-Chlor-7-methoxydibenzosuberon, 0,27 g (1,9 mmol) 2-Chlor, 4-fluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.

| | |
|---|---|
| Ausbeute: | 0,24 g(35%) |
| Schmp.: | 110,2 °C |
| GC | 38,4 min |
| MS m/z (%): | 385/383 (36/100, M⁺), 368/366 (2/6, M⁺-CH₃), 354/352 (4/9), 340/338 (2/4), 274/272 (2/6), 237 (4, M⁺-2-Cl, 4-F-Anilin), 208 (14), 194 (5), 178 (6, 208-CO), 165 (13), 151 (4), 136 (6). |
| IR (ATR) | 3336 (N-H), 3070 (C-H)ₐᵣₒₘ, 2939-2835 (C-H)ₐₗᵢₚₕ, 1602, 1554 (C=O), 1520, 1484, 1282, 1255, 1235, 863, 818, 785. |
| ¹H-NMR (DMSO-d6) δ in ppm: | 3.00 (s, 4 H, -CH₂-CH₂-), 3.77 (s, 3 H, -OCH₃), 6.60 (s, 1 H, C¹-H), 6.72 (d, 1 H, J = 8.8 Hz, C³-H), 7.03 (d, 1 H, J = 5.6 Hz, C⁶, -H), 7.18-7.57 (m, 5 H, C⁶-H, C⁸-H, C⁹-H, C^{3'}-H, C^{5'}-H), 7.94 d, 1 H, J = 8.7 Hz, C⁴-H), 8.46 (s, 1 H, -NH-). |
| ¹³C-NMR (DMSO-d6) δ in ppm: | 33.5 (C¹²), 36.2 (C¹¹), 55.5 (OCH₃), 112.8 (C⁶), 114.1 (C³), 114.6 (C¹), 115.6 (C^{5'}), 117.7 (C^{3'}), 118.6 (C⁸), 127.3 (C^{1'}), 127.8 (C⁴), 129.4 (C^{2'}), 130.6 (C⁹), 133.7 (C^{5a}), 134.7 (C⁴), 140.0 (C^{9a}), 145,7 (C^{11a}), 149.5 (C²), 158.1 (C⁷), 158.9 (C^{4'}), 190.8 (C⁵). |

### Beispiel 24

### 2-(2,4,5-Trifluoranilino)-7-methoxydibenzosuberon (10e)

C₂₂H₁₆F₃NO₂ (Mr = 383,37)

Zur Herstellung der Verbindung 10e nach Methode C werden 0,5 g (1,8 mmol) 2-Chlor-7-methoxydibenzosuberon, 0,27 g (1,8 mmol) 2-, 4-, 5-Trifluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.

| | |
|---|---|
| Ausbeute: | 0,30 g (43%) |
| Schmp.: | 132.1 °C |
| GC | 30,0 min |
| MS m/z (%): | 383 (100, M⁺), 368 (8, M⁺-CH₃), 355 (11), 340 (7), 237 (4, M⁺-2, 3, 4-Trifluoranilin), 208 (13),194 (5), 178 (8), 165 (13). |
| IR (ATR) | 3337 (N-H), 3298-3016 (C-H)ₐᵣₒₘ. 2912-2846 (C-H)ₐₗᵢₚₕ, 1582, 1567, 1518 (C=O), 1286, 1273, 1221, 1161, 856, 832, 808. |
| ¹H-NMR (DMSO-d6) δ in ppm: | 3.01 (s, 4 H, -CH₂-CH₂), 3.76 (s, 3 H, -OCH₃), 6,72 (s, 1 H, C¹-H), 6.82-7.05 (m, 2 H, C^{3'}-H, C^{6'}-H), 7.21 (d, 1 H, J = 8.3 Hz, C³-H), 7.38-7.65 (m, 2 H, C⁸-H, C⁹-H), 7.98 (d, 1 H, J = 7.98Hz, C²-H), 8.65 (s, 1 H, -NH-). |
| ¹³C-NMR (DMSO-d6) δ in ppm: | 33.5 (C¹⁰), 36.1 (C¹¹), 55.5 (-OCH₃), 106.4-107.4 (m, C^{3'}), 111.9-112.3 (m, C^{6'}), 113.3 (C⁶), 114.6 (C³), 114.7 (C¹), 118.7 (C⁸), 125.6-126.2 (m, C^{1'}), 126.2 (C^{4a}), 128.6 (C⁹), 130.6 (C^{5a}), 133.6 (C⁴), 134.6 (C^{9a}), 142.6-147.9 (m, C^{4'}), 143.6-148.9 (m, C^{5'}), 145.5 (C^{11a}), 148.3 (C²), 148.3-153,3 (m, C^{2'}), 158.1 (C⁷), 191.1 (C⁵). |

### Beispiel 25

### 2-(2-Trifluormethylanilino)-7-methoxydibenzosuberon (10f)

Zur Herstellung der Verbindung 10f nach Methode C werden 0,5 g (1,8 mmol) 2-Chlor-7-methoxydibenzosuberon,0,3 (1,9 mmol) 2-Aminotrifluorid, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₃H₁₈F₃NO₂ (Mr = 397,40)

| | |
|---|---|
| Ausbeute: | 0,23 g (32%) |
| Schmp.: | 152,5 °C |
| GC | 30,3 min |
| MS m/z (%): | 397 (100, M⁺), 282 (7, M⁺-CH₃), 368 (12), 354 (6), 237 (5, M⁺-2-CF₃-Anilin), 208 (12), 194 (5),178 (6), 165 (14). |
| IR (ATR) | 3319 (N-H), 3072-3007 (C-H)ₐᵣₒₘ, 2958-2836 (C-H)ₐₗᵢₚₕ, 1550 (C=O), 1460, 1353, 1323, 1285, 1264, 1111, 1091, 1050, 692. |
| ¹H-NMR (DMSO-d6) δ in ppm: | 3.02 (s, 4 H, -CH₂-CH₂-),3.76 (s, 3 H, -OCH₃), 6.91 (s, 1 H, C¹-H), 7.01-7.06 (m, 2 H, C³-H, C^{6'}-H), 7.20-7.25 (m, 2 H, C^{2'}-H, C^{4'}-H), 7.37-7.39 (m, 2 H, C⁸-H, C^{5'}-H), 7.47-7.52 (m, 2 H, C⁶-H, C⁹-H), 7.98 (d, 1 H, J = 8.7 Hz, C⁴-H), 9.10 (s, 1 H, -NH-). |
| ¹³C-NMR (DMSO-d6) δ in ppm: | 33.5 (C¹⁰), 36.1 (C¹¹), 55.5 (-OCH₃), 114.0 (C⁶), 114.5 (C³), 114.9-117.9 (m, C^{2'}), 115.1 (C¹), 118.7 (C^{4'}), 121.7 (C^{4a}), 127.1 (C¹¹), 129.1 (C^{3'}), 129.1-132.6 (m, -CF₃), 130.7 (C⁹), 133.7 (C^{5a}), 134.6 (C⁴), 139.9 (C^{9a}), 142.7 (C^{1'}), 145.7 (C^{11a}), 147.3 (C²), 158.1 (C⁷), 191.2 (C⁵). |

### Beispiel 26

### 2-(Anilino)-7-methoxydibenzosuberon (10g)

Zur Herstellung der Verbindung 10g nach Methode C werden 0,5 g (1,8 mmol) 2-Chlor-7-methoxydibenzosuberon, 0,2 g (2,1 mmol) Anilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) 0,70 g KO-tert-Bu, 5 ml Toluol und 1 ml tert-BuOH verwendet.
C₂₂H₁₉NO₂ (Mr = 329,40)

| | |
|---|---|
| Ausbeute: | 0,27 g (46%) |
| Schmp.: | 123.5 °C |
| GC | 35,1 min |
| MS m/z (%): | 329 (100, M⁺), 314 (6, M⁺-CH₃), 300 (11), 286 (5), 270 (3), 254 (3), 237 (2,M⁺-Anilin), 208 (10), 194 (4), 180 (4), 165 (11). |
| IR (ATR) | 3329 (N-H), 2992-2853 (C-H)ₐₗᵢₚₕ, 1560 (C=O), 1520, 1494, 1319, 1280, 1262, 1240, 1035, 817,742. |
| ¹H-NMR (DMSO-d6) δ in ppm: | 2.99 (s, 4 H, -CH₂-CH₂), 3.75 (s, 3 H, -OCH₃), 6.93-7.05 (m, 3 H, C³-H, C^{4'}-H, C^{6'}-H), 7.16-7.37 (m, 5 H, C⁶-H, C⁸-H, C⁹-H, C^{3'}-H, C^{5'}-H), 7.95 (d, 1H, J = 8.7 Hz, C⁴-H), 8.81 (s, 1H, -NH-). |
| ¹³C-NMR (DMSO-d6) δ in ppm: | 34.6 (C¹⁰), 36.3 (C¹¹), 55.5 (-OCH₃), 113.1 (C⁶), 114.5 (C³), 114.6 (C¹), 118.6 (C⁸), 120.0 (C^{2'}), 120.0 (C^{6'}), 122.5 (C^{4'}), 127.7 (C^{4a}), 129.7 (C^{3'}), 129.7 (C^{5'}), 130.6 (C⁹), 133.9 (C^{5a}), 134.7 (C⁴), 140.0 (C^{9a}), 141.4 (C^{1'}), 145.9 (C^{11a}), 148.6 (C²), 158.9 (C⁷), 190.9 (C⁵). |

### Beispiel 27

### 2-(2-Methoxyanilino)-7-methoxydibenzosuberon (10h)

Zur Herstellung der Verbindung 10h nach Methode C werden 0,5 g (1,8 mmol) 2-Chlor-7-methoxydibenzosuberon, 0,25 g (2,0 mmol) 2-Methoxyanilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₃H₂₁NO₃ (Mr = 359,43)

| | |
|---|---|
| Ausbeute: | 0,55 g (85%) |
| Schmp.: | 145.0°C |
| GC | 49,7 min |
| MS m/z (%): | 359 (100, M⁺), 344 (5, M⁺-CH₃), 326 (2), 316 (3), 208 (3), 196 (14), 183 (16), 165 (7), 152 (2), 136 (2), 121 (5), 108 (2). |
| IR (ATR) | 3323 (N-H), 3002 (C-H)ₐᵣₒₘ, 1956-2829 (C-H)ₐₗᵢₚₕ, 1557 (C=O), 1520, 1489, 1322, 1289, 1271,1252, 1211, 1111, 1027, 866, 754. |
| ¹H-NMR (DMSO-d6) δ in ppm: | 2.98 (s, 4 H, -CH₂-CH₂), 3.76 (s, 3 H, -OCH₃), 3.79 (s, 3 H, -OCH₃), 6.71 (s, 1 H, C¹-H), 6.83 (d, 1 H, J = 8.9 Hz, C³-H), 6.97-7.09 (m, 3 H, C^{3'}-H, C^{4'}-H, C^{5'}-H), 7.19-7.30 (m, 2 H, C⁸-H, C⁹-H), 7.38 (s, 1 H, C⁶-H), 7.93 (d, 1 H, J = 8.8 Hz, C⁴-H), 8.17 (s, 1 H, -NH-). |
| ¹³C-NMR (DMSO-d6) δ in ppm: | 33.6 (C¹⁰), 36.4 (C¹¹), 55.5 (-OCH₃), 55.8 (-OCH₃), 112.4 (C⁶), 112.8 (C³), 114.1 (C^{3'}), 114.7 (C¹), 118.4 (C⁸), 121.0 (C^{4'}), 122.7 (C^{6'}), 124.6 (C^{5'}), 127.1 (C^{4a}), 129.6 (C^{1'}), 130.5 (C⁹), 133.7 (C^{5a}), 134.5 (C⁴), 140.2 (C^{9a}), 145.6 (C^{11a}), 149.7 (C^{2'}), 152.3 (C²), 158.0 (C⁷), 190.6 (C⁵). |

### Beispiel 28

### 2-(3-Methyl-4-fluoranilino)-7-methoxydibenzosuberon (10i)

Zur Herstellung der Verbindung 10i nach Methode C werden 0,5 g (1,8 mmol) 2-Chlor-7-methoxydibenzosuberon, 0,25 g (2,0 mmol) 3-Methyl, 4-fluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₃H₂₀FNO₂ (Mr = 361,42)

| | |
|---|---|
| Ausbeute: | 0,51 g (78%) |
| Schmp.: | 158,9 °C |
| GC | 39,8 min |
| MS m/z (%): | 361 (100, M⁺), 346 (6, M⁺-CH₃), 333 (9), 237 (2, M⁺-3-CH₃, 4-F-Anilin), 208 (10), 178(3), 165(10). |
| IR (ATR) | 3363 (N-H), 3013 (C-H)ₐᵣₒₘ, 2934-2838 (C-H)ₐₗᵢₚₕ, 1582, 1561 (C=O), 1501, 1286, 1270, 1210, 1048, 971, 862, 806, 768. |
| ¹H-NMR (DMSO-d6) δ in ppm: | 2.21 (s, 3 H, -CH₃), 3.00 (s, 4 H, -CH₂-CH₂), 3.77 (s, 3 H, -OCH₃), 6.75 (s, 1 H, C¹-H), 6.89 (d, 1 H, J = 8.8 Hz, C³-H), 7.00-7.14 (m, 4 H, C⁸-H, C⁹-H, C^{5'}-H, C^{6'}-H), 7.22 (d, 1 H, J = 8.3 Hz, C^{2'}-H), 7.39 (s, 1 H, C⁶-H) 7.96 (d, 2 H, J = 8.8 Hz, C⁴-H), 8.69 (s, 1 H, -NH-). |
| ¹³C-NMR (DMSO-d6) δ in ppm: | 14.7 (-CH₃), 33.5 (C¹⁰), 36.3 (C¹¹), 55.7 (-OCH₃), 112.6 (C⁶), 114.0 (C³), 114.7 (C¹), 115.9 (C^{5'}), 118.5 (C⁸), 119.9 (C^{2'}), 123.8 (C^{6'}), 125.4 (C^{3'}), 127.4 (C^{4a}), 130.5 (C⁹), 134.0 (C^{5a}), 134.5 (C⁴), 137.3 (C^{1'}), 140.1 C^{9a}), 145.9 (C11^{a}), 149.2 (C²), 156.8 (C^{4'}), 158.1 (C⁷), 190.6 (C⁵). |

### Beispiel 29

### 2-(2-Amino-4-trifluormethylanilino)-7-methoxydibenzosuberon (10j)

Zur Herstellung der Verbindung 10j nach Methode C werden 0,5 g (1,8 mmol) 2-Chlor-7-methoxydibenzosuberon, 0,4 g (1,9 mmol) 2-Nitro-4-trifluormethylanilin, 0,05 g Pd(OAc)₂, 0,10 g 2-(Dicyclohexylphosphino)-2'-,4'-, 6'-triisopropyl-biphenyl, 0,70 g KO-tert-Bu, 5 ml Toluol und 1 ml tert-BuOH verwendet. Die Aufreinigung der Nitroverbindung erfolgt durch Umkristallisieren mit MeOH. Ohne weitere Aufreinigung werden 0,5 g der Nitroverbindung, 2,9 g ZnCl₂ x2 H₂O in 20 ml EtOH nach Methode D reduziert.
C₂₃H₁₉F₃N₂O₂ (Mr = 412,42)

| | |
|---|---|
| Schmp.: | 78,9 °C |
| GC | 50,8 min |
| MS m/z (%): | 412 (100, M⁺), 397 (8, M⁺-CH₃), 383 (4), 369 (6), 263 (6), 237 (M⁺-2-NH₂, 4-CF₃-Anilin), 208 (4), 192 (4), 175 (13). |
| IR (ATR): | 3344 (N-H), 2943 (C-H)ₐₗᵢₚₕ, 1567 (C=O), 1520,1496, 1333, 1283, 1260, 1213, 1162, 1147, 1112, 1036. |
| ¹H-NMR (DMSO-d6) δ in ppm: | 3.00 (s, 4 H, -CH₂-CH₂-), 3.77 (s, 3 H, -OCH₃), 5.33 (s, 2 H, -NH₂), 6.62 (s, 1 H, C¹-H), 6.76 (d, 1 H, J = 8.8 Hz, C³-H), 6.85 (d, 1 H, J = 8.1 Hz, C^{6'}-H), 7.01-7.06 (m, 2 H, C^{3'}-H, C^{5'}-H), 7.25-7.21 (m, 2 H, C⁸-H, C⁹-H), 7.39 (s, 1 H, C⁶-H), 7.95 (d, 1 H, J = 8.7 Hz, C⁴-H), 8.09 (s, 1 H, -NH-). |
| ¹³C-NMR (DMSO-d6) δ in ppm: | 33.6 (C¹⁰), 36.3 (C¹¹), 55.5 (-OCH₃), 111.7 (C^{3'}), 123.2 (C^{5'}) 113.1 (C⁶), 114.3 (C³), 114.7 (C¹), 118.6 (C⁸), 125.5 (-CF₃), 124.0 (C^{6'}), 125.8 (C^{4'}), 127.5 (C^{4a}), 129.3 (C^{2'}), 130.6 (C⁹), 133.8 (C^{5a}), 134.6 (C⁴), 140.1 (C^{9a}), 143.2 (C^{1'}), 145.7 (C^{11a}), 149.4 (C²), 158.1 (C⁷), 190.7 (C⁵). |

### Beispiel 30

### 2-(Phenyl)-7-methoxydibenzosuberon (10k)

Eine Mischung von 0,137 g (0,50 mmol) 2-Chlor-7-methoxydibenzosuberon, 0,092mg (0,75 mmol) Phenylboronsäure, 2,2 mg (2 mol%) Pd(OAc)₂, 0,138 g (1,0 mol) K₂CO₃, 4,0 g PEG-400 werden bei 45°C für 5 h gerührt, bis eine vollständige Umsetzung auf der DC beobachtet wird. Zur Mischung werden 15 ml NaOH zugefügt und 4x mit je 15 ml Diethylether extrahiert. Es wir im Vakuum eingeengt. Die Aufreinigung erfolgt über Flash-Chromatographie (SiO₂, Hexan/Ethylacetat 9+1).
C₂₂H₁₈O₂ (Mr = 314,39)

| | |
|---|---|
| Ausbeute: | 0,05 g (32%) |
| Schmp.: | 97,8 °C |
| GC | 24,7 min |
| MS m/z (%): | 314 (100, M⁺), 299 (11, M⁺-CH₃), 285 (20), 271 (11), 255 12), 239 (11), 228 (6), 215 (5), 178 (4), 165 (10), 120 (4). |
| IR (ATR) | 3027 (C-H)ₐᵣₒₘ, 2993-2834 (C-H)ₐₗᵢₚₕ, 1640, 1601 (C=O), 1493,1287 (C-O), 1245, 1048, 977,805, 770, 701. |
| ¹H-NMR (DMSO-d6) δ in ppm: | 2.47-2.51 (m, 4 H, -CH₂-CH₂), 3.78 (s, 3 H, -OCH₃), 7.08-7.12 (m, 1 H, C^{4'}-H), 7.26 (d, 1 H, J = 8.4 Hz, C³-H), 7.40-7.48 (m, 4 H, C¹-H, C⁶-H, C⁸-H, C⁹-H), 7.64 (m, 4 H, C^{2'}-H, C^{3'}-H, C^{5'}-H, C^{6'}-H), 7.97 (d, 1 H, J = 8.8 Hz, C²-H). |
| ¹³C-NMR (DMSO-d6) δ in ppm: | 33.6 (C¹⁰), 35.0 (C¹¹), 55.6 (-OCH₃), 114.2 (C⁶), 119.5 (C⁸), 125.0 (C³), 127.3 (C^{6'}), 127.3 (C^{2'}), 128.1 (C^{4'}), 128.7 (C⁹), 129.4 (C^{5'}), 129.4 (C^{3'}), 131.4 (C¹), 131.5 (C⁴), 134.9 (C^{4a}), 136.9 (C^{5a}), 139.1 (C^{9a}), 139.1 (C^{1'}), 143.4 (C²), 144.3 (C^{11a}), 158.1 (C⁷), 193.7(C⁵). |

### Beispiel 31

### 2-(2, 4-Difluoranilino)-7-methoxydibenzosuberenon (10I)

Zur Herstellung der Verbindung 10I nach Methode C werden 0,5 g (1,8 mmol) 2-Chlor-7-methoxydibenzosuberenon, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70 g KO-tert-Bu, 5 ml Toluol und 1 ml tert-BuOH verwendet.
C₂₂H₁₅F₂NO₂ (Mr = 363,37)

| | |
|---|---|
| Ausbeute: | 0,53 g (81%) |
| Schmp.: | 202,4 °C |
| GC | 36,2 min |
| MS m/z (%): | 363 (100, M⁺), 335 (52), 292 (48), 272 (6), 152 (13), 13), 145 (10), 136 (5). |
| IR (ATR) | 3313 (N-H), 3098-3018 (C-H)ₐᵣₒₘ, 2923-2851 (C-H)ₐₗᵢₚₕ, 1611, 1566 (C=O), 1531, 1498, 1360, 1345, 1280, 1259, 1096, 847, 834 |
| ¹H-NMR (DMSO-d6) δ in ppm: | 3.87 (s, 3 H, -OCH₃), 6.86-6.92 (m, 2 H, C¹-H, C³-H), 6.99-7.11 (m, 3 H, C^{3'}-H, C^{5'}-H, C^{6'}-H), 7.28-7.47 (m, 3 H, C⁸-H, C¹⁰-H, C¹¹-H), 7.63-7.72 (m, 2 H, C⁶-H, C⁹-H), 8.11 (d, 2 H, C⁴-H), 8.65 (s, 1 H, -NH). |
| ¹³C-NMR (DMSO-d6) δ in ppm: | 55.8 (-OCH₃), 105.4 (dd, J = 24.1 Hz, C^{3'}) 112.2 (d, J = 25.7 Hz, C^{5'}), 113.1 (C⁶), 114.0(C¹), 115.9 (C³), 119.9 (C⁸), 125.3 (d, J = 12.1, C^{1'}), 126.3 (d, J = 9.7 Hz, C^{6'}), 128.6 (C^{4a}), 128,7 (C^{5a}), 130,0 (C¹⁰), 131.7 (C¹¹), 133.1 (C⁹), 134.2 (C⁴), 137.6 (C^{9a}), 139.7 (C^{11a}), 149.1 (C²), 155.0 (d, J = 163.7 Hz, C^{4'}) 160.0 (d, J = 163.7 Hz, C^{2'}), 160.0 (C⁷), 188.1 (C⁵). |

### Beispiel 32

### 2-(2, 4-Difluoranilino)-7-(S-1,2-isopropylidenglycer-3-yl)-10,11-dihydro-dibenzo[a,d]-cyclohepten-5-on (14a)

Zur Herstellung der Verbindung nach Methode C werden 0,67 g (1,8 mmol) 2-Chlor-7-(S-1,2-isopropylidenglycer-3-yl)-10,11-dihydrodibenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₇H₂₅F₂₂O₄ (Mr = 465,50)

| | |
|---|---|
| Ausbeute: | 0,57 g(68%) |
| Schmp.: | 114,6 °C |
| GC | 76,6 min |
| MS m/z (%): | 465 (100, M⁺), 450 (6, M⁺-CH₃), 390 (36), 364 (42), 351, (40), 35 (7), 323 (11), 178 (38), 165 (16), 115 (13),101 (31). |
| IR (ATR) | 3352 (N-H), 2997-2944 (C-H)ₐₗᵢₚₕ, 1560, 1509 (C=O), 1289,1262, 1140, 843, 814,802 |
| ¹H-NMR (DMSO-d6) δ in ppm: | 1.29 (s, 3 H, -CH₃), 1.34 (s, 3 H, -CH₃), 3.31 (s, 4 H, -CH₂-CH₂), 3.72-4.45 (m, 5 H, -OCH₂-, -CH-, -CH₂O-), 6.60 (s, 1 H, C¹-H), 6.73 (d, 1 H, J = 9.3, C³-H), 7.00-7.47 (m, 6 H, C⁶-H, C⁸-H, C⁹-H), C^{3'}-H, C^{5'}-H, C^{6'}-H), 7.94 (d, 1 H, J = 8.6, C⁴-H), 8.54 (s, 1 H, -NH-). |
| ¹³C-NMR (DMSO-d6) δ in ppm: | 25,8 (-CH₃), 26.9 (-CH₃), 33.5 (C¹¹), 36.2 (C¹⁰), 66.0 (-CH₂-O-), 69.2 (-O-CH₂-), 74.1 (-OCH-), 105.3 (d, J = 24.3 C^{3'}), 109.2 (-C-), 112.1 (d, J = 21.9 (C^{5'}), 112.6 (C⁶), 113.9 (C³), 115.4 (C¹); 119.1 (C⁸), 125.3 (d, J = 12.0, C^{1'}), 126.4 (d, J = 9.8, C^{6'}),127.7 C^{4a}), 130.6 (C⁹), 133.8 (C^{5a}), 134.9 (C⁴) 140.1 (C^{9a}), 145.7 (C^{11a}), 149.5 (C²), 154.5 (d, J = 152.8, C^{4'}), 157.2 (C⁷); 160.2 (d, J = 169.5, C^{2'}) 190.7 (C⁵). |

### Beispiel 33

### 2-(2, 4-Difluoranilino)-7-(R-1,2-isopropylidenglycer-3-yl)-10,11-dihydro-dibenzo[a,d]-cyclohepten-5-on (14b)

Zur Herstellung der Verbindung nach Methode C werden 0,67 g (1,8 mmol) 2-Chlor-7-7-(R-1,2-isopropylidenglycer-3-yl)-10,11-dihydrodibenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₇H₂₅F₂₂O₄ (Mr = 465,50)

| | |
|---|---|
| Ausbeute: | 65 % |

### Beispiel 34

### 2-(2-Aminoanilino)-7-(S-1,2-isopropylidenglycer-3-yl)-10,11-dihydro-dibenzo[a,d]-cyclohepten-5-on (14c)

Zur Herstellung der Verbindung nach Methode C werden 0,67 g (1,8 mmol) 2-Chlor-7-7-(S-1,2-isopropylidenglycer-3-yl)-10,11-dihydrodibenzo[a,d]-cyclohepten-5-on, 1,0 (9,2 mmol) g 1, 2-Phenylendiamin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₇H₂₈N₂O₄ (Mr = 444,54)

| | |
|---|---|
| ¹H-NMR (DMSO-d6) δ in ppm: | 1.29 (s, 3 H, -CH₃), 1.34 (s, 3 H, -CH₃), 2.96 (s, 4 H, -CH₂-CH₂-), 3.72-3.79 (m, 1 H, -CH₂O-), 4.01-4.31 (m, 3 H, -CH₂O-, -CH₂O-), 4.37-4.42 (m, 1 H, -CH-), 4.83 (s, 2 H, -NH₂), 6.45 (s, 1 H, C¹-H), 6.53-6.63 (m, 2 H, C^{3'}-H, C^{6'}-H), 6.77 (d, 1 H, J = 7.4, C^{4'}-H), 6.90-7.05 (m, 3 H, C³-H, C⁸-H, C^{5'}-H), 7.20 (d, 1 H, J = 8.4, C⁹-H), 7.39 (s, 1 H, C⁶-H), 7.91-7.96 (m, 2 H, C⁴-H, -NH-). |
| ¹³C-NMR (DMSO-d6) δ in ppm: | 25.8 (-CH₃), 26.9 (-CH₃), 33.6 (C¹⁰), 36.6 (C¹¹), 66.1 (C³-Glyceryl), 69.2 (C¹-Glyceryl), 74.1 (C²-Glyceryl), 112.1 (C⁶), 113.1 (C³), 115.5(C¹), 115.8(C^{3'}), 116.8(C^{4'}), 118.8(C⁸). 125.2 (C^{5'}); 126.2(C^{6'}), 126.2(C^{1'}), 126.4(C^{4a}), 130.4(C⁹), 133.9 (C^{5a}), 134.8 (C⁴), 140.3 (C^{9a}), 144.0 (C^{2'}), 145.8 (C^{11a}), 151.1 (C²), 157.2 (C⁷), 190.2 (C⁵). |

### Beispiel 35

### 2-(2-Aminoanilino)-7-(R-1,2-isopropylidenglycer-3-yl)-10,11-dihydro-dibenzo[a,d]-cyclohepten-5-on (14d)

Zur Herstellung der Verbindung nach Methode C werden 0,67 g (1,8 mmol) 2-Chlor-7-(R-1,2-isopropylidenglycer-3-yl)-10,11-dihydrodibenzo[a,d]-cyclohepten-5-on, 1,0 (9,2 mmol) g 1, 2-Phenylendiamin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₇H₂₈N₂O₄ (Mr = 444,54)

| | |
|---|---|
| Ausbeute: | 72 % |
| ¹H-NMR (DMSO-d6) δ in ppm: | 1.29 (s, 3 H, -CH₃), 1.35 (s, 3 H, -CH₃), 2.96 (s, 4 H, -CH₂-CH₂-), 3.76 (q, 1 H, J₁ = 6.5, J₂ = 8.1, -CH₂O-), 3.99-4.12 (m, 3 H, -CH₂O-, -OCH₂-), 4.39 (quin, 1 H, -CH-), 4.83 (s, 2 H, -NH₂), 6.46 (s, 1 H, C'-H), 6.53-6.63 (m, 2 H, C^{3'}-H, C^{6'}-H), 6.77 (d, 1 H, J = 6.99, C^{4'}-H). 6.92 (d, 1 H, J = 7.0, C^{5'}-H), 7.00-7.06 (m, 2 H, C³-H, C⁵-H), 7.20 (d, 1 H, J = 8.2, C⁹-H), 7.40 (s, 1 H, C⁶-H), 7.92-7.96 (m, 2 H, C⁴-H, -NH-). |
| ¹³C-NMR (DMSO-d6) δ in ppm: | 25.8 (-CH₃), 26.9 (-CH₃), 33.6 (C¹⁰), 36.6 (C¹¹), 66.1 (C³-Glyceryl), 69.2 (C¹-Glyceryl), 74.1 (C²-Glyceryl), 112.1 (C⁶), 113.1 (C³), 115.5 (C¹), 115.8 (C^{3'}), 116.6 (C^{4'}), 118.8 (C⁸), 125.2 (C^{5'}), 126.2 (C^{1'}), 126.2 (C^{1'}), 126.4 (C^{4a}), 130.4 (C⁹), 133.9 (C^{5a}), 134.8 (C⁴), 140.3 (C^{9a}), 144.0 (C^{2'}), 145.7 (C^{11a}), 151.1 (C²), 157.2 (C⁷), 190.2 (C⁵). |

### Beispiel 36

### 2-(2, 4-Difluoranilino)-7-[2R-, 3-dihydroxypropoxy]-10,11-dihydro-dibenzo[a,d]-cyclohepten-5-on (14 e)

Zu einer Lösung von 1,0 g (2,1 mmol) 2-(2, 4-Difluoranilino)-7-(S-1,2-isopropylidenglycer-3-yl)-10,11-dihydrodibenzo[a,d]-cyclohepten-5-on. in 40 ml MeOH werden 10 ml H₂O und 0,25 g (1,3 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/Ethylacetat (1:1) gelöst und 1 h lang gerührt. Es wird abfiltriert und i. Vak. eingeengt.
C₂₄H₂₁F₂NO₄ (Mr = 425,44)

| | |
|---|---|
| Ausbeute: | 0,85 g (95%) |
| Schmp.: | 130,3°C |
| IR (ATR) | 3305 (N-H), 2934 (C-H)ₐₗᵢₚₕ, 1629, 1607, 1569 (C=O), 1510, 1327, 1278, 1218, 1097,847,781. |
| ¹H-NMR (DMSO-d6) δ in ppm: | 3.32 (s, 4 H, -CH₂-CH₂), 3.40-3.46 (m, 2 H, -CH₂OH), 3.77-4.05 (m, 3 H, -CHOH-, -OCH₂-), 4.66 (t, 1H, J = 5.7, -CH-OH), 4.94 (d, 1 H, J = 4.9, -CH₂-OH, 6.60 (s, 1 H, C¹-H), 6.73 (d, 1 H, J = 8.9, C³-H), 7.01-7.10 (m, 2 h, C^{3'}-H, C^{6'}-H), 7.22 (d, 1 H, J = 8.3, C^{4'}-H), 7.30-7.47 (m, 3 H, C⁶-H, C⁸-H, C⁹-H), 7.94 (d, 1 H, J = 8.8, C⁴-H), 8.54 (s, 1H, -NH-). |
| ¹³C-NMR (DMSO-d6) δ in ppm: | 33.6 (C¹¹), 36.3 (C¹⁰), 63.1 (-CH₂-OH), 70.2 (-O-CH₂-), 70.3 (-OCH-), 105.3 (d, J = 24.3, C³), 112.1 (d, J = 21.9, C^{5'}). 112.6 (C⁶), 113.8 (C³), 115.4 (C¹), 119.1 (C⁸), 125.3 (d, J = 12.0, C^{1'}), 126.4 (d, J = 9.8, C^{6'}), 127.7 (C^{4a}), 130.6 (C⁹), 133.8 (C^{5a}), 134.6 (C⁴), 139.9 (C^{9a}), 145.7 (C^{11a}), 149.4 (C²), 154_{.}5 (d, J =152.8, C^{4'}), 157.6 (C⁷), 160.2 (d, J = 169.5, C^{2'}), 190.8 (C⁵). |

### Beispiel 37

### 2-(2, 4-Difluoranilino)-7-[2S-, 3-dihydroxypropoxy]-10,11-dihydro-dibenzo[a,d]-cyclohepten-5-on (14 f)

Zu einer Lösung von 1,0 g (2,1 mmol) 2-(2, 4-Difluoranilino)-7-(R-1,2-isopropylidenglycer-3-yl)-10,11-dihydrodibenzo[a,d]-cyclohepten-5-on. in 40 ml MeOH werden 10 ml H₂O und 0,25 g (1,3 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und im Vakuum eingeengt. Das Diol fällt als weißer Feststoff aus. Der Feststoff wird in Hexan/ Ethylacetat (1:1) gelöst und 1 h lang gerührt. Es wird abfiltriert und i. Vak. eingeengt.
C₂₄H₂₁F₂NO₄ (Mr = 425,44)

| | |
|---|---|
| Ausbeute: | 95 % |
| ¹H-NMR (DMSO-d6) δ in ppm: | 2.99 (s, 4 H, -CH₂-CH₂-), 3.41-3.46 (m, 2 H, -CH₂O-, -CH-), 3.77-4.03 (m, 3 H, -CH₂O-, CH₂OH), 4.65 (t, 1 H, J = 5.7, -OH), 4.93 (d, 1 H, J = 4.8, -OH), 6.60 (s, 1 H, C¹-H), 6.73 (d, 1 H, J= 8.6, C³-H), 7.01-7.41 (m, 6 H, C⁶-H, C⁸-H, C⁹-H, C^{3'}-H, C^{5'}-H, C^{6'}-H), 7.94 (d, 1 H, J = 8.7, C⁴-H), 8.53 (s, 1 H, -NH-). |
| ¹³C-NMR (DMSO-d6) δ in ppm: | 33.6 (C¹⁰), 36.3 (C¹¹), 63.1 (C³-Propoxy), 70.2 (C¹-Propoxy), 70.3 (C²-Propoxy), 105.3 (dd, 1 C, J₁ = 24.2, J₂ = 26.6, C^{3'}), 112.2 (dd, 1 C, J₁ = 3.6, J₂ = 22.0, C^{5'}), 112.6 (C⁶), 113.8 (C³), 115.4 (C¹),119.1 (C⁸), 125.1-125.3 (m, 1 C, C^{1'}),126.4-126.6 (m, 1C, C^{6'}), 127.8 (C^{4a}), 130.6 (C⁹), 133.8 (C^{5a}), 134.6 (C⁴), 139.9 (C^{9a}), 145.7 (C^{11a}), 149.4 (C²), 153.4-156.5 (m, 1 C, C^{4'}), 157.3 (C⁷), 158.3-161.3 (m, 1 C, C^{2'}), 190.8 (C⁵). |

### Beispiel 38

### 2-(2-Aminoanilino-7-[2R-, 3-dihydroxypropoxy] -10,11-dihydro-dibenzo[a,d]-cyclohepten-5-on (14 g)

Zu einer Lösung von 1,0 g (2,1 mmol) 2-(2-Aminoanilino)-7-(S-1,2-isopropylidenglycer-3-yl)-10,11-dihydrodibenzo[a,d]-cyclohepten-5-on in 40 ml MeOH werden 10 ml H₂O und 0,50 g (2,6 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/Ethylacetat (1:1) gelöst und 1 h lang gerührt. Es wird abfiltriert und i. Vak. eingeengt.
C₂₄H₂₄N₂O₄ (Mr = 404,47)

| | |
|---|---|
| Ausbeute: | 96 % |
| ¹H-NMR (DMSO-d6) δ in ppm: | 2.96 (s, 4 H, -CH₂-CH₂-), 3.77-3.82 (m, 2 H, -OCH₂-, -CH-), 3.86-4.02 (m, 3 H, -OCH₂-, -CH₂O-), 4.65 (t, 1 H, J = 5.2, -OH), 4.83 (s, 2 H, -NH₂), 4.93 (d, 1 H, J = 4.5, -OH), 6.45 (s, 1 H, C¹-H), 6.54-6.62 (m, 2 H, C^{3'}-H, C^{6'}-H), 6.77 (d, 1 H, J = 7.2, C^{4'}-H), 6.90-7.03 (m, 3 H, C³-H, C⁸-H, C^{5'}-H), 7.20 (d, 1 H, J = 8.0, C⁹-H), 7.40 (s, 1 H, C⁶-H), 7.91-7.95 (m, 2 H, C⁴-H, -NH-). |
| ¹³C-NMR (DMSO-d6) δ in ppm: | 33.6 (C¹⁰), 36.6 (C¹¹), 63.1 (C³-Propoxy), 70.2 (C¹-Propoxy), 70.3 (C²-Propoxy), 112.1 (C⁶), 113.1 (C³), 115.5 (C¹ 115.8 (C^{3'}), 116.8 (C^{4'}), 118.9 (C⁸), 125.2 (C^{5'}), 126.2 (C^{6'}), 126.3 (C^{1'}), 126.4 (C^{4a}), 130.4 (C⁹), 134.0 (C^{5a}), 134.5 (C⁴), 140.2 (C^{9a}), 144.0 (C²), 145.8 (C^{11a}), 151.1 (C²), 157.6 (C⁷), 190.2 (C⁵). |

### Beispiel 39

### 2-(2-Aminoanilino-7-[2S-, 3-dihydroxypropoxy]-10,11-dihydro-dibenzo[a,d]-cyclohepten-5-on (14 h)

Zu einer Lösung von 1,0 g (2,1 mmol) 2-(2-Aminoanilino)-7-(R-1,2-isopropylidenglycer-3-yl)-10,11-dihydrodibenzo[a,d]-cyclohepten-5-on in 40 ml MeOH werden 10 ml H₂O und 0,50 g (2,6 mmol) p-Toluolsulfonsäure zugefügt. Die Lösung wird unter Argon-Schutzgasatmosphäre auf 50°C erhitzt. Nach 6 h wird die Lösung auf Raumtemperatur abgekühlt und im Vakuum eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt. Das Diol fällt als weißer Feststoff aus. Der Feststoff wird in Hexan/Ethylacetat (1:1) gelöst und 1 h lang gerührt. Es wird abfiltriert und i. Vak. eingeengt.
C₂₄H₂₄N₂O₄ (Mr = 404,47)

| | |
|---|---|
| Ausbeute: | 95 % |
| ¹H-NMR (DMSO-d6) δ in ppm: | 2.96 (s, 4 H, -CH₂-CH₂-), 3.77-3.90 (m, 2 H, -CH₂-O-, -CH-), 3.98-4.03 (m, 2 H, CH₂OH, -CH₂O-), 4.66 (s, 1 H, -OH), 4.83 (s, 2 H, -NH₂), 4.93 (s, 1 H, -OH), 6.45 (s, 1 H, C¹-H), 6.53-6.63 (m, 2 H, C^{3'}-H, C^{6'}-H), 6.77 (d, 1 H, J = 7.4, C^{4'} -H), 6.90-7.03 (m, 3 H, C⁸-H, C³-H, C^{5'}-H), 7.20 (d, 1 H, J = 8.1, C⁹-H), 7.40 (s, 1 H, C⁶-H), 7.91-7.95, (m, 2 H, C⁴-H, -NH-). |
| ¹³C-NMR (DMSO-d6) δ in ppm: | 33.6 (C¹⁰), 36.6 (C¹¹), 65.3 (C³-Propoxy), 70.2 (C¹-Propoxy), 70.3 (C²-propoxy), 112.1 (C⁶), 113.1 (C³), 115.5 (C¹), 115.8 (C^{3'}), 116.8 (C^{4'}), 118.9 (C⁸), 125.2 (C^{5'}), 126.2 (C^{6'}), 126.3 (C¹ ),126.4 (C^{4a}), 130.4 (C⁹), 134.0 (C^{5a}), 134.5 (C⁴), 140.2 (C^{9a}), 144.0 (C^{2'}), 145.8 (C^{11a}), 151.1 (C²), 157.6 (C⁷), 190.2 (C⁵). |

### Beispiel 40

### 2-(2, 4-Difluoranilino)-7-(2-hydroxy-ethoxy)-10,11-dihydro-dibenzo[a,d]-cyclohepten-5-on (14i)

Zur Herstellung der Verbindung nach Methode C werden 0,62 g (1,8 mmol) 2-Chlor-7-(2-acetoxyethoxy)-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₃H₁₉F₂NO₃ (Mr = 395,41)

| | |
|---|---|
| Ausbeute: | 34 % |
| ¹H-NMR (DMSO-d6) δ in ppm: | 2.99 (s, 4 H, -CH₂-CH₂-), 3.66-3.74 (m, 2 H, -CH₂-OH), 3.97-4.02 (m, 2 H, -OCH₂-), 4.85 (t, 1 H, J = 5.5, -OH), 6.60 (s, 1 H, C¹-H), 6.73 (d, 1 H, J = 8.6, C³-H), 7.01-7.42 (m, 6 H, C⁶-H, C⁸-H, C⁹-H, C^{3'}-H, C^{5'}-H, C^{6'}-H), 7.94 (d, 1 H, J = 8.8, C⁴-H), 8.53 (s, 1 H, -NH-). |
| ¹³C-NMR (DMSO-d6) δ in ppm: | 33.6 (C¹⁰), 36.2 (C¹¹), 59.9 (C²-Ethoxy), 70.0 (C¹-Ethoxy), 105.3 (t, 1 C, J = 24.2, C^{3'}), 112-0-112.4 (m, 1 C, C^{5'}), 112.6 (C⁶), 113.8 (C³), 115.4 (C¹), 119.1 (C⁸), 125.1-125.4 (m, 1 C, C¹), 126.3-126.5(m, 1 C, C⁶), 127.7 (C^{4a}), 130.6 (C⁹), 133.8 (C^{5a}), 134.6 (C⁴), 140.0 (C^{9a}), 145.7 (C^{11a}), 149.4 (C²), 153.4-156.5 (m, 1 C,C⁴), 149.4 (C⁷), 158.3-161.3 (m, 1 C, C^{2'}), 190.8 (C⁵). |

### Beispiel 41

### 2-(2, 4-Difluoranilino)-7-(3-hydroxy-propoxy)-10,11-dihydro-dibenzo[a,d]-cyclohepten-5-on (14 j)

Zur Herstellung der Verbindung nach Methode C werden 0,65 g (1,8 mmol) 2-Chlor-7-(3-acetoxypropoxy)-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₄H₂₁F₂NO₃ (Mr = 409,44)

| | |
|---|---|
| Ausbeute: | 40 % |
| ¹H-NMR (DMSO-d6) δ in ppm: | 1.85 (quin, 2 H, J = 6.3, -CH₂-), 2.99 (s, 4 H, -CH₂-CH₂-), 3.55 (q, 2 H, J = 6.1, -CH₂-OH), 4.04 (t, 2 H, J = 6.4 -OCH₂-), 4.54 (t, 1 H, J = 5.1, -OH), 6.60 (s, 1 H, C¹-H), 6.73 (d, 1 H, J = 8.6, C³-H), 7.00-7.42 (m, 6 H, C⁶-H, C⁸-H, C⁹-H, C^{3'}-H, C^{5'}-H, C^{6'}-H), 7.94 (d, 1 H, J = 8.8, C⁴-H), 8.54 (s, 1 H, -NH-). |
| ¹³C-NMR (DMSO-d6) δ in ppm: | 32.5 (C²-Propoxy), 33.6 (C¹⁰), 36.2 (C¹¹), 57.6 (C³-Propoxy), 65.1 (C¹-Propoxy),105.3 (t, 1 C, J = 24.2, C^{3'}), 11.9-112.4 (m, 1 C, C^{5'}), 112.6 (C⁶), 113.8 (C³), 115.3 (C¹), 119.1 (C⁸), 125.1-125.4 (m, 1 C, C^{1'}), 126.3-126.6 (m, 1 C, C^{6'}), 127.8 (C^{4a}), 130.6 (C⁹), 133.8 (C^{5a}), 134.5 (C⁴), 140.0 (C^{9a}), 145.7 (C^{11a}), 149.4(C²), 153.4-156.5 (m, 1 C, C^{4'} ), 157.5 (C⁷), 158.3-161.3 (m, 1 C, C^{2'}), 190.8 (C⁵). |

### Beispiel 42

### 2-(2, 4-Difluoranilino)-7-(2-morpholin-4-yl-ethoxy)-10,11-dihydro-dibenzo[a,d]-cyclohepten-5-on (14l)

Zur Herstellung der Verbindung nach Methode C werden 0,67 g (1,8 mmol) 2-Chlor-7-(2-morpholin-4-yl-ethoxy-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₄H₂₁F₂NO₄ (Mr = 425,44)

| | |
|---|---|
| Ausbeute: | 76 % |
| ¹H-NMR (DMSO-d6) δ in ppm: | 2.42-2.50 (m, 4 H, -CH₂-N-CH₂-), 2.67 (t, 2 H, -CH₂N-), 2.99 (s, 4 H, -CH₂-CH₂-), 3.56 (t, 4 H, J = 4.6, -CH₂-O-CH₂-), 4.09 (t, 2 H, J = 5.7, -CH₂O-), 6.61 (s, 1 H, C⁶-H), 6.73 (d, 1 H, J = 8.9, C³-H), 7.01-7.43 (m, 6 H, C¹-H, C⁸-H, C⁹-H, C^{3'}-H, C^{5'}-H, C^{6'}-H), 7.94 (d, 1 H, J = 8.8, C⁴-H); 8.52 (s, 1 H, -NH-). |
| ¹³C-NMR (DMSO-d6) δ in ppm: | 33.5 (C¹⁰), 36.2, 54.0 (2 C, C²/C⁶-Morpholinyl), 57.3 (C²-Ethoxy), 65.9 (C¹-Ethoxy), 66.5 (2 C, C³/C⁵-Morpholinyl), 105,3 (dd, 1 C, J₁ = 24.1, J₂ = 26.7, C³), 111.9-112.4 (m, 1 C, C^{5'}), 112.6 (C⁶), 113.9 (C³), 115.5 (C¹), 119.1 (C⁸), 125.2-125.4 (m, 1 C, C¹), 126.3-126.5 (m, 1 C, C⁶), 127.8 (C^{4a}), 130.6 (C⁹), 133.8 (C^{5a}), 134.6 (C⁴), 140.0 (C^{9a}), 145.7 (C^{11a}), 149.4 (C²), 154.9 (dd, 1 C, J₁ = 12.6, J₂ = 142.3, C⁴), 157.3 (C⁷), 159.8 (dd, 1 C, J₁ = 11.5, J₂ = 138.1, C^{2'}), 190.8 (C⁵). |

### Beispiel 43

### 2-(2-Aminoanilino)-7-(2-morpholin-4-yl-ethoxy)-10,11-dihydro-dibenzo[a,d]-cyclohepten-5-on (14m)

Zur Herstellung der Verbindung nach Methode C werden 0,67 g (1,8 mmol) 2-Chlor-7-(2-morpholin-4-yl-ethoxy)-10,11-dihydrodbenzo[a,d]-cyclohepten-5-on, 1,0 (9,2 mmol) g 1, 2-Phenylendiamin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₇H₂₉N₃O₃ (Mr = 443,55))

| | |
|---|---|
| Ausbeute: | 74 % |
| ¹H-NMR (DMSO-d6) δ in ppm: | 2.30-2.50 (m, 4 H, -CH₂-N-CH₂-), 2.67 (t, 2 H, J = 5.5, -CH₂-N-), 2.96 (s, 4 H, -CH₂-CH₂-), 3.56 (t, 4 H, J = 4.3, -CH₂-O-CH₂-), 4.09 (t, 2 H, J = 5.4, -CH₂O-), 4.83 (s, 2 H, -NH₂), 6.45 (s, 1 H, C¹-H), 6.54-6.63 (m, 2 H, C^{3'}-H, C^{6'}-H), 6.77 (d, 1 H, J = 7.6, C^{4'}-H), 6.92 (d, 1 H, J = 7.6, C^{5'}-H), 6.99-7.03 (m, 2 H, C³-H, C⁸-H), 7.19 (d, 1 H, J = 8.3, C⁹-H), 7.38 (s, 1 H, C⁶-H), 7.91-7.95 (m, 2 H, C⁴-H, -NH-). |
| ¹³C-NMR (DMSO-d6) δ in ppm: | 33.6 (C¹⁰), 36.6 (C¹¹), 54.0 (2 C, C²/C⁶-Morpholinyl), 57.3 (C²-Ethoxy), 65.9 (C¹-Ethoxy), 66.5 (2 C, C³/C⁵-Morpholinyl), 112.2 (C⁶), 113.1 (C³), 115.6 (C¹), 115.8 (C^{3'}), 116.8 (C^{4'}), 118.8 (C⁸), 125.2 (C^{5'}), 126.1 (C^{6'}), 126.2 (C^{1'}), 126.4 (C^{4a}), 130.4 (C⁹), 133.9 (C^{5a}), 134.6 (C⁴), 140.3 (C^{9a}), 144.0 (C^{2'}), 145.7 (C^{11a}), 151.1 (C²), 157.2 (C⁷), 190.2 (C⁵). |

### Beispiel 44

### 2-(2, 4-Difluoranilino)-7-(2-tetrahydropyran-4-yl-oxy)-10,11-dihydro-dibenzo[a,d]-cyclohepten-5-on (14k)

Zur Herstellung der Verbindung nach Methode C werden 0,62 g (1,8 mmol) 2-Chlor-7-(2-tetrahydropyran-4-yl-oxy)-10,11-dihydrodbenzo[a,d]-cyclohepten-5-on, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.
C₂₆H₂₃F₂NO₃ (Mr = 435,47)

| | |
|---|---|
| Ausbeute: | 56 % |
| ¹H-NMR (DMSO-d6) δ in ppm: | 1.52-1.61 (m, 2 H, C³/C⁵-Tetrahydropyranyl), 1.92-1.98 (m, 2 H, C³/C⁵-Tetrahydropyranyl), 1.99 (s, 4 H, -CH₂-CH₂), 3.41-3.53 (m, 2 H, C²/C⁶-Tetrahydropyranyl), 3.78-3.88 (m, 2 H, C²/C⁶-Tetrahydropyranyl), 4.51-4.67 (m, 1 H, C⁴-Tetrahydropyranyl), 6.60 (s, 1 H, C⁶-H), 6.73 (d, 1 H, J = 8.8, C³-H), 7.04-7.41 (C¹-H, C⁸-H, C⁹-H, C^{3'}-H, C^{5'}-H, C^{6'}-H), 7.94 (d, 1 H, C⁴-H), 8.53 (s, 1 H, -NH-). |
| ¹³C-NMR (DMSO-d6) δ in ppm: | 32.1 (2 C, C³/C⁵-Tetrahydropyranyl), 33.5 (C¹⁰), 36.2 (C¹¹), 64.8 (2 C, C²/C⁶-Tetrahydropyranyl), 171.9 (C⁴-Tetrahyropyranl), 105.3 (dd, 1 C, J₁ = 24.2, J₂ = 26.7, C^{3'}) 112.2 (dd, 1 C, J₁ = 3.7, J₂ = 22.1, C^{5'}), 112.6 (C⁶), 113.9 (C³), 117.1 (C¹), 120.4 (C⁸), 125.2-125.4 (m, 1 C, C^{1'}), 126.4 (dd, 1 C, J₁ = 3.2, J₂ = 9.7, C^{6'}) 127.7 (C^{4a}), 130.6 (C⁹), 133.8 (C^{5a}), 134.7 (C⁴), 140.2 (C^{9a}, 145.7 (C^{11a}), 149.5 (C²), 154.9 (dd, 1 C, J₁ = 11.8, J₂ = 143.5, C^{4'}) 158.7 (C⁷), 159.8 (dd, 1 C, J₁ = 11.9, J₂ = 137.6, C^{2'}), 190.8 (C⁵). |

### B. Substitution an Position 8 oder 9

### Methode H

### Bromierung der Methoxymethylbenzoesäure

Die Methoxymethylbenzoesäure und NBS werden in Chlorbenzol gegeben und die Reaktionsmischung auf 70°C erhitzt.

### Variante 1

Nach Zutropfen von AIBN/Chlorbenzol wird weitere 2 h 45 min bei 100°C gerührt. Dann wird die Lösung abgekühlt und das Chlorbenzol entfernt. Das Produkt wird ohne weitere Aufreinigung weiter verwendet. Das Succinimid wird in der folgenden Stufe entfernt.

### Variante 2

Nach Zutropfen von AIBN/Chlorbenzol wird weitere 1 h 30 min bei 70-75°C gerührt. Dann wird die Lösung abgekühlt und das Chlorbenzol entfernt. Das Produkt wird ohne weitere Aufreinigung weiter verwendet. Das Succinimid wird in der folgenden Stufe entfernt.

### Methode I

### Wittig-Reaktion der Brommethylmethoxybenzoesäure mit 3-Chlorbenzaldehyd

Triphenylphosphin wird in 150 ml MeOH gegeben, unter Rühren die Brommethylmethoxybenzoesäure (gelöst in MeOH) zugetropft und 2 Stunden unter Rückfluss erhitzt. Anschließend tropft man langsam die NaOMe-Lösung (30% in MeOH) zu. Nach beendigtem Zutropfen wird noch 15 min gewartet, dann wird der 3-Chlorbenzaldehyd zugegeben. Es folgen 6 h Refluxieren. Danach wird der Reaktionsansatz auf ein gerührtes Gemisch aus 75 g Eis und 175 ml Wasser gegossen. Im nächsten Schritt wird 3x mit je 200 ml Dichlormethan gewaschen. Anschließend wird die Wasserphase unter Eiskühlung mit konz. HCl stark angesäuert. Daraufhin bildet sich ein ölig-halbfester Niederschlag, der im Scheidetrichter abgetrennt werden kann. Das restliche Produkt wird mit Dichlormethan extrahiert.

### Methode J

### Reduktion der Chlorphenylvinylmethoxybenzoesäure

### Variante 1

Die Chlorphenylvinylmethoxybenzoesäure wird in Methanol gelöst und mit Salzsäure versetzt. Anschließend wird Pd / BaSO₄ (5 %) zugefügt. Nach Evakuieren wird mit 4 l H₂ gespült. Die Lösung wird unter H₂-Atmosphäre 2 Tage bei Raumtemperatur gerührt. Anschließend wird von der Kohle abfiltriert und im Vakuum eingeengt. Das entstehende Öl wird mit Dichlormethan in einen Scheidetrichter überführt und die organische Phase mit Wasser gewaschen. Nach Einengen erhält man ein hell-gelbes Öl erhält, das beim Stehen lassen kristallisiert.

### Variante 2

Die Chlorphenylvinylmethoxybenzoesäure wird im angegebenen Lösungsmittelgemisch gelöst. Anschließend wird Pd / C (10 %) zugefügt. Nach Evakuieren wird mit 4 Liter H₂ gespült. Die Lösung wird unter H₂-Atmosphäre (weiterer H₂-Ballon) 4 Stunden bei Raumtemperatur gerührt. Anschließend wird von der Kohle abfiltriert und im Vakuum eingeengt.

### Methode K

### Ringschluss mittels Friedel-Crafts Acylierung

Die Chlorphenylethylmethoxybenzoesäure wird in Dichlormethan (trocken) suspendiert und der Ansatz mit Argon gespült. Dann wird der Ansatz auf Rückflusstemperatur erhitzt und unter Rühren innerhalb einer Stunde der Lösung Thionylchlorid in Dichlormethan zugetropft. Anschließend wird noch eine weiter Stunde gerührt.

Der Reaktionsansatz wird auf Raumtemperatur abgekühlt und innerhalb von 30 min wird wasserfreies AlCl₃ in zugegeben. Danach wird noch weitere 45 min gerührt und das Reaktionsgemisch dann unter Rühren auf eine Mischung aus 90 g Eis und 150 ml Wasser gegossen. Das Hydrolysegemisch wird ca. 30 min gerührt. Anschließend werden die beiden Phasen getrennt. Die organische Phase wird im Vakuum eingeengt

### Methode L

### Methoxyspaltung am Ringgerüst

Das Methoxydihydrodibenzosuberenon wird in Eisessig gelöst und HBr (48 %, wässrig) zugefügt. Es wird 3 h bei 120-130°C erhitzt.

### Variante 1

Nach Hydrolyse mit ca. 150 g Eis wird vom Niederschlag abfiltriert.

### Variante 2

Nach Hydrolyse mit ca. 150 g Eis wird vom Niederschlag abfiltriert und nach Waschen mit Wasser der Filterrückstand wieder in Dichlormethan gelöst. Anschließend wird eingeengt.

### Methode M

### Bildung des Acetals

Zu der Lösung des Dihydrodibenzosuberenons in DMF wird zuerst der Toluen-4-sulfonsäure-2,2-dimethyl-[1,3]dioxolan-4-ylmethylester und anschließend K₂CO₃ hinzugefügt. Die Reaktionsmischung wird unter Argon auf 80°C erwärmt und nach 24 h wieder auf Raumtemperatur abgekühlt. Anschließend wird der Ansatz in 50 ml Wasser aufgenommen und mit Ethylacetat extrahiert, die vereinigten organischen Phasen werden mit NaOH gewaschen und über Na₂SO₄ getrocknet. Dann wird i.Vak. eingeengt, wobei man ein braunes Öl erhält, das über Flash (SiO₂, Hexan/Ethylacetat) gereinigt wird. Man erhält das reine Acetal.

### Methode N

### Spaltung des Acetals

Zu einer Lösung des Acetals in MeOH wird H₂O und p-Toluensulfonsäure zugefügt. Die Lösung wird unter Schutzgas auf 50°C erhitzt. Nach 6 h wird die Lösung auf Raumtemperatur abgekühlt und i. Vak. eingeengt. Man erhält ein gelbes Öl, das in Ethylacetat und 5%iger Na₂HCO₃- Lösung (50 ml) wieder gelöst wird. Die organisch Phase wird abgetrennt und i. Vak. eingeengt.

### Methode O

### Einführung von Resten über Buchwald - Hartwig - Reaktion

Die Mischung aus Chlordihydrodibenzosuberenon, Pd(OAc)₂, Phosphinligand (2-(Dicyclohexylphosphino)-2',4',6'-triisopropylbiphenyl), NaOtert-Bu, Aminosubstituent, Toluen und tert-BuOH wird bei 100°C 3-5 h gerührt. Danach wird abgekühlt, die Mischung in 150 ml Wasser aufgenommen und mit Diethylether extrahiert. Die vereinigten org. Phasen werden i. Vak. eingeengt und säulenchromatographisch (Flash; SiO₂; Hexan/Ethylacetat) gereinigt.

### Methode P

### Substitution mit Alkoholen

Unter Schutzgas wird eine Lösung aus Chlorhydroxydibenzodihydrodibenzosuberenon, Alkohol, Triphenylphosphin und THF hergestellt. Nach Zutropfen des Carboxylats wird 1h bei 0°C gerührt. Die Reaktion wird bis zur Beendigung bei Raumtemperatur fortgeführt. Danach wird i. Vak. eingeengt.

### Methode Q

### Substitution mit Alkylhalogeniden

Eine Mischung aus Phenol, Alkylhalogenid und K₂CO₃ wird im angegebenen Lösungsmittel auf 80°C erhitzt, 3 h gerührt, auf RT abgekühlt und wieder in EA und Wasser gelöst. Die org. Phase wird mit Wasser und Lauge gewaschen, über Na₂SO₄ getrocknet und i. Vakuum eingeengt.

### Methode R

### Herstellung des Triflats

Zu einer gerührten Lösung des Phenols in Pyridin wird bei 0 °C (Eisbad) unter Argon langsam Tf₂O über 0,5 h hinzugefügt. Die Reaktionsmischung wird bei Raumtemperatur über Nacht gerührt, mit EA extrahiert und die organische Phase mit 10%iger HCl und 5%iger NaHCO₃-Lösung gewaschen, über Na₂SO₄ getrocknet, abfiltriert und eingeengt.

### Substitution in Position 8 - Verbindungen und Vorstufen

### Beispiel 45

### 4-Methoxy-2-methylbenzoesäure (6)

Zur Synthese der Titelverbindung werden nach Methode H (Variante 2) 6,64 g (0,040 mol) 4-Methoxy-2-methylbenzoesäure, 7,1 g (0,040 mol) NBS und 0,25g AIBN in 150 ml Chlorbenzol umgesetzt.
C₉H₁₀O₃ (Mr = 166,18)
¹H-NMR (DMSO-d6) δ in ppm:
¹³C-NMR (DMSO-d6) δ in ppm:

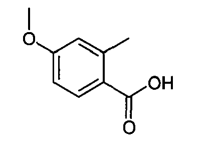

### Beispiel 46

### 2-[2-(3-Chlorphenyl)-vinyl]-4-methoxybenzoesäure (7)

Zur Synthese der Titelverbindung werden nach Methode I 45,6 g ( 0,186 mol) 2-Bromomethyl-4-methoxybenzoesäure, 48,8 g (0,186 mol) Triphenylphosphin, 26,1 g (0,186 mol) 3-Chlorbenzaldehyd und 84,0 g (0,435 mol) NaOMe (28%) in 150 ml Methanol umgesetzt.
Die Aufreinigung erfolgt durch Umkristallisation aus Methanol bei 4°C.
C₁₆H₁₃ClO₃ (Mr = 288,73)

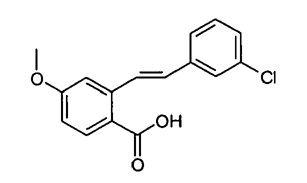

| | |
|---|---|
| ¹H-NMR (DMSO-d6) δ in ppm: | 3,86 (s, 3H, -OCH₃), 6,96 (d, 1H, J=8,78 Hz, C⁵-H), 7,12-7,59 (m, 6H, C³-H und C^{4'}-/C^{5'}-/C^{6'}-H und C¹-/C²-H Vinyl), 7,89 (d, 1H, J=8,72 Hz, C^{2'}), 8,01-8,09 (m, 1H, C⁶). |
| ¹³C-NMR (DMSO-d6) δ in ppm: | 55,9 (-OCH₃), 112,1 (C³), 113,9 (C⁵), 122,0 (C¹), 125,5 (C^{6'}), 126,7 (C^{2'}), 127,9 (C^{4'}), 129,6 (C¹ Vinyl), 129,6 (C² Vinyl), 131,0 (C^{5'}), 133,3 (C⁶), 133,9 (C^{3'}), 139,9 (C^{1'}), 140,7 (C¹), 162,3 (C⁴), 168,2 (-COOH). |

### Beispiel 47

### 2-[2-(3-Chlorphenyl)-ethyl]-4-methoxybenzoesäure (8)

Zur Synthese der Titelverbindung werden nach Methode J (Variante 2) 6,0 g 2-[2-(3-Chlorphenyl)-vinyl]-4-methoxybenzoesäure, 0,6 g Pd / C (10 %) und 4I Wasserstoff eingesetzt. Das Lösungsmittelgemisch besteht aus 75 ml Acetonitril, 75 ml Ethylacetat und 20 ml Methanol.
C₁₆H₁₅ClO₃ (Mr = 290,75)

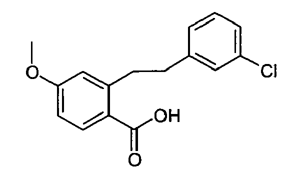

### Beispiel 48

### 2-Chlor-8-methoxy-10,11-dihydrodibenzo[a,d]cyclohepten-5-on (9)

Zur Synthese der Titelverbindung werden nach Methode K 16,6 g (0,057 mol) [2-(3-Chlorphenyl)-ethyl]-4-methoxybenzoesäure, 7,25 g (0,06 mol) SOCl₂, 250 ml Dichlormethan, und 10 g AlCl₃ (0,075 mol) eingesetzt.
Die Aufreinigung erfolgt säulenchromatographisch (Flash; SiO₂; Hexan 90% / Ethylacetat 10%).
C₁₆H₁₃ClO₂ (Mr = 272,73)

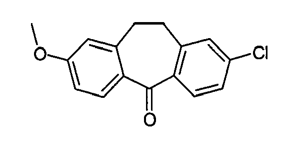

| | |
|---|---|
| MS m/z (%): | 274/272 (34/100, M⁺), 259/257 (2/5, M⁺-CH₃), 246/244 (9/26, M⁺-CO), 237 (19, M⁺-Cl), 231/229 (5/17, 259/257-CO), 208 (17, 244-Cl), 194 (10, 231/229-Cl), 178 (14, 194-O), 165 (43, 4-Methoxy-2-methylbenzoesäure). |
| IR (ATR) | 2921, 2852, 1595, 1266, 1203, 1185, 1110, 1031, 942, 912, 874, 839, 814, 769, 722, 596, 537 cm⁻¹. |
| ¹H-NMR (DMSO-d6) ö in ppm: | 3,11 (s, 4H, -CH₂-CH₂-), 3,82 (s, 3H, -OCH₃), 6,87-6,96 (m, 2H, C⁷-/C⁹-H), 7,37-7,46 (m, 2H, C¹-/C³-H), 7,87 (d, 1H, J=8,37 Hz, C⁶-H), 7,99 (d, 1H, 8,70 J=8,70 Hz, C⁴-H). |
| ¹³C-NMR (DMSO-d6) δ in ppm: | 34,0 (C¹¹), 35,1 (C¹⁰), 55,8 (-OCH₃), 113,1 (C⁷), 114,6 (C⁹), 126,9 (C³), 129,0 (C¹), 130,0 (C^{5a}), 132,7 (C⁶), 133,7 (C⁴), 137,1 (C^{4a}), 137,5 (C²), 144,5 (C^{9a}), 145,7 (C^{11a}), 163,1 (C⁸), 191, (C⁵). |

### Beispiel 49

### 2-Chlor-8-hydroxy-10,11-dihydrodibenzo[a,d]cyclohepten-5-on (10)

Zur Synthese der Titelverbindung werden nach Methode L 1,0 g (4,87 mmol) 2-Chlor-8-methoxy-10,11-dihydrodibenzo[a,d]cyclohepten-5-on, 10 ml HBr (48%, wässrig) und 10 ml Eisessig eingesetzt.
C₁₅H₁₁ClO₂ (Mr = 258,71)

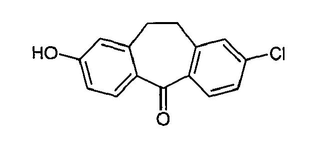

### Beispiel 50

### (S)-2-Chlor-8-(2,2-dimethyl-[1,3]dioxolan-4-ylmethoxy)-10,11-dihydrodibenzo[a,d]cyclohepten-5-on (14)

Zur Darstellung der Titelverbindung werden nach Methode M 0,50 g (1,9 mmol) 2-Chlor-8-hydroxy-10,11-dihydrodibenzo[a,d]cyclohepten-5-on, 0,75 g (2,6 mmol) (R)-Toluen-4-sulfonsäure-2,2-dimethyl-[1,3]dioxolan-4-ylmethylester und 0,80 g (5,8 mmol) K₂CO₃ in 10 ml trockenem DMF umgesetzt.
C₂₁H₂₁ClO₄ (Mr = 372,85)

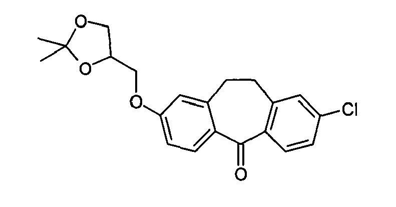

### Beispiel 51

### (S)-2-(2,4-Difluorophenylamino)-8-(2,2-dimethyl-[1,3]dioxolan-4-ylmethoxy)-10,11-dihydrodibenzo[a,d]cyclohepten-5-on (12a)

Zur Synthese der Titelverbindung werden nach Methode O 0,81 g (0,0022 mol) (S)-2-Chlor-1-(2,2-dimethyl-[1,3]dioxolan-4-ylmethoxy)-10,11-dihydrodibenzo[a,d]cyclohepten-5-on, 0,25 g (0,0019 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt.
Die Aufreinigung erfolgt säulenchromatographisch (Flash; SiO₂; Hexan 80% / Ethylacetat 20%).
C₂₇H₂₅F₂NO₄ (Mr= 465,50)

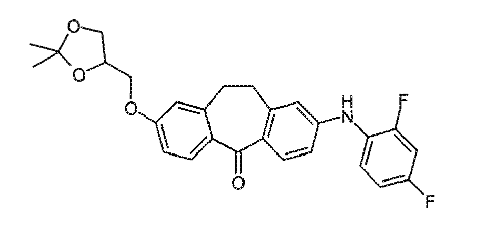

### Beispiel 52

### (R)-2-(2,4-Difluorophenylamino)-8-(2,3-dihydroxypropoxy)-10,11-dihydrodibenzo[a,d]cyclohepten-5-on (12c)

Zur Synthese der Titelverbindung werden nach Methode N 0,10 g (0,215 mmol) (S)-8-(2,4-Difluorophenylamino)-1-(2,2-dimethyl-[1,3]dioxolan-4-ylmethoxy)-10,11-dihydrodibenzo[a,d]cyclohepten-5-on und 0,0054 g (0,0284 mmol) p-Toluensulfonsäure-Monohydrat in 6 ml Methanol und 1,5 ml Wasser umgesetzt.
C₂₄H₂₁F₂NO₄ (Mr = 425,44)
¹H-NMR (DMSO-d6) δ in ppm: 2,95-3,09 (m, 4H, -CH₂-CH₂-), 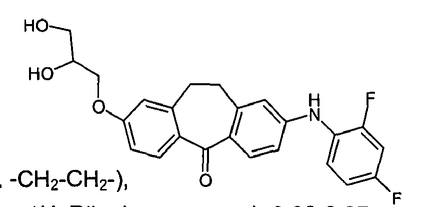 3,42-3,45 (m, 2H, Dihydroxypropoxy), 3,76-3,81 (m, 1H, Dihydroxypropoxy), 3,89-3,97 (m, 1H, Dihydroxypropoxy), 4,04-4,11 (m, 1H, Dihydroxypropoxy), 4,86 (s, 2H, C²-/C³-OH Dihydroxypropoxy), 6,61 (s, 1H, C¹-H), 6,72 (d, 1H, J=9,55 Hz, C³-H), 6,85-6,87 (m, 2H, C^{3'}-/C^{6'}-H), 7,05-7,12 (m, 1H, C^{5'}-H), 7,30-7,47 (m, 2H, C⁷-/C⁹-H), 7,95 (d, 2H, J=8,59 Hz, C⁶-/C⁴-H), 8,49 (s, 1H, -NH₂).

### Beispiel 53

### (S)-2-(2-Aminophenylamino)-8-(2,2-dimethyl-[1,3]dioxolan-4-ylmethoxy)-10,11-dihydrodibenzo[a,d]cyclohepten-5-on (12b)

Zur Synthese der Titelverbindung werden nach Methode O 0,91 g (0,0024 mol) (S)-2-Chlor-8-(2,2-dimethyl-[1,3]dioxolan-4-ylmethoxy)-10,11-dihydrodibenzo[a,d]cyclohepten-5-on, 1,00 g (0,0092 mol) Phenylendiamin, 2 Spatelspitzen Pd(OAc)₂, 0,18 g Phosphinligand und 1,60 g (0,0166 mol) NaOtert-Bu in 10 ml Toluen und 2 ml tert-BuOH umgesetzt.
Die Aufreinigung erfolgt säulenchromatographisch (Flash; SiO₂; Hexan 70% / Ethylacetat 30%).
C₂₇H₂₈N₂O₄ (Mr = 444,54) 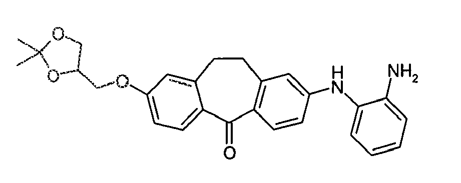 ¹H-NMR (DMSO-d6) δ in ppm: 1,30 (s, 3H, -CH₃), 1,35 (s, 3H, -CH₃), 2,92-3,05 (m, 4H, -CH₂-CH₂-), 3,71-3,78 (m, 1H, Dioxolan), 3,99-4,13 (m, 3H, Dioxolan), 4,38-4,44 (m, 1H, Dioxolan), 4,83 (s, 2H, -NH₂), 6,46-7,03 (m, 9H, C¹-/C³-/C⁴-/C⁷-/C⁹-H und C^{3'}-/C^{4'}-/C^{5'}-/C^{6'}-H), 7,91 (s, 1H, C⁶-H), 7,96 (s, 1H, -NH-).
¹³C-NMR (DMSO-d6) δ in ppm: 25,7 (-CH₃), 27,0 (-CH₃), 35,1 (C¹¹), 36,1 (C¹⁰), 66,0 (C³ Dioxolan), 69,2 (C¹ Dioxolan), 73,9 (C² Dioxolan), 109,3 (-C-(CH₃)₂-), 112,0 (C⁷), 112,9 (C⁹), 113,0 (C³), 114,3 (C¹), 115,8 (C^{3'}), 116,8 (C⁴'), 125,3 (C^{4a}), 126,1 (C^{5'}), 126,3 (C^{6'}), 126,7 (C^{5a}) 131,8 (C^{1'}), 133,6 (C⁶), 133,9 (C⁴), 144,0 (C^{2'}), 145,0 (C^{9a}), 145,4 (C²), 150,8 (C^{11a}), 161,3 (C⁸), 188,7 (C⁵).

### Beispiel 54

### (R)-2-(2-Aminophenylamino)-8-(2,3-dihydroxypropoxy)-10,11-dihydrodibenzo[a,d]cyclohepten-5-on (12d)

Zur Synthese der Titelverbindung werden nach Methode N 0,10 g (0,225 mmol) (S)-2-(2-Amino-phenylamino)-8-(2,2-dimethyl-[1,3]dioxolan-4-ylmethoxy)-10,11-dihydrodibenzo[a,d]cyclohepten-5-one und 0,0537 g (0,282 mmol) p-Toluensulfonsäure-Monohydrat in 6 ml Methanol und 1,5 ml Wasser umgesetzt.
C₂₄H₂₄N₂O₄ (Mr = 404,47) 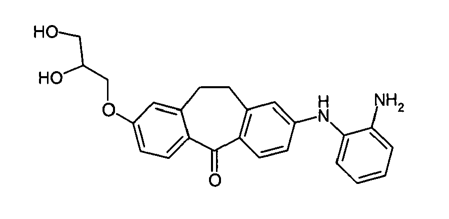 ¹H-NMR (DMSO-*d6*) δ in ppm: 2,83-3,00 (m, 4H, -CH₂-CH₂-), 3,43 (d, 2H, J=4,80 Hz, C³-H Dihydroxypropoxy), 3,93-4,05 (m, 3H, Dihydroxypropoxy), 4,68 (s, 1H, C³-OH), 4,83 (s, 2H, -NH₂). 4,96 (s, 1H, C³-OH), 6,44 (s, 1H, C^{6'}), 6,54-7,03 (m, 8H, C¹-/C³-/C⁴-/C⁷-/C⁹-H und C^{3'}-/C^{4'}-/C^{5'}-H), 7,89-7,96 (m, 2H, C⁶-H, -NH-)
¹³C-NMR (DMSO-d6) δ in ppm: 35,2 (C¹¹), 36,1 (C¹⁰), 63,0 (C³ Dihydroxypropoxy), 70,1 (C¹ Dihydroxypropoxy), 70,2 (C² Dihydroxypropoxy), 112,0 (C⁹), 112,8 (C⁷), 113,0 (C³), 114,3 (C¹), 115,8 (C^{3'}), 116,8 (C^{4'}), 125,4 (C^{4a}), 126,1 (C^{6'}), 126,3 (C^{5'}), 126,7 (C^{5a}), 131,5 (C^{1'}), 133,9 (C⁴), 144,0 (C^{2'}), 145,0 (C^{9a}), 145,4 (C²), 150,8 (C^{11a}), 161,8 (C⁸), 188,7 (C⁵)

### Beispiel 55

### 2-Chlor-8-(2-morpholin-4-ylethoxy)-10,11-dihydrodibenzo[a,d]cyclohepten-5-on (17)

Zur Synthese der Titelverbindung werden nach Methode Q 0,44 g (1,69 mmol) 2-Chlor-8-hydroxy-10,11-dihydrodibenzo[a,d]cyclohepten-5-on, 0,345 g (1,86 mmol) 4-(2-Chlorethyl)morpholinhydrochlorid und 0,93 g (6,75 mmol) K₂CO₃ in 15 ml Acetonitril umgesetzt.
C₂₁H₂₂ClNO₃ (Mr = 371,87)

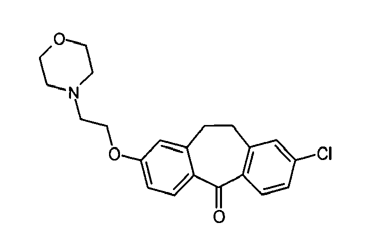

| | |
|---|---|
| ¹H-NMR (DMSO-d6) δ in ppm: | 2,47 (unter DMSO, 4H, C²-/C⁶-H Morpholin), 2,68 (t, 2H, J=5,73 Hz, C²-H Ethylmorphin), 3,10 (s, 4H, -CH₂-CH₂-Cyclohexan), 3,53-3,58 (m, 4H, C³-/C⁵-H Morpholin), 4,15 (t, 2H, J=5,69 Hz, C¹-H Ethylmorpholin), 6,88-6,96 (m, 2H, C⁷-/C⁹-H), 7,36-7,44 (m, 2H, C¹-/C³-H), 7,87 (d, 1H, J=8,37 Hz, C⁶-H), 7,98 (d, 1H, J=8,64 Hz, C⁴-H). |
| ¹³C-NMR (DMSO-d6) δ in ppm: | 34,0 (C¹¹), 35,1 (C¹⁰), 54,0 (2C, C²/C⁶ Morpholin), 57,2 (C² Ethylmorpholin), 66,0 (C¹ Ethylmorpholin), 66,5 (2C, C³/C⁵ Morpholin), 113,5 (C⁷), 115,2 (C⁹), 126,9 (C³), 129,0 (C¹), 130,0 (C^{5a}), 132,7 (C⁶), 133,7 (C⁴), 137,1 (C^{4a}), 137,5 (C²), 144,5 (C^{9a}), 145,7 (C^{11a}), 162,3 (C⁸), 191,0 (C⁵). |

### Beispiel 56

### 2-(2,4-Difluorphenylamino)-8-(2-morpholin-4-yl-ethoxy)-10,11-dihydrodibenzo[a,d]cyclohepten-5-on (12e)

Zur Synthese der Titelverbindung werden nach Methode O 0,45 g (0,0013) mol 8-Chlor-1-(2-morpholin-4-yl-ethoxy)-10,11-dihydrodibenzo[a,d]cyclohepten-5-on, 0,20 g (0,0015 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt.
Die Aufreinigung erfolgt säulenchromatographisch (Flash; SiO₂; Hexan 80% / Ethylacetat 20%).
C₂₇H₂₆F₂N₂O₂ (Mr = 464,52)

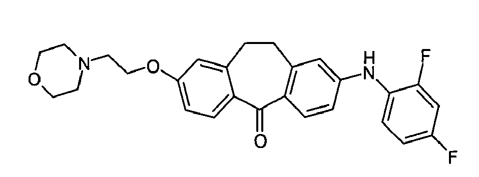

| | |
|---|---|
| ¹H-NMR (DMSO-d6) δ in ppm: | 2,49 (unter DMSO, 4H, C²-/C⁶-H Morpholin), 2,69 (t, 2H, J=5,51 Hz, C²-H Ethylmorpholin), 2,96-3,07 (m, 4H, -CH₂-CH2-Cycloheptan), 3,56 (t, 4H, J=4,50 Hz, C³-/C⁵-H Morpholin), 4,15 (t, 2H, J=5,44 Hz, C¹-H Ethylmorpholin), 6,61 (s, 1H, C¹-H), 6,73 (d, 1H, J=8,43 Hz, C³-H), 6,86-6,91 (m, 2H, C^{3'}-/C^{6'}-H), 7,05-7,13 (m, 1H, C^{5'}-H), 7,30-7,47 (m, 2H, C⁷-/C⁹-H), 7,95 (d, 2H, J=8,66 Hz, C⁴-/C⁶-H), 8,49 (s, 1H, -NH-). |
| ¹³C-NMR (DMSO-d6) δ in ppm: | 35,1 (s, C¹¹), 35,8 (s, C¹⁰), 53,9 (s, 2C, C²/C⁶ Morpholin), 57,3 (s, C² Ethylmorpholin), 65,9 (s, C¹, Ethylmorpholin), 66,5 (s, 2C, C³/C⁵ Morpholin), 105,3 (dd, J₁=25,5 Hz, J₂=24,2 Hz, C^{3'}), 112,2 (dd, J₁=21,9 Hz, J₂=3,8 Hz, C^{5'}), 112,5 (s, C⁷), 113,1 (s, C⁹), 113,6 (s, C⁹), 114,5 (s, C¹), 125,5 (dd, J₁=12,0 Hz, J₂=3,4 Hz, C^{1'}), 126,3 (dd, J₁=9,5 Hz, J₂=3,1 Hz, C^{6'}), 128,2 (s, C^{4a}), 131,3 (s, C^{5a}), 133,6 (s, C⁶), 133,8 (s, C⁴), 145,1 (s, C²), 145,3 (s, C^{11a}), 149,1 (s, C^{9a}), 154,9 (dd, J₁=141,3 Hz, J₂=11,0, C^{4'}), 159,7 (dd, J₁=129,7 Hz, J₂=12,0 Hz, C^{2'}), 161,6 (s, C⁸), 189,1 (s, C⁵)_{.} |

### Substitution in Position 9 - Verbindungen und Vorstufen

### Beispiel 57

### 2-Bromomethyl-3-methoxybenzoesäure (1)

Zur Synthese der Titelverbindung werden nach Methode H (Variante 1) 6,64 g (0,040 mol) 3-Methoxy-2-methylbenzoesäure, 7,1 g (0,040 mol) NBS und 0,25g AIBN in 150 ml Chlorbenzol umgesetzt.
C₉H₉BrO₃ (Mr = 245,07)

| | |
|---|---|
| Ausbeute: | 55% |

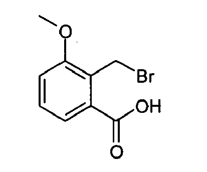

| | |
|---|---|
| ¹H-NMR (DMSO-d6) δ in ppm: | 3,89 (s, 3H, -OCH₃), 5,03 (s, 2H, -CH₂-Br), 7,23-7,28 (m, 1H, C⁵-H), 7,41-7,46 (m, 2H, C⁴-/C⁵-H), 13,25 (s, 1H, -COOH). |
| ¹³C-NMR (DMSO-d6) δ in ppm: | 56,1 (-OCH₃), 68,4 (-CH₂Br), 116,3 (C⁴), 116,8 (C⁶), 126,9 (C²), 131,4 (C⁵), 135,3 (C¹), 154,3 (C³), 170,9 (-COOH). |

### Beispiel 58

### 2-[2-(3-Chlorphenyl)-vinyl]-3-methoxybenzoesäure (2)

Zur Synthese der Titelverbindung werden nach Methode I 45,6 g ( 0,186 mol) 2-Bromomethyl-3-methoxybenzoesäure, 48,8 g (0,186 mol) Triphenylphosphin, 26,1 g (0,186 mol) 3-Chlorbenzaldehyd und 84,0 g (0,435 mol) NaOMe (28%) in 150 ml Methanol umgesetzt.
Die Aufreinigung erfolgt durch Umkristallisation aus Methanol mit Wasser.
C₁₆H₁₃ClO₃ (Mr = 288,73)

| | |
|---|---|
| Ausbeute: | 51 % |
| Schmp.: | 119 °C |

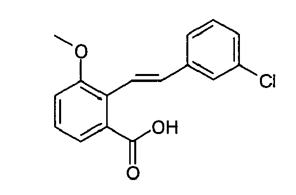

| | |
|---|---|
| IR (ATR) | 2923, 2360, 2342, 1685, 1587, 1451, 1300, 1275, 1260, 1220, 1197, 1178, 1049, 973, 964, 914, 889, 872, 810, 783, 761, 749, 729, 705, 684, 667, 621, 530, 457, 432 cm⁻¹. |
| ¹H-NMR (DMSO-d6) δ in ppm: | 3,86 (s, 3H, -OCH₃), 7,04-7,55 (m, 9H, Diphenylethen), 13,03 (s, 1H, -COOH). |

| | |
|---|---|
| ¹³C-NMR (DMSO-d6) δ in ppm: | 56,3 (O-CH₃), 114,2 (C⁴), 121,4 (C⁶), 124,8 (C²), 125,2 (C¹/C² Vinyl), 126,2 (C^{6'}), 127,6 (C^{2'}), 128,8 (C^{4'}), 130,9 (C⁵), 132,0 (C^{5'}), 133,9 (C¹), 134,0 (C^{3'}), 140,4 (C^{1'}), 157,9 (C³), 169,9 (-COOH). |

### Beispiel 59

### 2-[2-(3-Chlorphenyl)-ethyl]-3-methoxybenzoesäure (3)

Zur Synthese der Titelverbindung werden nach Methode J (Variante 1) 6,0 g 2-[2-(3-Chlorphenyl)-vinyl]-3-methoxybenzoesäure, 0,6 g Pd / BaSO₄ (5 %), 4 l Wasserstoff und 150 ml Methanol eingesetzt.
C₁₆H₁₅ClO₃ (Mr = 290,75)

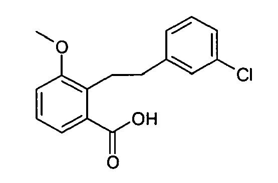

| | |
|---|---|
| Ausbeute: | 87 % |
| Schmp.: | 91 °C |

| | |
|---|---|
| IR (ATR) | 2947, 2359, 2342, 1691, 1461, 1436, 1276, 1250, 1214, 1089, 1058, 1000, 782, 753, 696, 681 cm⁻¹. |
| ¹H-NMR (DMSO-d6) δ in ppm: | 2,74 (t, 2H, J=8,07 Hz, C¹ Ethyl), 3,11 (t, 2H, J=8,08, C² Ethyl), 3,79 (s, 3H, -OCH₃), 7,15 (d, 2H, C⁴-H und C^{6'}-H), 7,22-7,25 (m, 2H, C⁵-H und C^{5'}-H), 7,27 (s, 1H, C^{2'}-H), 7,30- 7,37 (m, 2H, C⁶-H und C^{4'}-H). |
| ¹³C-NMR (DMSO-d6) δ in ppm: | 28,7 (C¹ Ethyl), 35,7 (C² Ethyl), 56,3 (-OCH₃), 114,2 (C⁴), 122,0 (C⁶), 126,1 (C^{6'}), 127,2 (C^{4'}), 128,3 (C^{2'}), 130,3 (C^{5'}), 130,4 (C¹), 132,6 (C²), 133,2 (C^{3'}), 145,0 (C^{1'}), 157,8 (C³), 169,4 (-COOH). |

### Beispiel 60

### 8-Chlor-1-methoxy-10,11-dihydrodibenzo[a,d]cyclohepten-5-on (4)

Zur Synthese der Titelverbindung werden nach Methode K 16,6 g (0,057 mol) [2-(3-Chlorphenyl)-ethyl]-3-methoxybenzoesäure, 7,25 g (0,06 mol) SOCl₂, 250 ml Dichlormethan, und 10 g AlCl₃ eingesetzt.
Die Aufreinigung erfolgt säulenchromatographisch (Flash; SiO₂; Hexan 90% / Ethylacetat 10%).
C₁₆H₁₃ClO₂ (Mr = 272,73)

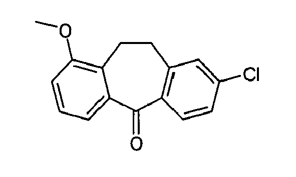

| | |
|---|---|
| Ausbeute: | 47 % |
| Schmp.: | 75 °C |
| GC | 13,5 min |

| | |
|---|---|
| MS m/z (%): | 274/272 (35/100, M⁺), 259/257 (6/18, M⁺-CH₃), 245/243 (5/15, M⁺-CO), 231/229 (3/11, 259/257-CO), 208 (17, 243-Cl), 194 (20, 231/229-Cl), 178 (23, 194-O), 165 (44, 3-Methoxy-2-methylbenzoesäure). |
| IR (ATR) | 2946, 2901, 2359, 1659, 1589, 1575, 1312, 1284, 1254, 1228, 1184, 1087, 1062, 960, 862, 795, 754, 722, 706 cm⁻¹. |
| ¹H-NMR (DMSO-d6) δ in ppm: | 3,08 (s, 4H, -CH₂-CH₂-) 3,82 (s, 3H, -OCH₃), 7,18-7,42 (m, 5H, C²-/C³-/C⁴-/C⁷-/C⁹-H), 7,68 (d, 1H, J=8,28 Hz, C⁶-H). |
| ¹³C-NMR (DMSO-d6) δ in ppm: | 26,2 (C¹¹), 32,9 (C¹⁰), 56,4 (-OCH₃), 114,9 (C²), 121,7 (C⁴), 127,0 (C⁷), 127,6 (C³), 129,0 (C⁹), 130,3 (C^{5a}), 131,3 (C⁶), 137,1 (C^{4a}), 138,1 (C^{11a}), 139,4 (C⁸), 143,7 (C^{9a}), 156,7 (C¹), 195,2 (C⁵). |

### Beispiel 61

### 8-Chlor-1-hydroxy-10,11-dihydrodibenzo[a,d]cyclohepten-5-on (5)

Zur Synthese der Titelverbindung werden nach Methode L 1,0 (4,87 mmol) g 8-Chlor-1-methoxy-10,11-dihydrodibenzo[a,d]cyclohepten-5-on, 10 ml HBr (48%, wässrig) und 10 ml Eisessig eingesetzt.
C₁₅H₁₁ClO₂ (Mr = 258,71)

| | |
|---|---|
| Ausbeute: | 95 % |
| Schmp.: | 185 °C |
| GC | 15,2 min |

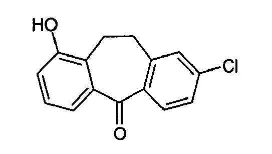

| | |
|---|---|
| MS m/z (%): | 260/258 (34/100, M⁺), 243/241 (2/7, M⁺-OH ), 232/230 (9/27, M⁺-CO), 223 (21, M⁺-Cl), 217/215 (2/7), 207/205 (2/6), 195 (28, 223-CO), 177 (12), 165 (39). |
| IR (ATR) | 3251, 2360, 1637, 1573, 1307, 1282, 1241, 1221, 1176, 1159, 886, 759 cm⁻¹. |
| ¹H-NMR (DMSO-d6) δ in ppm: | 3,07 (s, 4H, -CH₂-CH₂-), 7,04 (d, 1H, J=7,84 Hz, C²-H), 7,15 (t, 1H, J=7,78 Hz, C³-H), 7,28 (d, 1H, J=7,69 Hz, C⁷-H), 7,38 (d, 1H, J=8,31 Hz, C⁴-H), 7,45 (s, 1H, C⁹-H), 7,67 (d, 1H, J=8,32, C⁶-H), 9,84 (s, 1H, -OH). |
| ¹³C-NMR (DMSO-d6) δ in ppm: | 26,5 (C¹¹), 33,0 (C¹⁰), 119,1 (C²), 120,6 (C⁴), 126,9 (C⁷), 127,2 (C³), 128,7 (C⁹), 129,0 (C^{5a}), 131,3 (C⁶), 136,9 (C^{4a}), 138,4 (C^{11a}), 139,5 (C⁸), 143,8 (C^{9a}), 155,0 (C¹), 195,4 (C⁵). |

### Beispiel 62

### 8-(2,4-Difluorphenylamino)-1-hydroxy-10,11-dihydro-dibenzo[a,d]cyclohepten-5-on (11c)

Zur Synthese der Titelverbindung werden nach Methode O 0,52 g (0,0020 mol) 8-Chlor-1-hydroxy-10,11-dihydro-dibenzo[a,d]cyclohepten-5-on, 0,26 g (0,0020 mol) 2,4-Difluoranilin, 2 Spatelspitzen Pd(OAc)₂, 0,14 g Phosphinligand und 0,90g (0,0094 mol) NaOtert-Bu in 10 ml Toluen und 2 ml tert-BuOH umgesetzt.
Die Aufreinigung erfolgt Säulenchromatographisch (Flash; Kieselgel, 90% Hexan + 10% Ethylacetat).
C₂₁H₁₅F₂NO₂ (Mr=351,36)

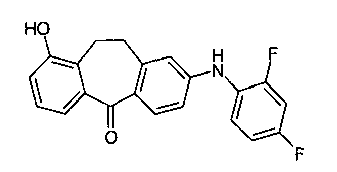

| | |
|---|---|
| GC | 35,3 min |
| MS m/z (%): | 351 (100, M+), 336 (4, M+-O), 323 (10, M+-CO), 308 (3, 323-O), 223 (2, 336-Difluorbenzen), 194 (12, 223-CO), 165 (13, 3-Methoxy-2-methylbenzoesäure), 129 (1, Difluoranilin), 115 (1, Difluoranilin). |
| IR (ATR) | (cm⁻¹) |
| ¹H-NMR (DMSO-d6) δ in ppm: | 2,93-3,06 (m, 4H, -CH₂-CH₂-), 6,61 (s, 1H, C⁹-H), 6,71 (d, 1H, J=8,04 Hz, C^{6'}-H), 6,98 (d, 1H, J=7,84 Hz, C⁷-H), 7,05-7,13 (m, 2H, C^{3'}-/C^{5'}-H), 7,24 (d, 1H, J=7,62 Hz, C²-H), 7,34-7,39 (m, 2H, C³-/C⁴-H), 7,41-7,78 (m, 1H, C⁶-H), 8,46 (s, 1H, -NH-), 9,69 (s, 1H, -OH). |
| ¹³C-NMR (DMSO-d6) δ in ppm: | |

### Beispiel 63

### 8-(2,4-Difluorphenylamino)-1-methoxy-10,11-dihydrodibenzo[a,d]cyclohepten-5-on (11a)

Zur Synthese der Titelverbindung werden nach Methode O 0,54 g (0,0020 mol) 8-Chlor-1-methoxy-10,11-dihydro-dibenzo[a,d]cyclohepten-5-on, 0,26 g (0,0020 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt.
Die Aufreinigung erfolgt säulenchromatographisch (Flash; SiO₂; Hexan 90% / Ethylacetat 10%).
C₂₂H₁₇F₂NO₂ (Mr = 365,38)

| | |
|---|---|
| Ausbeute: | g (%) |
| Schmp.: | 216 °C |

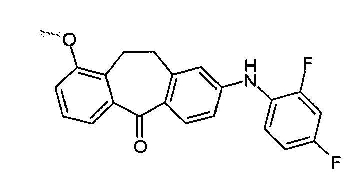

| | |
|---|---|
| GC 31,6 min | |
| MS m/z (%): | 365 (100, M⁺), 350 (6, M⁺-CH₃), 336(8), 322 (5, 350-CO), 306 (3, 322-O), 208 (11, M⁺-CO -NH-Phe), 194 (8, 208-CH₃), 178 (9, 194-O), 165 (17). |
| IR (ATR) | 3276, 1299, 1286, 1269, 1255, 1193, 1073, 966, 863, 828, 764, 727, 603, 534, 496, 460, 446 cm⁻¹. |
| ¹H-NMR (DMSO-d6) δ in ppm: | 2,94-3,08 (m, 4H, -CH₂-CH₂-), 3,82 (s, 3H, -OCH₃), 6,61 (s, 1H, C⁹-H), 6,72 (d, 1H, J=8,72 Hz, C⁷-H), 7,03-7,17- (m, 2H, C^{3'}-/C^{6'}-H), 7,23-7,45- (m, 4H, C²-/C³-/C⁴-H und C^{5'}-H), 7,83 (d, 1H, J=8,69, C⁶-H), 8,49 (s, 1H, NH). |
| ¹³C-NMR (DMSO-d6) δ in ppm: | 25,0 (s, C¹¹), 35,1 (s, C¹⁰), 56,4 (s, -OCH₃), 105,5 (d, J=26,6 Hz, C^{3'}), 112,2 (d, J=25,6 Hz, C^{5'}), 112,6 (s, C⁷), 113,8 (s, C⁹), 114,3 (s, C²), 121,9 (s, C⁴), 125,4 (d, J=12,1 Hz, C^{1'}), 126,3(d, J=9,6 Hz, C^{6'}), 127,3 (s, C³), 128,4 (s, C^{5a}), 130,1 (s, C^{4a}), 132,9 (s, C⁶), 141,2 (s, C^{11a}), 145,2 (s, C⁸), 149,3 (s, C^{9a}), 154,9 (d, J=141,2 Hz, C^{4'}), 155,8 (s, C¹), 159,8 (d, J=137_{,}1 Hz, C^{2'}), 192,6 (s, C⁵). |

### Beispiel 64

### 8-(2-Aminophenylamino)-1-methoxy-10,11-dihydrodibenzo[a,d]cyclohepten-5-on (11b)

Zur Synthese der Titelverbindung werden nach Methode O 0,54 g (0,0020 mol) 8-Chlor-1-methoxy-10,11-dihydro-dibenzo[a,d]cyclohepten-5-on, 1,08 g (0,010 mol) Phenylendiamin, 2 Spatelspitzen Pd(OAc)₂, 0,14 g Phosphinligand und 1,60g (0,0166 mol) NaOtert-Bu in 10 ml Toluen und 2 ml tert-BuOH umgesetzt.
Die Aufreinigung erfolgt säulenchromatographisch (Flash; SiO₂; Hexan 80% / Ethylacetat 20%).
C₂₂H₂₀N₂O₂ (Mr = 344,42)

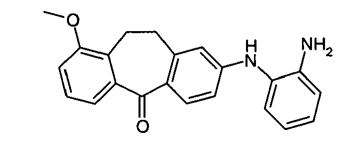

| | |
|---|---|
| Ausbeute: | 46 (%) |
| Schmp.: | 132 °C |
| GC | 54,5 min |

| | |
|---|---|
| MS m/z (%): | 344 (100, M⁺), 329 (13, M⁺-CH₃), 315 (6, M⁺-CO), 301 (5, 315-CH₂), 285 (5, 301-O), 195 (9, 301-1,2-Diaminobenzen), 182 (6), 165 (8, 3-Methoxy-2-methylbenzoesäure), 107 (13, 1,2-Diaminobenzen), 80 (7). |
| IR (ATR) | 3356, 3282, 2852, 1616, 1587, 1563, 1553, 1497, 1335, 1293, 1272, 1255, 1222, 1192, 1075, 826, 759, 749, 706, 540, 505, 438 cm⁻¹. |
| ¹H-NMR (DMSO-d6) δ in ppm: | 2,93-3,02 (m, 4H, -CH₂-CH₂-), 3,81 (s, 3H, -OCH₃), 4,82 (s, 2H, -NH₂), 6,45 (s, 1H, C⁹-H), 6,59 (d, 2H, J=8,22 Hz, C^{3'}-/C^{6'}-H), 6,76 (d, 1H, J=7,66 Hz, C⁷-H), 6,89-7,02 (m, 2H, C^{4'}-/C^{5'}-H), 7,13 (d, 1H, J=7,47 Hz, C²-H), 7,21-7,37 (m, 2H, C³-/C⁴-H), 7,80-8,01 (m, 2H, -NH, C⁶-H). |
| ¹³C-NMR (DMSO-d6) δ in ppm: | 24,8 (C¹¹), 35,5 (C¹⁰), 56,4 (-OCH₃), 112,0 (C⁷), 113,0 (C^{3'}), 114,1 (C⁹), 115,8 (C²), 116,8 (C^{4'}), 122,0 (C^{5'}), 125,3 (C^{5a}), 126,1 (C^{6'}), 126,3 (C⁴), 126,8 (C^{4a}), 127,2 (C³), 130,0 (C^{11a}), 133,2 (C⁶), 141,5 (C^{1'}), 144,0 (C^{2'}), 145,4 (C^{9a}), 151,0 (C⁸), 155,6 (C¹), 191,9 (C⁵). |

### Beispiel 65

### (S)-8-Chlor-1-(2,2-dimethyl-[1,3]dioxolan-4-ylmethoxy)-10,11-dihydrodibenzo[a,d]cyclohepten-5-on (13)

Zur Darstellung der Titelverbindung werden nach Methode M 0,50 g (1,9 mmol) 8-Chlor-1-hydroxy-10,11-dihydrodibenzo[a,d]cyclohepten-5-on, 0,75 g (2,6 mmol) (R)-Toluen-4-sulfonsäure-2,2-dimethyl-[1,3]dioxolan-4-ylmethylester und 0,80 g (5,8 mmol) K₂CO₃ in 10 ml trockenem DMF umgesetzt.
C₂₁H₂₁ClO₄ (Mr = 372,85)

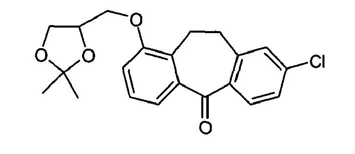

| | |
|---|---|
| Ausbeute: | 53 % |
| Schmp.: | °C |
| GC | 22,8 min |

| | |
|---|---|
| MS m/z (%): | 374/372 (35/100, M⁺), 359/357 (6/17, M⁺-CH₃), 337 (2, M⁺-Cl), 316/314 (10/26, 359/357-CH₃), 299/297 (16/39, 316/314-OH), 285/283 (3/8, 299/297-CH₂), 269 (283), 258/256 (15/6 M⁺-Dioxolan), 194 (12, 258/256-Cl-CO), 178 (23, 194-CO), 165 (51, 3-Methoxy-2-methylbenzoesäure). |
| IR (ATR) | 2962, 2359, 1650, 1586, 1449, 1370, 1310, 1284, 1257, 1213, 1177, 1156, 1057, 974, 873, 790, 761, 694, 665, 555 cm⁻¹. |
| ¹H-NMR (DMSO-d6) δ in ppm: | 1,31 (s, 3H, -CH₃), 1,36 (s, 3H, -CH₃), 3,11 (s, 4H, -CH₂-CH₂), 3,78-3,86 (m, 1H, Dioxolan), 4,00-4,11 (m, 3H, Dioxolan), 4,41-4,46 (m, 1H, Dioxolan), 7,25-7,44 (m, 4H, C²-/C³-/C⁴-/C⁷-H), 7,47 (s, 1H, C⁹-H), 7,69 (d, 1H, J=8,34 Hz, C⁶-H). |
| ¹³C-NMR (DMSO-d6) δ in ppm: | 25,7 (-CH₃), 26,3 (-CH₃), 26,9 (C¹¹), 32,8 (C¹⁰), 66,0 (C³ Dioxolan), 69,8 (C¹ Dioxolan), 74,1 (C² Dioxolan), 109,2 (-C(CH₃)₂), 116,3 (C²), 122,1 (C⁴), 127,0 (C⁷), 127,6 (C³), 129,1 (C⁹), 130,7 (C^{5a}), 131,3 (C⁶), 137,1 (C^{4a}), 138,2 (C^{11a}), 139,5 (C⁸), 143,7 (C^{9a}), 155,9 (C¹), 195,2 (C⁵). |

### Beispiel 66

### (S)-8-(2,4-Difluorphenylamino)-1-(2,2-dimethyl-[1,3]dioxolan-4-ylmethoxy)-10,11-dihydrodibenzo[a,d]cyclohepten-5-on (11d)

Zur Synthese der Titelverbindung werden nach Methode O 0,50 g (0,0013 mol (S)-8-Chlor-1-(2,2-dimethyl-[1,3]dioxolan-4-ylmethoxy)-10,11-dihydrodibenzo[a,d]cyclohepten-5-on, 0,17 g (0,0013 mol) 2,4-Difluoranilin, 2 Spatelspitzen Pd(OAc)₂, 0,14 g Phosphinligand und 0,70 g (0,0073 mol) NaOtert-Bu in 10 ml Toluen und 2 ml tert-BuOH umgesetzt.
Die Aufreinigung erfolgt säulenchromatographisch (Flash; SiO₂; Hexan 80% / Ethylacetat 20%).
C₂₇H₂₅F₂NO₄ (Mr = 465,50)

| | |
|---|---|
| Ausbeute: | 37 % |
| Schmp.: | 127 °C |
| GC min | |
| MS m/z (%): | |

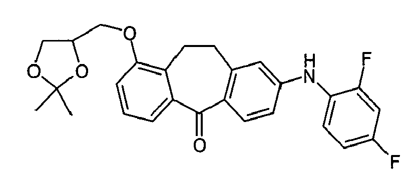

| | |
|---|---|
| IR (ATR) | 3307, 2981, 1503, 1287, 1257, 1207, 1139, 1062, 1041, 864, 843, 832, 810, 757, 541, 515 cm⁻¹. |
| ¹H-NMR (DMSO-d6) δ in ppm: | 1,31 (s, 3H, -CH₃), 1,36 (s, 3H, -CH₃), 2,96-3,11 (m, 4H, -CH₂-CH₂-), 3,78-3,85 (m, 1H, Dioxolan), 4,04-4,14 (m, 3H, Dioxolan), 4,38-4,46 (m, 1H, Dioxolan), 6,62-6,73 (m, 2H, C7-/C9-H), 7,08-7,43 (m, 6H, C²-/C³-/C⁴-H und C^{3'}-/C^{5'}-/C^{6'}-H), 7,81 (d, 1H, J=8,72 Hz, C⁶), 8,49 (s, 1H, -NH-). |
| ¹³C-NMR (DMSO-d6) δ in ppm: | 25,1 (s, C¹⁰), 25,7 (s, -CH₃), 26,9 (s, -CH₃), 35,0 (s, C¹¹), 66,0 (s, C³ Dioxolan), 69,9 (s, C¹ Dioxolan), 74,1 (s, C² Dioxolan), 105,0 (d, J=26,4 Hz, C^{3'}), 109,2 (s, -C(CH₃)₂), 112,2 (d, J=21,8 Hz, C^{5'}), 112,6 (s, C⁷), 113,9 (s, C⁹), 115,7 (s, C²), 122,3 (s, C⁴), 125,4 (d, J=15,8 Hz, C^{1'}), 126,2 (d, J=6,7 Hz, C^{6'}), 127,2 (s, C³), 128,5 (s, C^{5a}), 130,4 (s, C^{4a}), 132,9 (s, C⁶), 141,3 (s, C^{11a}), 145,1 (s, C⁸), 149,3 (s, C^{9a}), 154,9 (d, J=138,5 Hz, C^{4'}), 155,0 (s, C¹), 159,7 (d, J=148,2 Hz, C^{2'}), 192,6 (s, C⁵). |

### Beispiel 67

### (R)-8-(2,4-Difluorphenylamino)-1-(2,3-dihydroxypropoxy)-10,11-dihydrodibenzo-[a,d]cyclohepten-5-on (11f)

Zur Synthese der Titelverbindung werden nach Methode N 0,10 g (0,215 mmol) (S)-8-(2,4-Difluorophenylamino)-1-(2,2-dimethyl-[1,3]dioxolan-4-ylmethoxy)-10,11-dihydrodibenzo[a,d]cyclohepten-5-on und 0,0054 g (0,0284 mmol) p-Toluensulfonsäure-Monohydrat in 6 ml Methanol und 1,5 ml Wasser umgesetzt.
C₂₄H₂₁F₂NO₄ (Mr = 425,44)

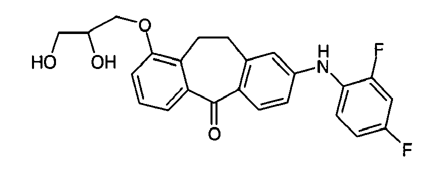

| | |
|---|---|
| Ausbeute: | 88 % |
| Schmp.: | 125°C |
| GC min | |
| MS m/z (%): | |

| | |
|---|---|
| IR (ATR) | 3307, 1541, 1305, 1257, 1217, 1138, 1116, 1098, 1061, 967, 847, 833, 812, 758, 601, 570, 539, 458 cm⁻¹. |
| ¹H-NMR (DMSO-d6) δ in ppm: | 3,00-3,08 (m, 4H, -CH₂-CH₂-), 3,46-3,49 (m, 2H, Dihydroxypropoxy), 3,78-4,03 (m, 3H, Dihydroxypropoxy), 6,63-6,73 (m, 2H, C⁷-/C⁹-H), 7,04-7,41 (m, 6H, C²-/C³-/C⁴-H und C^{3'}-/C⁵'-/C⁶'-H), 7,80 (d, 1H, J=8,73 Hz, C⁶), 8,48 (s, 1H, -NH). |
| ¹³C-NMR (DMSO-d6) δ in ppm: | 25,2 (s, C¹¹), 35,0 (s, C¹⁰), 63,1 (s, C³ Dihydroxypropoxy), 70,4 (s, C² Dihydroxypropoxy), 70,9 (s, C¹ Dihydroxypropoxy), 105,3 (t, J=25,3 Hz, C^{3'}), 112,1 (d, J=25,9 Hz, C^{5'}), 112,5 (s, C⁷), 113,9 (s, C⁹), 115,5 (s, C²), 121,9 (s, C⁴), 125,4 (d, J=15,6 Hz, C^{1'}), 126,1 (d, J=9,6 Hz, C^{6'}), 127,2 (s, C³), 128,6 (s, C^{5a}), 130,5 (s, C^{4a}), 132,8(s, C⁶), 141,2 (s, C^{11a}), 145,1 (s, C⁸), 149,2 (s, C^{9a}), 154,8 (d, J=141,5 Hz, C^{4'}), 155,5 (s, C¹), 159,7 (d, J=136,2 Hz, C^{2'}), 192,7 (s, C⁵). |

### Beispiel 68

### (S)-8-(2-Aminophenylamino)-1-(2,2-dimethyl-[1,3]dioxolan-4-ylmethoxy)-10,11-dihydrodibenzo[a,d]cyclohepten-5-on (11e)

Zur Synthese der Titelverbindung werden nach Methode O 0,50 g (0,0013 mol) (S)-2-Chlor-8-(2,2-dimethyl-[1,3]dioxolan-4-ylmethoxy)-10,11-dihydrodibenzo[a,d]cyclohepten-5-on, 0,70 g (0,0065 mol) Phenylendiamin, 2 Spatelspitzen Pd(OAc)₂, 0,14 g Phosphinligand und 1,04 g (0,0108 mol) NaOtert-Bu in 10 ml Toluen und 2 ml tert-BuOH umgesetzt.
Die Aufreinigung erfolgt säulenchromatographisch (Flash; SiO₂; Hexan 70% / Ethylacetat 30%).
C₂₇H₂₈N₂O₄ (Mr = 444,54)

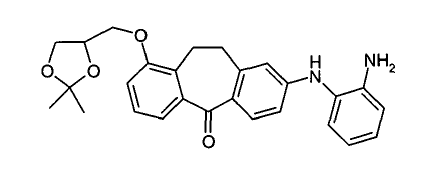

| | |
|---|---|
| Ausbeute: | 23 % |
| Schmp.: | <35°C |
| GC min | |
| MS m/z (%): | |

| | |
|---|---|
| IR (ATR) | 3347, 2927, 1566, 1499, 1450, 1254, 1212, 1155, 1069, 969, 908, 832, 745, 703, 599, 512, 448 cm⁻¹. |
| ¹H-NMR (DMSO-d6) δ in ppm: | 1,31 (s, 3H, -CH₃), 1,36 (s, 3H, -CH₃), 2,91-3,10 (m, 4H, -CH₂-CH₂-), 3,78-3,85 (m, 1H, Dioxolan), 4,00-4,03 (m, 3H Dioxolan), 4,40-4,43 (m, 1H, Dioxolan), 4,82 (s, 2H, -NH₂), 6,47- 6,62 (m, 3H, C⁹-H und C^{5'}-/C^{6'}-H), 6,78 (d, 1H, J=7,29 Hz, C³'-H), 6,92 (d, 1H, J=7,17 Hz, C⁷-H), 7,01 (d, 1H, J=7,76 Hz, C²-H), 7,16-7,24 (m, 2H, C³-H und C^{4'}-H), 7,38 (d, 1H, J=7,55 Hz, C⁴-H), 7,82 (d, 1H, J=8,74, C⁶-H), 7,91 (s, 1H, -NH-). |
| ¹³C-NMR (DMSO-d6) δ in ppm: | 24,9 (C¹¹), 25,7 (-CH₃), 26,9 (-CH₃), 35,4 (C¹⁰), 66,0 (C³ Dioxolan), 69,9 (C¹ Dioxolan), 74,1 (C² Dioxolan), 109,2 (-C(CH₃)₂), 112,0 (C⁷), 113,1 (C⁹), 115,5 (C³'), 115,8 (C²), 116,8 (C^{4'}), 122,4 (C^{5'}), 125,3 (C^{5a}), 126,0 (C^{6'}), 126,3 (C⁴), 126,9 (C³), 127,2 (C^{4a}), 130,4 (C^{11a}), 133,1 (C⁶), 141,6 (C^{1'}), 193,9 (C^{2'}), 145,3 (C⁸), 150,9 (C^{9a}), 154,9 (C¹), 191,9 (C⁵). |

### Beispiel 69

### (R)-8-(2-Aminophenylamino)-1-(2,3-dihydroxypropoxy)-10,11-dihydrodibenzo[a,d]cyclohepten-5-on (11g)

Zur Synthese der Titelverbindung werden nach Methode N 0,10 g (0,225 mmol) (S)-8-(2-Aminophenylamino)-1-(2,2-dimethyl-[1,3]dioxolan-4-ylmethoxy)-10,11-dihydrodibenzo[a,d]cyclohepten-5-on und 0,0537 g (0,282 mmol) p-Toluensulfonsäure-Monohydrat in 6 ml Methanol und 1,5 ml Wasser umgesetzt.
C₂₄H₂₄N₂O₄ (Mr = 404,47)

| | |
|---|---|
| Ausbeute: | 40 % |

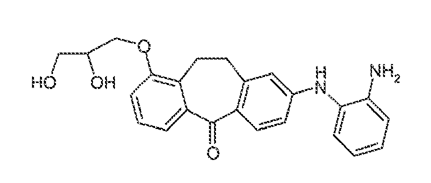

| | |
|---|---|
| IR (ATR) | 3305, 2919, 1566, 1498, 1449, 1255, 1216, 1189, 1155, 1111, 1043, 967, 908, 831, 749, 703, 448, 405 cm⁻¹. |
| ¹H-NMR (DMSO-d6) δ in ppm: | 2,93-3,11 (m, 4H, -CH₂-CH₂-), 3,47 (d, 2H, J=5,48 Hz, C³-H Dihydroxypropoxy), 3,83 (q, 1H, J=5,60 Hz, C²-H Dihydroxypropoxy), 3,85-4,06 (m, 2H, C¹-H Dihydroxypropoxy), 4,82 (s, 2H, -NH₂), 6,48 (s, 1H, C⁹-H), 6,59 (d, 2H, J=7,39 Hz, C^{3'}-/C^{6'}-H), 6,76 (d, 1H, J=7,41 Hz, C⁷-H), 6,90-7,02 (m, 2H, C⁴-H und C^{5'}-H), 7,12 (d, 1H, J=7,37 Hz, C²-H), 7,23 (t, 1H, J=7,80 Hz, C³-H), 7,34 (d, 1H, J=7,09 Hz, C⁴-H), 7,80 (d, 1H, J=8,74 Hz, C⁶-H), 7,91 (s, 1H, -NH-). |
| ¹³C-NMR (DMSO-d6) δ in ppm: | 25,0 (C¹¹), 35,4 (C¹⁰), 63,1 (C³ Dihydroxypropoxy), 70,4 (C² Dihydroxypropoxy), 70,9 (C¹ Dihydroxypropoxy), 112,0 (C⁷), 113,2 (C⁹), 115,4 (C^{3'}), 115,8 (C²), 116,8 (C^{4'}), 122,0 (C^{6'}), 125,4 (C^{5a}), 126,0 (C^{5'}), 126,3 (C⁴), 127,0 (C³), 127,1 (C^{4a}), 130,5 (C^{11a}), 133,1 (C⁶), 141,5 (C¹), 143,9 (C^{2'}), 145,3 (C⁷), 150,9 (C^{9a}), 155,3 (C¹), 192,1 (C⁵). |

### Beispiel 70

### 8-Chlor-1-(tetrahydropyran-4-yloxy)-10,11-dihydrodibenzo[a,d]cyclohepten-5-on (15)

Zur Synthese der Titelverbindung werden nach Methode P 0,45 g (1,7 mmol) 8-Chlor-1-hydroxy-10,11-dihydrodibenzo[a,d]cyclohepten-5-on, 0,28 g (0,0027 mol) Tetrahydropyran-4-ol, 0,77 g (2,9 mmol) P(Ph)₃ und 0,41 g (2,0 mmol) Diisopropylazodicarboxylat in 2 ml THF umgesetzt.
C₂₀H₁₉ClO₃ (Mr = 342,83)

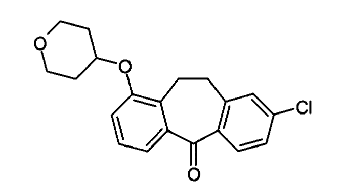

| | |
|---|---|
| Ausbeute: | 45 % |

| | |
|---|---|
| ¹H-NMR (DMSO-d6) δ in ppm: | 1,63 (s, 2H, C²-H Pyran), 1,91 (s, 2H, C⁶-H Pyran), 3,10 (s, 4H, -CH₂-CH₂-), 3,48 (s, 2H, C³-H Pyran), 3,79 (s, 2H, C⁵-H Pyran), 4,61 (s, 1H, C¹-H Pyran), 7,05-7,42 (m, 5H, C²-/C³-/C⁴-/C⁶/C⁷-H), 7,67 (s, 1H, C⁹-H). |
| ¹³C-NMR (DMSO-d6) δ in ppm: | 26,4 (C¹¹), 31,9 (2C, C²/C⁶ Pyran), 32,9 (C¹⁰), 64,6 (2C, C³/C⁵ Pyran), 72,4 (C¹ Pyran), 118,1 (C²), 122,1 (C⁴). 127,0 (C⁷), 127,4 (C³), 129,1 (C⁹), 131,4 (C⁶), 137,1 (C^{4a}), 138,0 (C^{5a}), 139,9 (C^{11a}), 143,8 (C⁸), 154,3 (C^{9a}), 156,5 (C¹), 195,2 (C⁵). |

### Beispiel 71

### 8-(2,4-Difluorphenylamino)-1-(tetrahydropyran-4-yloxy)-10,11-dihydrodibenzo[a,d]cyclohepten-5-on (11h)

Zur Synthese der Titelverbindung werden nach Methode O 0,45 g (0,0013) mol 8-Chlor-1-(tetrahydropyran-4-yloxy)-10,11-dihydrodibenzo[a,d]cyclohepten-5-on, 0,17 g (0,0013 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt.
Die Aufreinigung erfolgt säulenchromatographisch (Flash; SiO₂; Hexan 70% / Ethylacetat 30%).
C₂₆H₂₃F₂NO₃ (Mr = 435,47)

| | |
|---|---|
| Ausbeute: | 52 % |
| Schmp.: | 134 °C |

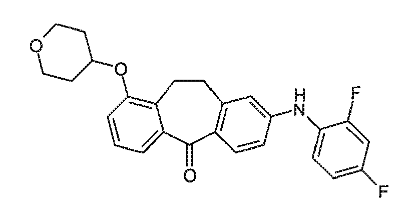

| | |
|---|---|
| IR (ATR) | 3327, 2961, 2857, 1564, 1496, 1300, 1277, 1257, 1243, 1187, 1150, 1131, 1099, 1088, 1005, 957, 858, 833, 813, 769, 630, 577, 550, 508 cm⁻¹. |
| ¹H-NMR (DMSO-d6) δ in ppm: | 1,54-1,71 (m, 2H, C²-H Pyran), 1,91-2,00 (m, 2H, C⁶-H Pyran), 2,96-3,12 (m, 4H, -CH₂-CH₂-), 3,43-3,54 (m, 2H, C³-H Pyran), 3,78-3,88 (m, 2H, C⁴-H Pyran), 4,59 (q, 1H, J=3,68 Hz, C¹-H Pyran), 6,62 (s, 1H, C⁹-H), 6,71 (d, 1H, J=8,68 Hz, C⁷-H), 7,03-7,13 (m, 1H, C^{6'}-H), 7,22-7,25 (m, 2H, C^{3'}-/C^{5'}-H), 7,29-7,42 (m, 3H, C²/C³-/C⁴-H), 7,82 (d, 1H, J=8,66 Hz, C⁶-H), 8,48 (s, 1H, -NH-). |
| ¹³C-NMR (DMSO-d6) δ in ppm: | 25,3 (s, C¹¹), 32,0 (s, 2C, C²/C⁶ Pyran), 35,1 (s, C¹⁰), 64,7 (s, 2C, C³/C⁵ Pyran), 72,8 (s, C¹ Pyran), 105,3 (t, J=25,5 Hz, C^{3'}), 112,2 (d, J=22,1 Hz, C^{5'}), 112,7 (s, C⁷), 113,9 (s, C⁹), 117,9 (s, C²), 122,4 (s, C⁴), 125,4 (d, J=15,7 Hz, C^{1'}), 126,2 (d, J=9,5 Hz, C^{6'}), 127,1 (s, ^{C3}), 128,4(s, C^{5a}), 131,5(s, C^{4a}), 132,9 (s, C⁶), 141,7 (s, C^{11a}), 145,2 (s, C⁸), 149,2 (s, C^{9a}), 155,0 (d, J=141,0 Hz, C^{4'}), 153,4 (s, C¹), 159,6 (d, J=137_{,}2 Hz, C^{2'}), 192,6 (s, C⁵). |

### Beispiel 72

### 8-Chlor-1-(2-morpholin-4-yl-ethoxy)-10,11-dihydrodibenzo[a,d]cyclohepten-5-on (16)

Zur Synthese der Titelverbindung werden nach Methode Q 0,44 g (1,69 mmol) 8-Chlor-1-hydroxy-10,11-dihydrodibenzo[a,d]cyclohepten-5-on, 0,345 g (1,86 mmol) 4-(2-Chlorethyl)morpholinhydrochlorid und 0,93 g (6,75 mmol) K₂CO₃ in 15 ml Acetonitril umgesetzt.
C₂₁H₂₁ClNO₃ (Mr = 371,87)

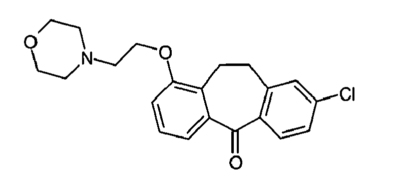

| | |
|---|---|
| Ausbeute: | g (%) |
| Schmp.: | <35°C |
| GC | 29,7min |

| | |
|---|---|
| MS m/z (%): | 373/371 (1/4, M⁺), 165 (4, 3-Methoxy-2-methyl-benzoesäure), 114 (1, Ethylmorpholin), 100 (100, 114-CH₂), 87 (1, 100-CH₂). |
| IR (ATR) | 2854, 1585, 1451, 1282, 1252, 1115, 1090, 1062, 909, 860, 757 cm⁻¹ |
| ¹H-NMR (DMSO-d6) δ in ppm: | 2,50 (unter DMSO, 4H, C²-/C⁶-H Morpholin), 2,71 (t, 2H, J=5,61 Hz, C²-H Ethylmorpholin), 3,09 (s, 4H, -CH₂-CH₂-Cycloheptan), 3,47-3,58 (m, 4H, C³-/C⁵-H Morpholin), 4,12 (t, 2H, J=5,59 Hz, C¹-H Ethylmorpholin), 7,23-7,70 (m, 6H, C²-/C³-/C⁴-/C⁶-/C⁷-/C⁹-H). |
| ¹³C-NMR (DMSO-d6) δ in ppm: | 26,4 (C¹¹), 32,9 (C¹⁰), 53,9 (C²/C⁶ Morpholin), 57,3 (C² Ethylmorpholin), 66,6 (C³/C⁵ Morpholin), 67,0 (C¹ Ethylmorpholin), 116,3 (C²), 121,9 (C⁴), 127,0 (C⁷), 127,5 (C³), 129,1 (C⁹), 130,7 (C⁶), 131,3 (C^{5a}), 137,1 (C^{4a}), 138,1 (C^{11a}), 139,5 (C⁸), 143,7 (C^{9a}), 156,0 (C¹), 195,2 (C⁵). |

### Beispiel 73

### 8-(2,4-Difluorphenylamino)-1-(2-morpholin-4-yl-ethoxy)-10,11-dihydrodibenzo[a,d]cyclohepten-5-on(11j)

Zur Synthese der Titelverbindung werden nach Methode O 0,48 g (0,0013) mol 8-Chlor-1-(2-morpholin-4-yl-ethoxy)-10,11-dihydrodibenzo[a,d]cyclohepten-5-on, 0,17 g (0,0013 mol) 2,4-Difluoranilin, 2 Spatelspitzen Pd(OAc)₂, 0,14 g Phosphinligand und 0,70g (0,0073 mol) NaOtert-Bu in 10 ml Toluen und 2 ml tert-BuOH umgesetzt.
Die Aufreinigung erfolgt säulenchromatographisch (Flash; SiO₂; Hexan 60% / Ethylacetat 40%).
C₂₇H₂₆F₂N₂O₃ (Mr = 464,52)

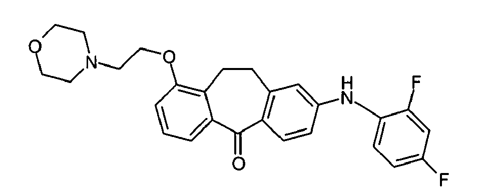

| | |
|---|---|
| ¹H-NMR (DMSO-d6) δ in ppm: | 2,49 (unter DMSO, 4H C²-/C⁶-H Morpholin), 2,72 (t, 2H, J=5,62 Hz, C²-H Ethylmorpholin), 2,99-3,10 (m, 4H, -CH₂-CH₂-Cycloheptan), 3,56 (t, 4H, J=4,58 Hz, C³-/C⁵-H Morpholin), 4,12 (t, 2H, J=5,62 Hz, C¹-H Ethylmorpholin), 6,62 (s, 1H, C⁹-H), 6,71 (d, 1H, J=8,68 Hz, C⁷-H), 7,08-7,41 (m, 6H, C²-/C³-/C⁴-H und C^{3'}-/C^{5'}-/C^{6'}-H), 7,82 (d, 1H, J=8,67 Hz, C⁶-H), 8,48 (s, 1H, -NH-). |
| ¹³C-NMR (DMSO-d6) δ in ppm: | 25,2 (s, C¹¹), 35,1 (s, C¹⁰), 53,9 (s, 2C, C²/C⁶ Morpholin), 57,4 (s, C² Ethylmorpholin), 66,6 (s, 2C, C³/C⁵ Morpholin), 67,1 (s, C¹ Ethylmorpholin), 105,3 (dd, J₁=26,6 Hz, J₂= 24,1 Hz, C^{3'}), 112,1 (dd, J₁=21,8 Hz, J₂=3,7 Hz, C^{5'}), 112,6 (s, C⁷), 113,9 (s, C⁹), 115,8 (s, C²), 122,1 (s, C⁴), 125,4 (dd, J₁=12,1 Hz, J₂=3,6 Hz, C^{1'}), 126,2 (dd, J₁=9,6 Hz, J₂=3,2 Hz, C^{6'}), 127,2 (s_{,} C^{5a}), 128,5 (s, C³), 130,5 (s, C^{4a}), 132,9 (s, C⁶), 141,3 (s, C^{11a}), 145,1 (s, C⁸), 149,2 (s, C^{9a}), 154,8 (dd, J₁=140,7 Hz, J₂=12,6 Hz, C^{4'}), 155,1 (s, C¹ 159,7 (dd, J₁=136,5 Hz, J₂=11,8 Hz, C^{2'}), 196,6 (s, C⁵). |

### Beispiel 74

Trifluormethansulfonsäure-8-chlor-5-oxo-10,11-dihydro-5H-dibenzo[a,d]cyclohepten-1-ylester Zur Herstellung der Titelverbindung werden nach Methode R 0,517 g (0,0020 mol) 8-Chlor-1-hydroxy-10,11-dihydrodibenzo[a,d]cyclohepten-5-on und 0,846 g (0,0030 mol) Tf₂O in 5 ml Pyridin umgesetzt.
C₁₆H₁₀CIF₃O₄S (Mr = 390,77)

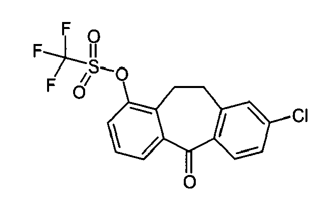

| | |
|---|---|
| Ausbeute: | g (%) |

| | |
|---|---|
| GC | 11,9 min |
| MS m/z (%): | 392/390 (39/100, M⁺), 364/362 (9/24, M⁺-CO), 259/257 (17/50, M⁺-SO₂CF₃), 231/229 (10/31, 259/257-CO), 194 (54, 231/229-CI). |

### C. An Position 8 substituierte Dibenzooxoepinone

### Material und Methoden

Schmelzpunkte: Büchi Melting Point B-545 (thermodynamische Korrektur)
NMR-Spektroskopie: Bruker Advance 200 (200 MHz)
Interner Standard: Tetramethylsilan (TMS), δ [ppm]=0
IR-Spektroskopie: Perkin Elmer Spectrum One (ATR Technik)
DC: SiO₂ 60 F₂₅₄- Alufolien, Familie Merck
GC/MS: Hewlett Packard HP 6890 Series GC-System (Säule: HP-5MS: 5% Phenyl-Methyl-Siloxan; Länge: 30,0 m, Durchmesser: 250 µm, Filmdicke: 0,25 µm; oder ähnliche Säule anderer Hersteller
Trägergas: Helium 6.0
Flussrate: 1,2 ml/min
Hewlett Packard HP 5973 Mass Selective Detector

### Methode 1:

Einlasstemperatur: 250°C

| Heizrate [K/min] | Endtemperatur [ºC] | Haltezeit [min] |
|---|---|---|
| | 100 | 1 |
| 10 | 160 | 10 |
| 15 | 200 | 15 |

### Methode 2:

Einlasstemperatur: 250°C

| Heizrate [K/min] | Endtemperatur [ºC] | Haltezeit [min] |
|---|---|---|
| | 100 | 1 |
| 10 | 160 | 10 |
| 15 | 200 | 15 |
| 10 | 270 | 20 |

### Methode 3:

Einlasstemperatur: 250°C

| Heizrate [K/min] | Endtemperatur [°C] | Haltezeit [min] |
|---|---|---|
| | 160 | 1 |
| 10 | 240 | 5 |
| 10 | 270 | 15 |

### Methode 3 L:

Einlasstemperatur: 250°C

| Heizrate [K/min] | Endtemperatur [°C] | Haltezeit [min] |
|---|---|---|
| | 160 | 1 |
| 10 | 240 | 5 |
| 10 | 270 | 30 |

LC/MS:
TSQ Quantum
Surveyor HPLC (Thermo Finnigan, CA, San Jose) binary pump/autosampler/PDA detector
gekuppelt an Thermo Finnigan TSQ Quantum triple quadrupole MS

### Allgemeine Synthesevorschriften

### Allgemeine Methode S

Zur Veresterung der Säuregruppe der Ausgangsverbindung 2-Methyl-4-nitro-benzoesäure wird die angegebene Menge in einem 100 ml Einhalskolben in Methanol bei Raumtemperatur gelöst und mit konzentrierter H₂SO₄ versetzt und bei ca.60°C für 6h refluxiert. Der überschüssige Alkohol wird im Vakuum entfernt, der Rückstand wird mit EtOAc aufgenommen und wiederholt mit 20%iger Natronlauge entsäuert. Die vereinigten organischen Phasen werden nach Trocknen (Na₂SO₄) im Vakuum eingeengt.

### Allgemeine Methode T

In einem 250 ml-Dreihalskolben mit Rückflusskühler und Trockenrohr wird die zu halogenierende Substanz in Tetrachlorkohlenstoff bei ca.70°C gelöst und mit den angegebene Mengen von N-Bromsuccinimid und Azobisisobutyronitril versetzt. Die Mischung wird 5 h ggf. unter Bestrahlung mit einem 500 W Scheinwerfer refluxiert. Am Ende der Reaktion hat sich N-Bromsuccinimid aufgelöst und ist in Succinimid übergegangen, das sich an der Oberfläche absetzt. Nach dem Abkühlen auf Raumtemperatur wird abgesaugt. Das Filtrat wird einrotiert, um das Produkt als gelb-oranges Öl zu erhalten.

### Allgemeine Methode U

Zur weiteren Umsetzung des bromierten Eduktes mit substituierten Phenolen wird in einem 250 ml-Dreihalskolben die angegebene Menge Kaliumcarbonat in Aceton suspendiert. Das zu deprotonierende Phenol wird zugegeben und bei Raumtemperatur 15 min gerührt. Dann wird die Mischung mit dem bromierten Benzoesäuremethylester versetzt und nach Erhitzen auf 70°C 6 h refluxiert. Das Aceton wird im Vakuum entfernt und der Rückstand wird mehrfach mit EtOAc ausgeschüttelt. Nach Ansäuern der wässrigen Phase mit 20%iger HCI werden die vereinigten organischen Phasen im Vakuum nach Trocknen (Na₂SO₄) eingeengt und durch Umkristallisation mit Methanol aufgereinigt.

### Allgemeine Methode V

Der zu verseifende Carbonsäureester wird bei Raumtemperatur in einem 100 ml-Einhalskolben in Methanol unter Rühren gelöst und anschließend mit der angegebenen Menge wässriger KOH-Lösung versetzt. Nach Erwärmen der Mischung auf 40°C wird 4 h refluxiert. Der überschüssige Alkohol wird im Vakuum entfernt, der Rückstand wird mit H₂O aufgenommen. Es folgt Ansäuern der wässrigen Phase mit 20%iger HCI und anschließendes Rühren auf Eis, das dabei ausfallende Rohprodukt wird abgesaugt und über Calciumchlorid getrocknet.

### Allgemeine Methode W

Zur Synthese der Ketone wird die angegebene Menge Carbonsäure in einem trockenen 250 ml Dreihalskolben in Sulfolan unter Argon-Atmosphäre und Erwärmen suspendiert. Es wird Polyphosphorsäure zugegeben und der Ansatz für ca. 2 h bei 100°C refluxiert. Nach Abkühlen auf Raumtemperatur wird die Mischung in 250 ml Eiswasser gegossen und bei RT nachgerührt. Das ausgefallene Rohprodukt wird abfiltriert.

### Allgemeine Methode X

In einem 100 ml Einhalskolben mit Rückflusskühler wird das acetylierte Ringgerüst in Methanol durch Erwärmen gelöst und anschließend mit der angegeben Menge konzentrierter Salzsäure versetzt. Die Reaktionsmischung wird für 4h unter Rückfluss gerührt. Anschließend engt man den Ansatz im Vakuum ein. Den Rückstand versetzt man mit Salzsäure (10%) und rührt ihn auf, stellt ihn anschließend auf Eis und wartet bis das Produkt vollständig auskristallisiert ist und filtriert es ab.

### Allgemeine Methode Y

Zur Reduktion der Nitroverbindung wird die angegebene Menge Zinn(II)chlorid-Dihydrat in Ethanol unter Rühren gelöst. Die zu reduzierende Nitroverbindung wird hinzugegeben und die Mischung wird für 2 h bei 100 °C refluxiert. Nach Abkühlen auf Raumtemperatur wird der Ansatz mit Eiswasser versetzt und mit 20%iger Natronlauge alkalisiert. Es erfolgt wiederholte Extraktion mit Ethylacetat; die vereinigten organischen Phasen werden nach Trocknen (Na₂SO₄) im Vakuum eingeengt.

### Allgemeine Methode Z

In einen trockenen 100 ml-Dreihalskolben mit Rückflusskühler, Blasenzähler und Thermometer werden die angegebenen Mengen von 3-Amino-8-nitro-6H-dibenzo[b,e]oxepin-11-on, Phosphinligand, KOt-Bu, tert-Butanol, Halogenaromat, Pd(OAc)₂ eingewogen. Die Mischung wird unter Argon-Atmosphäre in Toluol (wasserfrei) suspendiert und auf 110°C erhitzt und für 2 h refluxiert. Es erfolgt Hydrolyse mit Eiswasser und wiederholte Extraktion mit EtOAc. Die vereinigten organischen Phasen werden filtriert und anschließend im Vakuum eingeengt. Der Rückstand wird chromatographisch aufgereinigt.

### Beispiel 75

### 2-Methyl-4-nitro-benzoesäuremethylester (1)

Nach der Allgemeinen Methode S werden 5,00 g (27,60 mmol) 2-Methyl-4-nitro-benzoesäure in 20 ml Methanol unter Erwärmen gelöst und mit 6 ml konzentrierter Schwefelsäure versetzt. Die Ansatzmischung wird für 5h refluxiert.
C₉H₉NO₃(Mᵣ= 195,18)

| | |
|---|---|
| Ausbeute | 4,90 g (90,9%) |
| Schmelzpunkt | 73,6°C |
| ¹H-NMR (CDCl₃) δ in ppm: | 8.19-8.09 (m, 2H, aryl H), 7.99 (d, 1H, J= 8.52 Hz, aryl H), 3.87 (s, 3H, -OCH₃), 2.59 (s, 3H, -CH₃) |
| ¹³C-NMR (CDCl₃) in ppm: | 166.35 (-C=O), 159.29 (C⁴), 141.79 (C²), 135.14 (C¹), 131.43 (C⁶), 126.06 (C³), 120.48 (C⁵), 52.37 (-OCH₃), 21.50 (-CH₃) |
| IR (ATR) (cm⁻¹): | 1722, 1523, 1432, 1348, 1260, 1081, 896, 821, 786, 730 |
| GC (Methode 1) | 9,55 min |
| MS m/z (%): | 195 (70), 164 (100), 134 (21), 118 (29), 63 (15) |

### Beispiel 76

### 2-Brommethyl-4-nitro-benzoesäuremethylester (2)

Nach der Allgemeinen Methode T werden 4,70 g (24,00 mmol) (1) in 30 ml Tetrachlorkohlenstoff unter Erwärmen gelöst und anschließend mit 5,00 g (28,00 mmol) N-Bromsuccinimid und 2 Spatelspitzen Azobisisobutyronitril versetzt. Die Ansatzmischung wird für 6h unter Bestrahlung mit einem 500 W Scheinwerfer refluxiert. Beim Abkühlen auf Raumtemperatur scheidet sich Succinimid ab, das abfiltriert wird. Das Filtrat wird einrotiert, um das Produkt als gelbes Öl zu erhalten.
C₉H₈BrNO₄ (Mᵣ = 274,07)

| | |
|---|---|
| Ausbeute | 5,85 g (88,8%) |
| ¹H-NMR (CDCl₃) δ in ppm: | 4.96 (s, 2H, -CH₂-Br), 2.71 (s, 3H, -CH₃) |
| GC (Methode 3) | 7,98 min |
| MS m/z (%): | 274 (100), 193 (38), 178 (46), 147 (12), 132 (18), 88 (9) |

### Beispiel 77

### 2-(3-Acetylaminophenoxymethyl)-4-nitrobenzoesäuremethylester (3)

Nach der Allgemeinen Methode U werden 1,50 g (10,86 mmol) Kaliumcarbonat in 15 ml Aceton suspendiert und anschließend mit 1,80 g (11,90 mmol) 3-Acetamidophenol versetzt, die Ansatzmischung wird für 15 min gerührt. 2,44 g (8,91 mmol) (2) werden der Ansatzmischung zugegeben.
C₁₇H₁₆N₂O₆ (Mᵣ = 344,33)

| | |
|---|---|
| Ausbeute | 1,33 g (43,3%) |
| Schmelzpunkt | 139,2°C |

¹H-NMR (DMSO-_{d6}) δ in ppm: 9.95 (s, 1H, -NH), 8.45 (d, 1H, J= 1.98 Hz, aryl H), 8.27 (m, 1H, aryl H), 8.12 (d, 1H, J= 8.54 Hz, aryl H), 7.37 (s, 1H, aryl H), 7.21-7.16 (m, 2 H, aryl H), 6.71-6.68 (m, 1H, aryl H), 5.44 (s, 2 H, -CH₂-O), 3.86 (s, 3H, -O-CH₃)
¹³C-NMR (DMSO-_{d6}) δ in ppm: 168.75 (-NH-C=O-), 166.00 (-C=O), 158.41 (C¹), 149.75 (C⁴), 141.00 (C²), 140.77 (C^{3'}), 134.48 (C¹), 132.10 (C⁶), 130.02 (C^{5'}), 123.08 (C³), 122.64 (C⁵), 112.37 (C^{4'}), 109.49 (C^{6'}), 105.94 (C^{2'}), 65.01 (-CH₂-O), 52.16 (-O-CH₃), 24.41 (-NH=O-CH₃)

| | |
|---|---|
| IR (ATR) (cm⁻¹): | 1729, 1719, 1606, 1349, 1289, 1253, 1158, 1057, 771, 727 |
| GC (Methode 3) | 23,07 min |
| MS m/z (%): | 344 (10), 327 (10), 271 (3), 194 (100), 148 (44) |

### Beispiel 78

### 2-(3-Acetylaminophenoxymethyl)-4-nitrobenzoesäure (4)

Nach der Allgemeinen Methode V werden 1,30 g (3,78 mmol) (3) in 20 ml Methanol gelöst und mit 0,70 g (12,50 mmol) KOH versetzt.
C₁₆₇H₁₄N₂O₆ (Mᵣ = 330,30)

| | |
|---|---|
| Ausbeute | 1,20 g (96,1%) |
| Schmelzpunkt | 225,0°C |

¹H-NMR (DMSO-_{d6}) δ in ppm: 9.96 (s, 1H, -COOH), 8.42 (d, 1H, J= 2.06 Hz, aryl H), 8.26 (dd, 1H, J₁= 8.52 Hz, J₂= 2.38 Hz, aryl H), 8.13 (d, 1H, J = 8.00 Hz, aryl H), 7.37 (s, 1H, aryl H), 7,26-7.14 (m, 2H, aryl H), 6.72-6.66 (m, 1H, aryl H), 5.47 (s, 2H, -CH₂-O), 2.01 (s, 3H, -CH₃)
¹³C-NMR (DMSO-_{d6}) δ in ppm: 168.74 (-COOH), 167.14(-NH-C=O), 158.46 (C^{1'}), 149.58 (C⁴), 141.00 (C²), 140.81 (C^{3'}), 135.63 (C¹), 132.27 (C⁶), 130.02 (C^{5'}), 122.89 (C³), 122.28 (C⁵), 112.30 (C^{4'}), 109.55 (C^{6'}), 105.90 (C^{2'}), 67.10 (-CH₂-O-), 24.40 (-NH-C=O-CH₃)

| | |
|---|---|
| IR (ATR) (cm⁻¹): | 1732, 1603, 1520, 1487, 1343, 1284, 1159, 1053, 767, 733 |

### Beispiel 79

### 3-Acetamido-8-nitro-6H-dibenzo[b,e]oxepin-11-on (5)

Nach der Allgemeinen Methode W werden 0,80 g (2,42 mmol) (4) in 10 ml Sulfolan bei 100°C angelöst. Anschließend werden 15 ml (30,0 g) Polyphosphorsäure dem Reaktionsgemisch hinzugegeben.
C₁₆H₁₂N₂O₅ (Mᵣ = 312,28)

| | |
|---|---|
| Ausbeute | 0,50 g (66,2%) |
| Schmelzpunkt | 171,4°C |

¹H-NMR (DMSO-_{d6}) δ in ppm: 9.95 (s, 1H, -NH), 8.42 (d, 1H, J= 2.32 Hz, aryl H), 8.51 (dd, 1H, J₁= 17.4 Hz, J₂= 2.36 Hz, aryl H), 8.13 (d, 1H, J = 8.56 Hz, aryl H), 7.36 (s, 1H, aryl H), 7.21 (s, 1H, aryl H), 7.17 (s, 1H, aryl H), 6.72-6.66 (m, 1H, aryl H), 5.47 (s, 2H, -CH₂-O)

| | |
|---|---|
| IR (ATR) | (cm¹): 1670, 1580, 1526, 1406, 1344, 1294, 1247, 905, 740, 708 |
| LC | 18,62 min |
| MS (ESI) | 312.9 [M+H]⁺ |

### Beispiel 80

### 3-Amino-8-nitro-6H-dibenzo[b,e]oxepin-11-on (6)

Nach der Allgemeinen Methode X werden 0,70 g (2,24 mmol) (5) in 25 ml Methanol gelöst und mit 8,0 ml konzentrierter Salsäure versetzt.
C₁₄H₁₀N₂O₄ (Mᵣ = 270,25)

| | |
|---|---|
| Ausbeute | 0,40 g (66,1%) |
| Schmelzpunkt | 59,0°C |

¹H-NMR (DMSO-_{d6}) δ in ppm: 8.45 (d, 1H, J= 2.10 Hz, aryl H), 8.30 (dd, 1H, J₁= 2.54 Hz, J₂= 2.35 Hz, aryl H), 7.99 (d, 1H, J= 8.58 Hz, aryl H), 7.87 (d, 1H, J= 8.90 Hz, aryl H), 6.42 (dd, 1H, J₁= 2.89 Hz, J₂= 2.14 Hz, aryl H), 6.10 (d, 1H, J= 2.0 Hz, aryl H), 5.29 (s, 2H, -CH₂-O)
¹³C-NMR (DMSO-_{d6}) δ in ppm: 185.21 (C¹¹), 163.65 (C^{4a}), 162.24 (C³), 157.01 (C⁸), 154.81 (C^{6a}), 137.84 (C^{10a}), 134.01 (C¹), 131.02 (C¹⁰), 123.91 (C⁷), 123.38 (C⁹), 114.37 (C^{11a}), 110.98 (C²), 100.98 (C⁴), 71.90 (C⁶)

| | |
|---|---|
| IR (ATR) (cm⁻¹): | 2790, 2565, 1644, 1608, 1531, 1297, 1250, 1150, 1085, 907, 819, 708 |
| GC (Methode 3) | 20,74 min |
| MS m/z (%): | 270 (100), 241 (13), 224 (14), 195 (22), 167 (19), 139 (10), 83 (9), 63 (9) |

### Beispiel 81

### 3-(2,4-Difluorphenylamino)-8-nitro-6H-dibenzo[b,e]oxepin-11-on (7)

Nach der Allgemeinen Methode Z werden 0,50 g (1,85 mmol) (6), 0,28 g (1,89 mmol) 1-Chlor-2,4-difluorbenzen, 2 Spatelspitzen Pd(OAc)₂, 0,09 g 2-(Dicyclohexylphosphino)-2',4',6'-triisopropylbiphenyl (Phosphinligand), 0,50 g KOt-Bu, 1,5 ml t-BuOH eingewogen und in 10 ml Toluol (wasserfrei) gelöst. Die Ansatzmischung wird unter Argon-Atmosphäre für 4h bei 100°C refluxiert. Das Rohprodukt wird über Kieselgel mit DCM/EtOH (98/2) chromatographisch aufgereinigt.
C₂₀H₁₂F₂N₂O₄ (Mᵣ = 382,33)

| | |
|---|---|
| Ausbeute | 0,05 g (7,0%) |
| Schmelzpunkt | 210-214°C |

¹H-NMR (DMSO-_{d6}) δ in ppm: 8.84 (s, 1H, aryl H), 8.48 (d, 1H, J= 2.01 Hz, aryl H), 8.30 (m, 1H, aryl H), 8.01 (dd, 2H, J₁= 2.70 Hz, J₂= 3.25 Hz, aryl H), 7.47-7.33 (m, 2H, aryl H), 7.15-7.04 (m, 2H, aryl H), 6.63 (d, 1H, J= 10.0 Hz, aryl H), 6.24 (s, 1H, aryl H), 5.34 (s, 2H, -CH₂-O)
¹³C-NMR (DMSO-_{d6}) δ in ppm: 186.11 (C¹¹), 163.30 (C^{4a}), nicht detektiert (C4', C2'), 152.89 (C⁸), 149.52 (C³), 145.34 (C^{6a}), 137.76 (C^{10a}), 133.85 (C¹), 131.01 (C¹⁰), 127.38 (C^{1'}, J= 9.85 Hz), 124.23 (C^{6'}, J= 14.23 Hz), 116.52 (C⁷), 112.57 (C^{5'}, J= 3.52 Hz), 110.35 (C²), 105.45 (C^{3'}, J= 24.29 Hz), 101.93 (C⁴), 72.03 (C⁶)

| | |
|---|---|
| IR (ATR) | (cm⁻¹): 2849, 1643, 1608, 1531, 1297, 1249, 1150, 740 |
| LC | 8,97 min |
| MS (ESI) | 381.1 [M-H]⁻ |

### Beispiel 82

### 3-(2,4-Difluorphenylamino)-8-amino-6H-dibenzo[b,e]oxepin-11-on (8)

Nach der Allgemeinen Methode Y werden 0,10 g (0,44 mmol) Zinn(II)-Chlorid-Dihydrat in 7 ml Ethanol gelöst und mit 0,45 g (0,11 mmol) (7) versetzt.
C₂₀H₁₄F₂N₂O₂ (Mᵣ = 282,30)

| | |
|---|---|
| Ausbeute | 0,035 g (90,3%) |
| Schmelzpunkt | 184-193°C |

¹H-NMR (DMSO-_{d6}) δ in ppm: 8.60 (s, 1H, -NH), 8.01 (d, 1H, J= 10.00 Hz, aryl H), 7.66 (d, 1H, J= 8.0 Hz, aryl H), 7.46-7.30 (m, 2H, aryl H), 7.13-7.04 (m, 1H, aryl H), 6.58 (d, 2H, J= 12.0 Hz, aryl H), 6.55 (s, 1H, aryl H), 6.46 (s, 1H, aryl H), 6.06 (s, 2H, -NH₂), 4.97 (s, 2H, -CH₂-O)
¹³C-NMR (DMSO-_{d6}) δ in ppm: 184.59 (C¹¹), 162.65 (C^{4a}), nicht detektiert (C^{4'}, C^{2'})153.51 (C⁸), 151.07 (C³), 139.19 (C^{6a}), 133.86 (C¹), 132.47 (C¹⁰), 127.02 (C⁹), 126.99 (C^{1'}), 125.55 (C⁶), 118.32 (C^{10a}), 113.50 (C⁷), 111.67 (C^{5'}), 109.85 (C²), 105.32 (C^{3'}), 102.56 (C⁴), 74.41 (C⁶)

| | |
|---|---|
| IR (ATR) (cm⁻¹): | 1613, 1586, 1557, 1523, 1402, 1324, 1312, 1281, 1258, 1115, 816 |
| LC | 7,76 min |
| MS (ESI) | 353.2 [M+H]⁺ |

### Beispiel 83

### 3-(2-Nitrophenylamino)-8-nitro-6H-dibenzo[b,e]oxepin-11-on (9)

Nach der Allgemeinen Methode Z werden 1,00 g (3,70 mmol) (6), 0,80 g (3,96 mmol) 1-Brom-2-nitrobenzen, 2 Spatelspitzen Pd(OAc)₂, 0,10 g 2-(Dicyclohexylphosphino)-2',4',6'-triisopropylbiphenyl (Phosphinligand), 0,70 g KOt-Bu, 2,0 ml t-BuOH eingewogen und in 10 ml Toluol (wasserfrei) gelöst. Die Ansatzmischung wird unter Argon-Atmosphäre für 4h bei
100°C refluxiert. Das Rohprodukt wird über Kieselgel mit DCM/EtOH (98/2) chromatographisch aufgereinigt.
C₂₀H₁₃N₃O₆ (Mᵣ = 391,34)

| | |
|---|---|
| Ausbeute | 0,35 g (24,2%) |
| Schmelzpunkt | 212,5°C |

¹H-NMR (DMSO-_{d6}) δ in ppm: 9.34 (s, 1H, -NH), 8.50 (d, 1H, J= 2.24 Hz, aryl H), 8.34 (dd, 1H, J₁= 2.39 Hz, J₂= 2.40 Hz, aryl H), 8.10-7.98 (m, 3H, aryl H), 7.66-7.56 (m, 2H, aryl H), 7.25 (t, 1H, J₁= 1.6 Hz, J₂= 6.8 Hz, aryl H), 6.97 (dd, 1H, J₁= 2.04 Hz, J₂= 2.23 Hz, aryl H), 6.71 (s, 1H, aryl H), 5.39 (s, 2H, -CH₂-O)
¹³C-NMR (DMSO-_{d6}) δ in ppm: 184.34 (C¹¹), 162.90 (C^{4a}), 149.93 (C⁸), 149.69 (C³), 145.19 (C^{6a}), 137.69 (C^{10a}), 136.34 (C^{1'}), 135.45 (C^{5'}),134.08 (C^{2'}), 133.68 (C¹), 130.99 (C¹⁰), 128.40 (C^{3'} und C⁷),126.40 (C^{6'}), 123.66 (C⁹),123.33 (C^{4'}), 118.55 (C^{11a}), 112.45 (C²), 106.36 (C⁴), 72.34 (C⁶)

| | |
|---|---|
| IR (ATR) (cm⁻¹): | 1642, 1595, 1582, 1500, 1356, 1340, 1269, 1248, 1226, 1152, 1131, 1034,736 |
| LC | 9,18 min |
| MS (ESI) | 390.3 [M-H]- |

### Beispiel 84

### 3-(2-Aminophenylamino)-8-amino-6H-dibenzo[b,e]oxepin-11-on (10)

Nach der Allgemeinen Methode Y werden 2,40 g (10,66 mmol) Zinn(II)-Chlorid-Dihydrat in 15 ml Ethanol gelöst und mit 0,35 g (0,89 mmol) (9) versetzt.
C₂₀H₁₇N₃O₂ (Mᵣ = 331,38)

| | |
|---|---|
| Ausbeute | 0,20 g (67,9%) |
| Schmelzpunkt | 203,9°C |

¹H-NMR (MeOH-_{d4}) δ in ppm: 8.09 (d, 1H, J= 9.0 Hz, aryl H), 7.78 (d, 1H, J= 8.55 Hz, aryl H), 7.09-7.00(m, 2H, aryl H), 6.89-6.88 (m, 1H, aryl H), 6.71-6.64 (m, 2H, aryl H), 6.55-6.49 (m, 2H, aryl H),6.14 (d, 1H, J= 2.26 Hz, aryl H), 4.97 (s, 2H, -CH₂-O)
¹³C-NMR (MeOH-_{d4}) δ in ppm: 186.65 (C¹¹), 163.58 (C^{4a}), 153.31 (C⁸), 153.12 (C³), 143.31 (C^{6a}), 139.37 (C^{1'}), 133.34 (C¹), 131.97 (C¹⁰), 127.65 (C^{6'}), 126.51 (C^{2'}), 125.66(C^{10a}), 117.775 (C^{5'}), 116.89 (C^{11a}), 115.96 (C^{4'}), 113.34 (C⁹), 111.46 (C^{3'}), 109.27 (C²), 101.14 (C⁴), 73.99 (C⁶)

| | |
|---|---|
| IR (ATR) (cm⁻¹): | 1601, 1579, 1550, 1497, 1291, 1267, 1228, 1112, 828, 757, 747 |
| LC | 7,05 min |
| MS (ESI) | 332.9 [M+H]⁺ |

### Beispiel 85

### 3-(4-Fluor-2-nitrophenylamino)-8-nitro-6H-dibenzo[b,e]oxepin-11-on (11)

Nach der Allgemeinen Methode Z werden 0,85 g (3,14mmol) (6), 0,70 g (3,19 mmol) 1-Brom-4-fluor-2-nitrobenzen, 2 Spatelspitzen Pd(OAc)₂, 0,10 g 2-(Dicyclohexylphosphino)-2',4',6'-triisopropylbiphenyl (Phosphinligand), 0,70 g KOt-Bu, 2,0 ml t-BuOH eingewogen und in 10 ml Toluol (wasserfrei) gelöst. Die Ansatzmischung wird unter Argon-Atmosphäre für 4h bei 100°C refluxiert. Das Rohprodukt wird über Kieselgel mit DCM/EtOH (98/2) chromatographisch aufgereinigt.
C₂₀H₁₂FN₂O₄ (Mᵣ = 409,33)

| | |
|---|---|
| Ausbeute | 0,10 g (7,8%) |
| Schmelzpunkt | 210,7°C |

¹H-NMR (DMSO-_{d6}) δ in ppm: 9.22 (s, 1H, -NH), 8.48 (d, 1H, J= 6.86 Hz, aryl H), 8.32 (d, 1H, J= 9.10 Hz, aryl H), 8.10-8.09 (m, 3H, aryl H), 8.04-7.98 (m, 2H, aryl H), 6.87(d, 1H, J= 9.36 Hz, aryl H), 6.59 (s, 2H, -CH₂-O)
¹³C-NMR (DMSO-_{d6}) δ in ppm: 186.67 (C¹¹), 163.01 (C^{4a}), 161.01 (C^{4'}, J= 251.75 Hz), 150.73 (C⁸), 149.67 (C³), 145.22 (C^{6a}), 141.37 (C^{2'} J= 7.30 Hz), 137.71 (C^{10a}), 133.77 (C¹), 132.56 (C1'), 131.00 (C¹⁰), 124.19 (C^{5'}), 123.66 (C⁷), 123.15 (C^{6'}), 122.70 (C⁹), 118.18 (C^{11a}), 113.03 (C^{3'}, J= 23.44 Hz), 112.23 (C²), 105.28 (C⁴), 72.03 (C⁶)

| | |
|---|---|
| IR (ATR) (cm⁻¹): | 1585, 1531, 1516, 1308, 1264, 1246, 1138, 1036, 890, 872, 815 |
| LC | 9,15 min |
| MS (ESI) | 408.3 [M-H]⁻ |

### Beispiel 86

### 3-(2-Amino-4-fluorphenylamino)-8-nitro-6H-dibenzo[b,e]oxepin-11-on (12)

Nach der Allgemeinen Methode Y werden 0,60 g (2,66 mmol) Zinn(II)-Chlorid-Dihydrat in 10 ml Ethanol gelöst und mit 0,10 g (0,89 mmol) (11) versetzt.
C₂₀H₁₆FN₃O₂ (Mᵣ = 349,37)

| | |
|---|---|
| Ausbeute | 0,01 g (11,9%) |
| Schmelzpunkt | 194,5°C |

¹H-NMR (MeOH-_{d4}) δ in ppm: 8.08 (d, 1H, J= 8.97 Hz, aryl H), 7.77 (d, 1H, J= 8.96 Hz, aryl H), 7.07-6.96 (m, 1H, aryl H), 6.69-6.38 (m, 5H, aryl H), 6.07 (s, 1H, aryl H), 4.97 (s, 2H, -CH₂-O)
¹³C-NMR (MeOH-_{d4}) δ in ppm: 186.68 (C¹¹), 163.57 (C^{4a}), 155.84 (C^{4'}, J= 217.20 Hz), 153.68 (C⁸), 149.21 (C³), 139.15 (C^{6a}), 133.41 (C¹), 131.81 (C¹⁰), 130.23 (C¹), 128.66 (C^{6'}, J= 11.6 Hz), 116.88 (C^{11a}), 115.34 (C⁹), 113.61 (C⁷), 109.27 (C²), 105.34 (C^{5'},J= 24.45 Hz), 103.03 (C^{3'}, J= 23.18 Hz), 101.15 (C⁴), 73.67 (C⁶)

| | |
|---|---|
| IR (ATR) (cm⁻¹): | 1602, 1579, 1551, 1505, 1293, 1265, 1233, 1161,1113, 831, 759 |
| LC | 7,34 min |
| MS (ESI) | 350.5 [M+H]⁺ |

### Beispiel 87

### 3-(2-Methoxyphenylamino)-8-nitro-6H-dibenzo[b,e]oxepin-11-on (14)

Nach der Allgemeinen Methode Z werden 0,80 g (2,96 mmol) (6), 0,70 g (3,80 mmol) 2-Bromanisol, 2 Spatelspitzen Pd(OAc)₂, 0,10 g 2- (Dicyclohexylphosphino)-2',4',6'-triisopropylbiphenyl (Phosphinligand), 0,50 g KOt-Bu, 2,0 ml t-BuOH eingewogen und in 10 ml Toluol (wasserfrei) gelöst. Die Ansatzmischung wird unter Argon-Atmosphäre für 4h bei 100°C refluxiert. Das Rohprodukt wird über Kieselgel mit DCM/EtOH (98/2) chromatographisch aufgereinigt.

| | |
|---|---|
| C₂₁H₁₆N_{z}O₅ (Mᵣ= 376,37) | |
| Ausbeute | 0,025g (2,2%) |
| Schmelzpunkt | 200,3°C |

¹H-NMR (DMSO-_{d6}) δ in ppm: 8.55 (s, 1H, aryl H), 8.47 (d, 1H, J= 2.31 Hz, aryl H), 8.30 (dd, 1H, J₁= 2.47 Hz, J₂= 2,41 Hz, aryl H), 8.02-7.92 (m, 2H, aryl H), 7.27-7.23 (m, 1H, aryl H), 7.16-7.10 (m, 2H, aryl H), 6.99-6.95 (m, 1H, aryl H), 6.69 (dd, 1H, J₁= 3.07 Hz, J₂= 2.26 Hz, aryl H), 6.32 (d, 1H, J= 2.19 Hz, aryl H), 5.32 (s, 2H, -CH₂-O), 3.78 (s, 3H, -O-CH₃)
¹³C-NMR (DMSO-_{d6}) δ in ppm: 185.73 (C¹¹), 163.30 (C^{4a}), 153.28 (C⁸),152.84 (C^{2'}), 149.43 (C³), 145.46 (C^{6a}), 137.82 (C^{10a}), 133.60 (C¹), 131.02 (C¹⁰), 128.68 (C^{1'}), 125.78 (C⁹), 124.06 (C⁷), 123.51 (C^{5'}), 121.00 (C^{4'}), 115.96 (C^{11a}), 112.60 (C^{3'}), 110.67 (C⁴), 71.98 (-O-CH₂), 55.84 (-O-CH₃)

| | |
|---|---|
| IR (ATR) (cm⁻¹): | 3410, 1622, 1593, 1520, 1343, 1296, 1235, 1027, 768, 761, 742 |
| LC | 9,03 min |
| MS (ESI) | 377.4 [M+H]⁺ |

### Beispiel 88

### 8-Amino-3-(2-methoxyphenylamino)-6H-dibenzo[b,e]oxepin-11-on (15)

Nach der Allgemeinen Methode Y werden 0,10 g (0,44 mmol) Zinn(II)- Chlorid- Dihydrat in 15 ml Ethanol gelöst und mit 0,04 g (0,10 mmol) (14) versetzt.

| | |
|---|---|
| C₂₁H₁₈N₂O₃ (Mᵣ = 346,39) | |
| Ausbeute | 0,0046 g (13,3%) |
| Schmelzpunkt | 68,4°C |

¹H-NMR (DMSO-_{d6}) δ in ppm: 8.52 (s, 1H, aryl H), 8.32 (d, 1H, J= 5.19 Hz, aryl H), 7.98 (d, 1H, J= 6.31 Hz, aryl H), 7.85-7.24 (m, 4H, aryl H), 6.70-6.19 (m, 4H, aryl H), 5.95 (s, 2H, - NH₂), 4.96 (s, 2H, -CH₂-O), 3.78 (s, 3H, -OCH₃)
¹³C-NMR (DMSO-_{d6}) δ in ppm: 184.50 (C¹¹), 162.65 (C^{4a}), 153.38 (C⁸), 152.30 (C^{2'}), 151.29 (C³), 139.20 (C^{6a}), 133.61 (C¹), 132.44 (C¹⁰), 129.51 (C^{1'}), 127.17 (C^{10a}), 124.77 (C⁵), 122.91 (C^{6'}), 120.93 (C^{4'}), 117.81 (C^{11a}), 113.47 (C^{3'}), 112.43 (C⁹), 111.66 (C⁷), 10.19 (C²), 102.65 (C⁴), 74.34 (C⁶), 55.81 (-OCH₃)

| | |
|---|---|
| LC | 18,55 min |
| MS (ESI) | 347.3 [M+H]⁺ |

### Beispiel 89

### 8-Nitro-3-(4-fluor-2-methoxyphenylamino)-6H-dibenzo[b,e]oxepin-11-on (16)

Nach der Allgemeinen Methode Z werden 0,70 g (2,60 mmol) (6), 0,55 g (2,68 mmol) 2-Brom-5-fluoranisol, 2 Spatelspitzen Pd(OAc)₂, 0,10 g 2- (Dicyclohexylphosphino)-2',4',6'-triisopropylbiphenyl (Phosphinligand), 0,50 g KOt-Bu, 2,0 ml t-BuOH eingewogen und in 10 ml Toluol (wasserfrei) gelöst. Die Ansatzmischung wird unter Argon-Atmosphäre für 4h bei 100°C refluxiert. Das Rohprodukt wird über Kieselgel mit DCM/EtOH (98/2) chromatographisch aufgereinigt.
C₂₁H₁₅FN₂O₅ (Mᵣ = 394,36)

| | |
|---|---|
| Ausbeute | 0,07 g (0,01%) |
| Schmelzpunkt | 222,3°C |

¹H-NMR (DMSO-_{d6}) δ in ppm: 8.64-8.27 (m, 3H, aryl H), 8.20-7.92 (m, 2H, aryl H), 7.24-7.01 (m, 2H, aryl H), 6.87-6.58 (m, 2H, aryl H), 5.75 (s, 1H, -NH), 5.31 (s, 2H, -CH₂-O), 3.94 (s, 3H, -OCH₃)
¹³C-NMR (DMSO-_{d6}) δ in ppm: 185.72 (C¹¹), 163.34 (C^{4a}), 160.42 (C^{4'}, J= 241.23 Hz), 154.77 (C^{2'}, J= 10.51 Hz), 153.84 (C⁸), 149.40 (C³), 145.45 (C^{6a}), 137.80 (C^{10a}), 133.66 (C¹), 131.01 (C¹⁰), 126.29 (C^{6'} J= 10.16 Hz), 124.81 (C^{1'}, J= 30.68 Hz), 124.01 (C⁷), 123.49 (C⁹), 115.80 (C^{11a}), 110.29 (C²), 106.96 (C^{5'}, J= 22.03 Hz), 101.05 (C^{3'}, J= 26.81 Hz), nicht detektiert (C⁴), 71.98 (C⁶), 56.34 (-OCH₃)

| | |
|---|---|
| IR (ATR) (cm⁻¹): | 3314, 1613, 1552, 1510, 1307, 1279, 1235, 1151, 1189, 957, 829, 816 |
| LC | 8,90 min |
| MS (ESI) | 395.3 [M+H]⁺ |

### Beispiel 90

### 8-Amino-3-(4-fluor-2-methoxyphenylamino)-6H-dibenzo[b,e]-oxepin-11-on (17)

Nach der Allgemeinen Methode Y werden 0,30 g (1,32 mmol) Zinn(II)-Chlorid-Dihydrat in 15 ml Ethanol gelöst und mit 0,07 g (0,19 mmol) (16) versetzt.
C₂₁H₁₇FN₂O₃ (Mᵣ = 364,38)

| | |
|---|---|
| Ausbeute | 0,042 g (60,6%) |
| Schmelzpunkt | 38,9°C |

¹H-NMR (DMSO-_{d6}) δ in ppm: 8.17 (s, 1H, aryl H), 7.98 (d, 1H, J= 14.89 Hz, aryl H), 7.64 (d, 1H, J= 11.40 Hz, aryl H), 7.27-7.19 (m, 1H, aryl H), 7.04-6.98 (m, 1H, aryl H), 6.81-6.72 (m, 1H, aryl H), 6.59-6.46 (m, 3H, aryl H), 6.05 (s, 1H, -NH), 5.97 (s, 2H, -NH₂), 4.95 (s, 2H, -CH₂-O), 3.78 (s, 3H, -OCH₃
¹³C-NMR (DMSO-_{d6}) δ in ppm: 184.49 (C¹¹), 162.70 (C^{4a}), 159.92 (C^{4'}, J= 240.08 Hz), 154.35 (C^{2'}, J= 10.41 Hz), 153.37 (C⁸), 151.98 (C³), 139.18 (C^{6a}), 133.67 (C¹), 132.43 (C¹⁰), 127.15 (C^{10a}), 125.59 (C^{6'}, J= 3.07 Hz), 125.28 (C^{1'}), 117.58 (C^{11a}), 113.46 (C⁹), 111.65 (C⁷), 109.72 (C²), 106.81 (C^{5'}, J= 21.98 Hz), 101.99 (C⁴), 100.92 (C^{3'},J= 26.96 Hz), 74.33 (C⁶), 56.29 (-OCH₃)

| | |
|---|---|
| IR (ATR) (cm⁻¹): | 3329, 2923, 1599, 1584, 1520, 1268, 1229, 1110, 1028, 949, 832 |
| LC | 18,82 min |
| MS (ESI) | 365.2 [M+H]⁺ |

### Beispiel 91

### 8-Nitro-3-(2-nitro-4-trifluormethylphenylamino)-6H-dibenzo[b,e]oxepin-11-on (18)

Nach der Allgemeinen Methode Z werden 0,70 g (2,60mmol) (6), 0,55 g (3,05 mmol) 4-Chlor-3-nitro-benzotrifluorid, 2 Spatelspitzen Pd(OAc)₂, 0,10 g 2- (Dicyclohexylphosphino)-2',4',6'-triisopropylbiphenyl (Phosphinligand), 0,50 g KOt-Bu, 2,0 ml t-BuOH eingewogen und in 10 ml Toluol (wasserfrei) gelöst. Die Ansatzmischung wird unter Argon-Atmosphäre für 4h bei 100°C refluxiert. Das Rohprodukt wird über Kieselgel mit n-Hexan/Ethylacetat (5/2) chromatographisch aufgereinigt.
C₂₁H₁₂F₃N₃O₆ (Mᵣ = 459,34)

| | |
|---|---|
| Ausbeute | 0,047 g (3,9%) |
| Schmelzpunkt | 196,3°C |

¹H-NMR (DMSO-_{d6}) δ in ppm: 9.74 (s, 1H, -NH), 8.54 (s, 1H, aryl H), 8.37-8.31 (m, 2H, aryl H), 8.11-7.99 (m, 2H, aryl H), 7.90 (d, 1H, J= 9.30 Hz, aryl H), 7.13 (d, 1H, J= 8.08 Hz, aryl H), 6.98 (s, 1H, aryl H), 5.45 (s, 2H, -CH₂-O)
¹³C-NMR (DMSO-_{d6}) δ in ppm: 187.42 (C¹¹), 162.55 (C^{4a}), 149.84 (C⁸), 147.66 (C³), 145.08 (C^{1'}), 141.09 (C^{6a}), 137.64 (C^{2'}), 136.89 (C^{10a}), 133.52 (C^{5'}), 131.72 (C¹⁰), 130.98 (C¹), 124.32 (C⁹), 123.77 (C⁷), 121.73(CF₃), 121.33 (C^{3'}), 120.65 (C^{4'}), 120.32 (C^{6'}), 120.00 (C^{11a}), 115.32 (C²), 110.32 (C⁴), 72.04 (C⁶)

| | |
|---|---|
| IR (ATR) | (cm⁻¹): 3324, 1636, 1603, 1574, 1531, 1324, 1150, 1112, 1083, 914, 819, 709 |
| LC | 9,67 min |
| MS (ESI) | 458.6 [M-H]⁻ |

### Beispiel 92

### 8-Amino-3-(2-amino-4-trifluormethylphenylamino)-6H-dibenzo[b,e]oxepin-11-on (19)

Nach der Allgemeinen Methode Y werden 0,30 g (1,32 mmol) Zinn(II)-Chlorid-Dihydrat in 15 ml Ethanol gelöst und mit 0,095 g (0,20 mmol) (18) versetzt.
C₂₁H₁₆F₃N₃O₂ (Mᵣ = 399,38)

| | |
|---|---|
| Ausbeute | 0,061 g (76,4%) |
| Schmelzpunkt | 93,9°C |

¹H-NMR (DMSO-_{d6}) δ in ppm: 8.14 (s, 1H, aryl H), 8.00 (d, 1H, J= 8.90 Hz, aryl H), 7.65 (d, 1H, J= 8.53 Hz, aryl H), 7.20 (d, 1H, J= 8.12 Hz, aryl H), 7.06 (s, 1H, aryl H), 6.85 (d, 1H, J= 8.57 Hz, aryl H), 6.62-6.55 (m, 2H, aryl H), 6.45 (s, 1H, aryl H), 6.23 (d, 1H, J= 2.04 Hz, aryl H), 6.05 (s, 2H, -NH₂), 5.31 (s, 2H, -NH₂), 4.96 (s, 2H, -CH₂-O)
¹³C-NMR (DMSO-_{d6}) δ in ppm: 184.61 (C¹¹), 162.66 (C^{4a}), 153.44 (C⁸), 151.07 (C³), 143.35 (C^{1'}), nicht detektiert (C^{6a}) 139.20 (C^{2'}), 133.79 (C¹), 132.47 (C¹⁰), 129.17 (CF₃), 127.06 (C^{4'}), 125.45 (C^{5'}), 124.47 (C^{6'}), 118.09 (C^{11a}), 113.50 (C9), 113.01 (C^{3'}), 111.70 (C⁷), 110.33 (C²), 102.90 (C⁴), 74.39 (C⁶)

| | |
|---|---|
| IR (ATR) (cm⁻¹): | 3352, 1599, 1521, 1334, 1293, 1231, 1160, 1116, 917, 815 |
| LC | 19,06 min |
| MS | 400.3 [M+H]⁺ |

### Beispiel 93

### 3-(Tetrazol-1-yl)-8-nitro-6H-dibenzo[b,e]oxepin-11-on (20)

0,50 g (1,85 mmol) (6) und 0,35 g (5,38 mmol) Natriumazid werden in 2,20 g (14,84 mmol) Trimethylorthoformat bei 0°C suspendiert. Dann wird der Ansatzmischung 1,20 g (20,00 mmol) Eisessig hinzugefügt, die Mischung wird auf 100°C erhitzt und für 4h refluxiert. Nach Abkühlen auf Raumtemperatur wird der Ansatz im Vakuum eingeengt, der Rückstand mit EtOAc aufgenommen und in der Wärme gelöst. Das weiß-graue Produkt fällt in der Kälte aus.
C₁₅H₉NsO₄ (Mᵣ = 323,27)

| | |
|---|---|
| Ausbeute | 0,50 g (83,6 %) |
| Schmelzpunkt | 123-126°C |

¹H-NMR (DMSO-_{d6}) δ in ppm: 10.27 (s, 1H, Tetrazol), 8.59 (s, 1H, aryl H), 8.39-8.30 (m, 2H, aryl H), 8.03 (d, 1H, J= 8.62, aryl H), 7.85-7.80 (m, 2H, aryl H), 5.60 (s, 2H, -CH₂-O)
¹³C-NMR (DMSO) δ in ppm: 188.40 (C¹¹), 161.95 (C^{4a}), 150.21 (C⁸), 144.68 (Tetrazol), 137.54 (C^{10a}), 133.19 (C¹⁰), 131.19 (C¹), 125.02 (C⁷), 124.55 (C²), 124.00 (C^{11a}), 114.81 (C⁹), 112.81 (C⁴), 70.01 (C⁶)

### Beispiel 94

### 8-Amino-3-(tetrazol-1-yl)-6H-dibenzo[b,e]oxepin-11-on (21)

Nach der Allgemeinen Methode Y werden 1,50 g (6,66 mmol) Zinn(II)-Chlorid-Dihydrat in 10 ml Ethanol gelöst und mit 0,50 g (1,54 mmol) (20) versetzt.
C₁₅H₁₁N₅O₂ (Mᵣ = 293,29)

| | |
|---|---|
| Ausbeute | 0,027 g (6,1%) |
| Schmelzpunkt | 277,0°C |

¹H-NMR (DMSO-d6) δ in ppm: 10.21 (s, 1H, Tetrazol), 8.39 (d, 1H, J= 8.58 Hz, aryl H), 7.73 (d, 3H, J= 9.92 Hz, aryl H), 6.64-6.54 (m, 2H, aryl H), 6.34 (s, 2H, -NH₂), 5.18 (s, 2H, -CH₂-O) ¹³C-NMR (DMSO) δ in ppm: 184.72 (C¹¹), 161.51 (C^{4a}), 154.61 (C⁸), 142.78 (C^{6a}), 139.50 (C³), 137.80 (Tetrazol), 134.41 (C¹⁰), 133.14 (C¹), 127.30 (C²), 126.05 (C^{10a}), 114.65 (C^{11a}), 113.78 (C⁹), 112.69 (C⁷), 111.90 (C⁴), 74.99 (C⁶)

| | |
|---|---|
| IR (ATR) | (cm⁻¹ 1608, 1588, 1553, 1501, 1374, 1300, 1196, 1002,842,762,700 |

### Beispiel 95

### 3-(2,4-Difluorphenylamino)-8-tetrazol-1-yl-6H-dibenzo[b,e]oxepin-11-on (13)

0,015 g (0,042 mmol) (8) und 0,01 g (0,15 mmol) Natriumazid werden in 0,05 g (0,33 mmol) Trimethylorthoformiat bei 0°C suspendiert. Dann wird der Ansatzmischung 0,03 g (0,50 mmol) Eisessig hinzugefügt, die Mischung wird auf 100°C erhitzt und für 4h refluxiert. Nach Abkühlen auf Raumtemperatur wird der Ansatz im Vakuum eingeengt, der Rückstand mit EtOAc aufgenommen und in der Wärme gelöst. Das gelbe Produkt fällt in der Kälte aus.

| | |
|---|---|
| C₂₁H₁₃F₂N₅O₂ (Mᵣ = 405,37) | |
| Ausbeute | 0,26 g (88,1%) |
| Schmelzpunkt | 59,0°C |

¹H-NMR (DMSO_{-d6}) δ in ppm: 10.21 (s, 1H, Tetrazol), 9.02 (s, 1H, -NH), 8.18 (s, 1H, aryl H), 8.10-7.98 (m, 3H, aryl H), 7.48-7.39 (m, 2H, aryl H), 7.38-7.32 (m, 1H, aryl H), 6.65 (d, 1H, J= 9.84 Hz), aryl H), 6.26 (s, 1H, aryl H), 5.34 (s, 2H, -CH₂-O)
¹³C-NMR (DMSO-_{d6}) δ in ppm: 174.70 (C¹¹), 163.21 (C^{4a}), icht detektiert (C4'), 155.34 (C^{2'}, J= 267.55 Hz), 142.79 (C³), 140.74 (Tetrazol), 138.38 (C^{6a}), 136.36 (C¹), 133.85 (C⁸), 131.53 (C^{10+10a}), nicht detektiert (C⁹), 121.42 (C^{6'}), 120.00 (C^{1'}), 116.75 (C^{5'}), 110.28 (C²), 102.02 (C⁴) , 72.60 (C⁶)

| | |
|---|---|
| IR (ATR) (cm⁻¹): | 2790, 2565, 1644, 1608, 1531, 1297, 1250, 1150, 1085, 907, 819, 708 |
| LC | 8,15 min |
| MS (ESI) | 404.1 [M-H]⁻ |

### Beispiel 96

### 2-(2-Methyl-4-Fluoranilino)-7-methoxydibenzosuberon (10m)

Zur Herstellung der Verbindung 10m werden 0,5 g (1,8 mmol) 2-Chlor-7-methoxydibenzosuberon, 0,25 g (2,0 mmol) 3-Methyl-4-fluoranilin, 0,05 g (0,22 mmol) Pd(OAc)₂, 0,10 g (0,21 mmol) 2-(Dicyclohexylphosphino)-2',4',6'-triisopropyl-biphenyl, 0,70g (6,2 mmol) KOt-Bu, 5 ml Toluol und 1 ml t-BuOH verwendet und nach Methode C umgesetzt.
H₁-NMR (DMSO-*d6*) δ in ppm: 2.96 (s, 4 H, -CH₂-CH₂-), 3.76 (s, 3 H, -OCH₃), 6.46 (s, 1 H, C¹-H), 6.61 (d, 1 H, J = 8.8, C³-H), 6.99-7.26 (m, 5 H, C⁸-H, C⁹-H, C²-H, C⁴-H, C⁵-H), 7.38 (s, 1 H, C⁶-H), 7.94 (s, d, 1 H, J = 8.7, C⁴-H), 8.22 (s, 1 H, -NH-).
¹³C-NMR (DMSO-d6) δ in ppm: 33.5 (C¹²), 36.4 (C¹¹), 55.5 (-OCH₃), 111.9 (C⁶), 113.1 (C³), 113.7 (d, 1 C, J = 22.2, C⁵), 114.7 (C¹), 117.7 (d, 1 C, J = 22.0, C³), 118.4 (C³), 126.7 (C⁸), 127.0 (C^{1'}), 130.4 (C⁹), 134.0 (C^{5a}), 134.5 (C⁴), 135.4 (d, 1 C, J = 2.6, C²), 136.2 (d, 1 C, J = 8.3, C^{1'}-H), 140.2 (C^{9a}), 145.9 (C^{11a}), 150.9 (C²), 158.0 (C⁷), 159.6 (d, 1 C, J = 241.0, C^{4'}), 190.5 (C⁵)_{.}

### Beispiel 97

### 2-(2-Chloranilino)-7-methoxydibenzosuberon (10n)

Zur Herstellung der Verbindung 10n werden 0,5 g (1,8 mmol) 2-Chlor-7-methoxydibenzosuberon, 0,27 g (2,1 mmol) 2-Chloranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet und nach Methode C umgesetzt.
H₁-NMR (DMSO-d6) δ in ppm: 3.00 (s, 4 H, -CH₂-CH₂-), 3.77 (s, 3 H, -OCH₃), 6.73 (s, 1 H, C₁-H), 6.84 (d, 1 H, C³-H), 7.02-7.54 (m, 7H, C⁶-H, C⁷-H, C₈-H, C₂-H, C³-H, C⁴-H, C⁵), 7.95 (d, 1 H, J = 8.7, C⁴-H), 8.45 (s, 1 H, -NH-).
¹³C-NMR (DMSO-*d*6) δ in ppm: 33.5 (C¹²), 36.2 (C¹¹), 55.5 (-OCH₃), 113.4 (C⁶), 114.6 (C³), 114.9 (C¹), 118.6 (C⁸), 124.5 (C^{4'}), 125.4 (C⁶), 127.3 (C⁵'), 128.1 (C^{4a}), 128.4 (C^{2'}) 130.6 (C⁹), 130.7 (C^{3'}), 133.7 (C⁴), 134.5 (C^{5a}), 138.1 (C^{1'}) 140.0 (C^{9a}), 145.6 (C^{11a}), 148.9 (C²), 158.1 (C⁷), 190.9 (C⁵).

### Beispiel 98

### 2-(2, 4-Difluoranilino)-7-hydroxy-10,11-dihydro-dibenzo[a,d]-cyclohepten-5-on (11a)

0,2 g (0,55 mmol) 2-(2, 4-Difluoranilino)-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolisiert und es wird vom Niederschlag abfiltriert. Man erhält das Produkt in Form eines weißen Pulvers.
C₂₁H₁₅F₂NO₂ (Mr = 351,35)

### Beispiel 99

### 2-(2-Amino-4-fluoranilino)-7-hydroxy-10,11-dihydro-dibenzo[a,d]-cyclohepten-5-on (11b)

0,2 g (0,55 mmol) 2-(2-Amino-4-fluoranilino)-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolysiert und es wird vom Niederschlag abfiltriert. Man erhält das Produkt in Form eines weißen Pulvers.
C₂₁H₁₇FN₂O₂ (Mr= 348,38)

### Beispiel 100

### 2-(2-Chlor-4-fluoranilino)-7-hydroxy-10,11-dihydro-dibenzo[a,d]-cyclohepten-5-on (11c) C₂₁H₁₅CIFNO₂ (Mr = 367,81)

0,2 g (0,52 mmol) 2-(2-Chlor-4-fluoranilino)-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolysiert und es wird vom Niederschlag abfiltriert. Man erhält das Produkt in Form eines weißen Pulvers.

### Beispiel 101

### 2-(2-Chloranilino)-7-hydroxy-10,11-dihydro-dibenzo[a,d]-cyclohepten-5-on (11d)

0,2 g (0,55 mmol) 2-(2-Chloranilino)-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolysiert und es wird vom Niederschlag abfiltriert. Man erhält das Produkt in Form eines weißen Pulvers.

### Beispiel 102

### 2-(Anilino)-7-hydroxy-10,11-dihydro-dibenzo[a,d]-cyclohepten-5-on (11e)

0,2 g (0,61 mmol) 2-Anilino-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolysiert und es wird vom Niederschlag abfiltriert. Man erhält das Produkt in Form eines weißen Pulvers.
C₂₁H₁₇NO₂ (Mr = 315,380)

### Beispiel 103

### 2-(2, 4-Difluoranilino)-7-hydroxy-dibenzo[a,d]-cyclohepten-5-on

0,2 g (0,55 mmol) 2-(2-Amino-4-fluoranilino)-7-methoxy-10,11-dihydro-dibenzo[a,d]-cyclohepten-5-on werden in 2 ml Eisessig gelöst, 2 ml HBr (45%) hinzu gefügt und 6 h refluxiert. Die Lösung wird mit 200 g Eis hydrolysiert und es wird vom Niederschlag abfiltriert. Man erhält das Produkt in Form eines weißen Pulvers.

### Beispiel 104

### 2-(2, 4-Difluoranilino)-7-[3-(4-Hydroxypiperidin-4-yl-propoxy)]-10,11-dihydro-dibenzo[a,d]-cyclohepten-5-on (14n)

Zur Herstellung der Verbindung 14 werden 0,72 g (1,8 mmol) 2-Chlor-7-[3-(4-Hydroxypiperidin-4-yl-propoxy)]--10,11-dihydrodbenzo[a,d]-cyclohepten-5-one, 0,25 g (1,9 mmol) 2, 4-Difluoranilin, 0,05 g (0,22 mmol) Pd(OAc)₂, 0,10 g (0,21 mmol) 2-(Dicyclohexylphosphino)-2'-,4'-, 6'-triisopropyl-biphenyl, 0,70g (6,2 mmol) KOt-Bu, 5 ml Toluol und 1 ml t-BuOH verwendet.

### Beispiel 105

### 3-(2-Amino-4-fluorphenylamino)-8-amino-6H-dibenzo[b,e]oxepin-11-on (58)

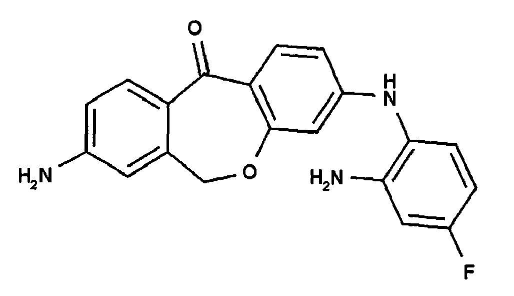

0,60 g (2,66 mmol) Zinn(II)chlorid-Dihydrat werden in 10 ml EtOH gelöst und mit 0,10 g (0,89 mmol) der 8-Nitroverbindung versetzt und gemäß Methode D umgesetzt.
C₂₀H₁₆FN₃O₂ (Mᵣ = 349.37)

| | |
|---|---|
| Ausbeute | 0,01 g (11,9 %) |
| Schmelzpunkt | 194,5°C |

¹H-NMR (MeOH-d₄) δ in ppm: 8.08 (d, 1 H, J = 8.97 Hz, aryl H), 7.77 (d, 1 H, J = 8.96 Hz, aryl H), 7.07-6.96 (m, 1 H, aryl H), 6.69-6.38 (m, 5 H, aryl H), 6.07 (s, 1 H, aryl H), 4.97 (s, 2H, -CH₂-O)
¹³C-NMR (MeOH-d₄) δ in ppm: 186.68 (C¹¹), 163.57 (C^{4a}), 155.84 (C^{4'}), J = 217.20 Hz), 153.68 (C⁸), 149.21 (C³), 139.15 (C^{6a}), 133.41 (C¹), 131.81 (C¹⁰), 130.23 (C^{1'}), 128.66 (C^{6'}, J = 11.6 Hz), 116.88 (C11^{a}), 115.34 (C⁹), 113.61 (C⁷), 109.27 (C²), 105.34 (C^{5'},J = 24.45 Hz), 103.03 (C^{3'}, J= 23.18 Hz), 101.15 (C⁴), 73.67 (C⁶)

| | |
|---|---|
| IR (ATR) (cm⁻¹): | 1602, 1579, 1551, 1505, 1293, 1265, 1233, 1161,1113, 831, 759 |
| LC | 7,34 min |
| MS (ESI) | 350.3 [M+H]⁺ |

### Beispiel 106

### Morpholin-4-carbonsäure[3-(2,4-difluorphenylamino)-1-oxo-6,11-dihydro-dibenzo[b,e]oxepin-8-yl]amid

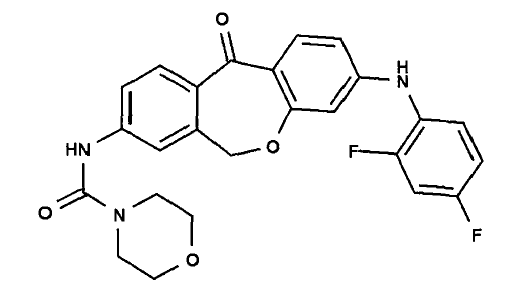

Zu einer Lösung aus 0,05 g (0,14 mmol) 3-(2,4-difluorphenylamino)-8-amino-6H-dibenzo[b,e]oxepin-11-on und 0,03 g (0,30 mmol) Triethylamin (frisch destilliert) in 3 ml Tetrahydrofuran wird tropfenweise 0,025 g (0,16 mmol) Morpholin-4-carbonsäurechlorid unter Eiskühlung langsam hinzugegeben. Nach beendeter Zugabe wird die Ansatzmischung bei RT 2 h unter Argon-Atmosphäre gerührt. Am Ende der Reaktion wird überschüssiges Lösungsmittel einrotiert, der Rückstand in EtOAc aufgenommen und gegen 5%-ige NaHCO₃-Lösung ausgeschüttelt. Die organische Phase wird einrotiert, das Rohprodukt wird in MeOH/H₂O umkristallisiert und anschließend mit Diethylether gewaschen und getrocknet.
C₂₅H₂₁F₂NsO₄ (Mᵣ = 465.46)

| | |
|---|---|
| Ausbeute | 0,03 g (46,0 %) |
| Schmelzpunkt | 244,3 °C |

¹H-NMR (DMSO-d₆) δ in ppm: 7.99 (d, 1 H, J = 8.69 Hz, aryl H), 7.70 (d, 1 H, J = 2.83 Hz, aryl H), 7.39-7.34 (m, 2 H, aryl H), 7.12-7.08 (m, 1 H, aryl H), 6.70-6.57 (m, 3 H, aryl H), 6.24 (s, 1 H, aryl H), 5.05 (s, 2 H, -CH₂-O)

| | |
|---|---|
| IR (ATR) (cm⁻¹): | 1556,1512,1309,1229,1097,911,872,833,694 |
| LC | 7,76 min |
| MS (ESI) | 464.1 [M-H]⁻ |

### Verbindungen Position 8

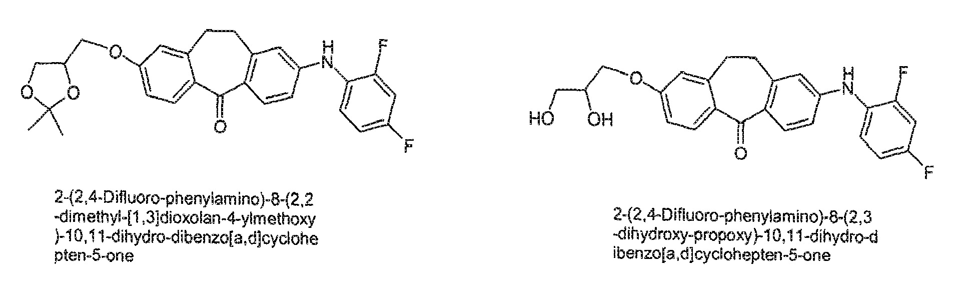

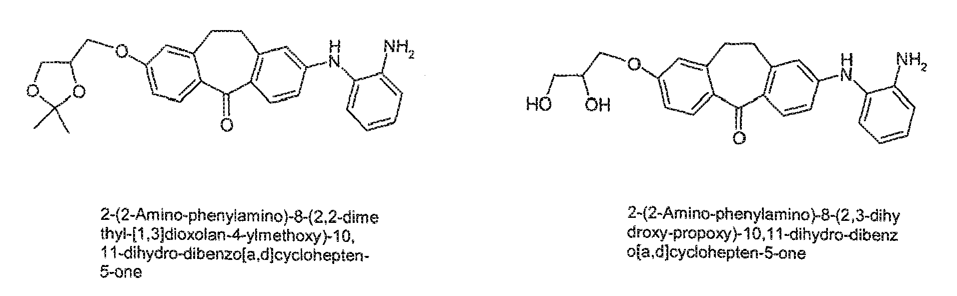

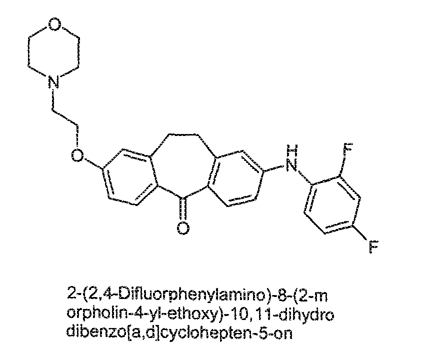

### Verbindungen Position 9

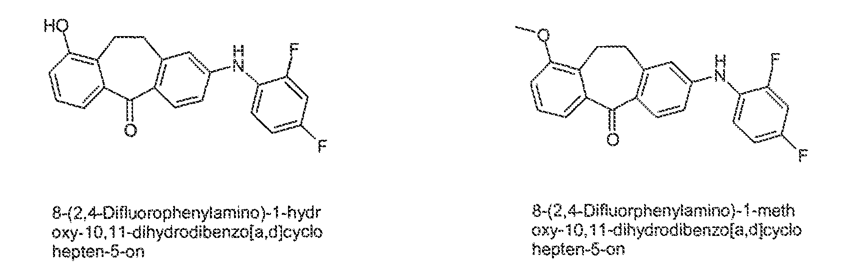

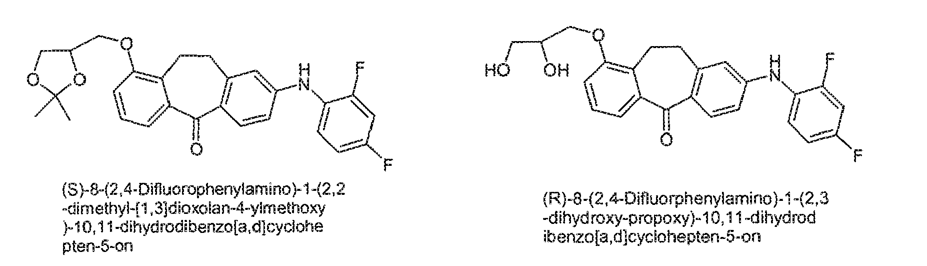

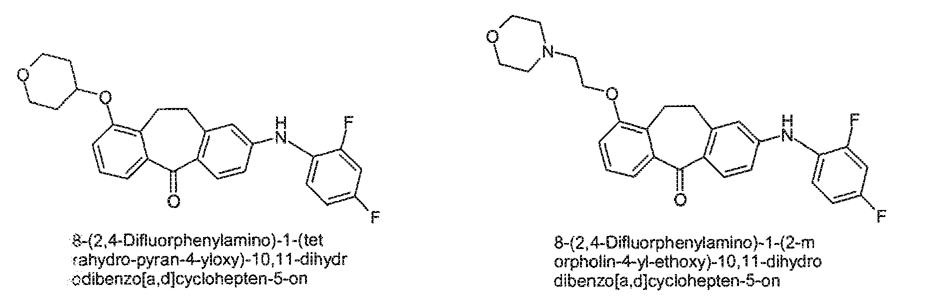

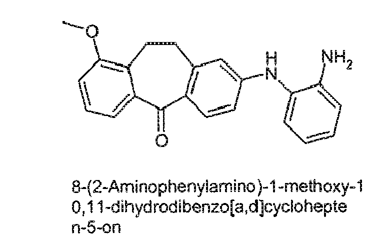

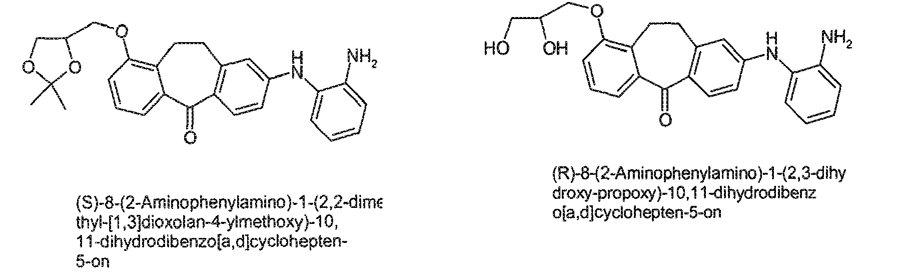

## Patentansprüche

1. Verbindungen der Formel 1 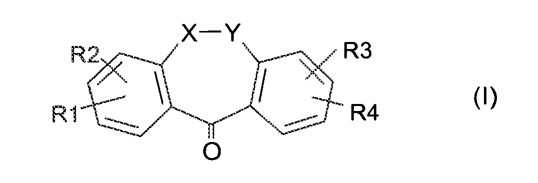 worin
eines der Ringatome X und Y für CH₂ und das andere für O, S, SO, SO₂ oder NR5 steht; oder -X-Y- für -CH₂-CH₂- oder -CH=CH- steht;
R1 ausgewählt ist unter:
a) Hydroxy;
b) C₁-C₆-Alkoxy;
c) C₁-C₆-Alkoxy, das mit 1, 2 oder 3 Hydroxy- oder C₁-C₆-Alkoxygruppen substituiert ist;
d) NR6R7;
e) C₁-C₆-Alkoxy, das substituiert ist mit einem gesättigten oder ungesättigten, aromatischen oder nicht-aromatischen Heterocyclus mit 5 oder 6 Ringatomen, der 1, 2 oder 3 Heteroatome aufweist, die unabhängig voneinander ausgewählt sind unter O, N und S, wobei der Heterocyclus gegebenenfalls 1 oder 2 Hydroxy-, C₁-C₆-Alkoxy- oder C₁-C₆-Alkylsubstituenten aufweisen und mit einem Phenylring oder einem gesättigten oder ungesättigtenCarbocyclus mit 5 oder 6 Ringatomen kondensiert sein kann; und
f) Tetrazolo;
g) NR8CONR13R14;
g) CF₃SO₂O-;
h) OR16;
i) C₁-C₆-Alkylcarbonyloxy-C₁-C₆-alkoxy;
j) NO₂;
R2 für H oder C₁-C₆-Alkyl steht;
R3 ausgewählt ist unter:
a) -NR6R7;
b) 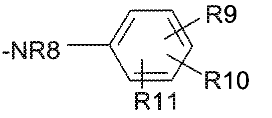
c) 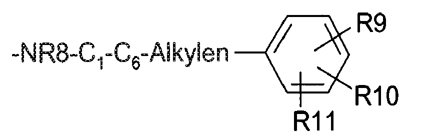
d) 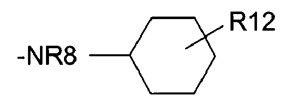
e) 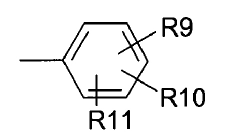
f) -NH-C₁-C₆-Alkylen-NR6R7
g) Tetrazolo;
h) Halogen;
i) OR15; und
j) NO₂; und
k) NR8COC₁-C₆-Alkyl;
R4 für H, Halogen oder C₁-C₆-Alkyl steht;
R5 für H oder C₁-C₆-Alkyl, das mit 1, 2 oder 3 Hydroxy- oder C₁-C₆-Alkoxygruppen substituiert ist, steht;
R6 und R7, die gleich oder verschieden sein können, für H oder C₁-C₆-Alkyl, das mit 1, 2 oder 3 Hydroxy- oder C₁-C₆-Alkoxygruppen substituiert ist, stehen;
R8 für H oder C₁-C₆-Alkyl steht;
R9, R10 und R11, die gleich oder verschieden sein können, ausgewählt sind unter:
j) H,
k) NH₂,
l) mono-C₁-C₆-Alkylamino,
m) di-C₁-C₆-Alkylamino,
n) C₁-C₆-Alkyl,
o) C₁-C₆-Alkoxy,
p) Hydroxy,
q) Halogen,
r) C₁-C₆-Alkyl, das mit 1, 2 oder 3 Halogenatomen substituiert ist;
j) CONR6R7; und
k) NO₂;
R12 für H oder NH₂ steht;
R13 und R14, die gleich oder verschieden sein können, für H oder C₁-C₆-Alkyl stehen oder zusammen mit dem Stickstoffatom an das sie gebunden sind, für einen nicht-aromatischen Heterocyclus mit 5 oder 6 Ringatomen, der 1 oder 2 Heteroatome aufweist, die unabhängig voneinander ausgewählt sind unter O und N;
R15 für H oder C₁-C₆-Alkyl, das mit 1, 2 oder 3 Hydroxy- oder C₁-C₆-Alkoxygruppen substituiert ist, steht;
R16 für einen nicht-aromatischen Heterocyclus mit 5 oder 6 Ringatomen, der 1 oder 2 Heteroatome aufweist, die unabhängig voneinander ausgewählt sind unter O und N;
und die optischen Isomeren, physiologisch verträglichen Salze und Solvate davon.

2. Verbindungen nach Anspruch 1 der Formel la 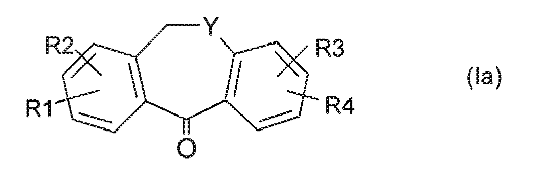 worin Y für O steht und R1, R2, R3 und R4 die in Anspruch 1 angegebenen Bedeutungen besitzen.

3. Verbindungen nach Anspruch 1 der Formel Ib: 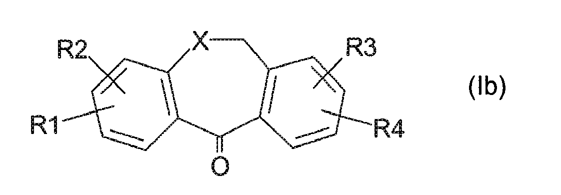 worin X für O steht und R1, R2, R3 und R4 die in Anspruch 1 angegebenen Bedeutungen besitzen.

4. Verbindungen nach Anspruch 1 der Formel Ic: 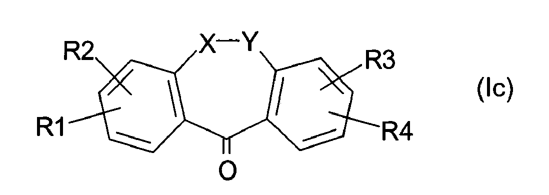 worin -X-Y- für -CH₂-CH₂- oder -CH=CH- steht und R1, R2, R3 und R4 die in Anspruch 1 angegebenen Bedeutungen besitzen.

5. Verbindungen nach einem der vorhergehenden Ansprüche, wobei R1 ausgewählt ist unter NR6R7, Hydroxy, C₁-C₆-Alkoxy, C₁-C₆-Alkyl, das mit 1, 2 oder 3 Hydroxygruppen substituiert ist, und einem gesättigten nicht-aromatischen Heterocyclus mit 5 oder 6 Ringatomen, der 1 oder 2 Heteroatome aufweist, die unabhängig voneinander ausgewählt sind unter O und N, und der gegebenenfalls 1 oder 2 C₁-C₆-Alkylsubstituenten aufweisen kann.

6. Verbindungen nach einem der vorhergehenden Ansprüche, wobei R3 ausgewählt ist unter
b) 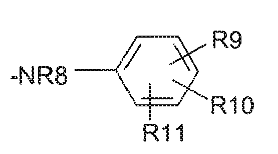
e) Phenyl und
g) Tetrazolo.

7. Verbindungen nach Anspruch 6, wobei R9 und R10 unabhängig voneinander ausgewählt sind unter H, NH2, mono-C₁-C₆-Alkylamino, di-C₁-C₆-Alkylamino, Halogen, CF₃, C₁-C₆-Alkyl und C₁-C₆-Alkoxy und R11 für H oder Halogen steht.

8. Verbindungen nach Anspruch 6 oder 7, wobei R3 für einen Rest der Formel 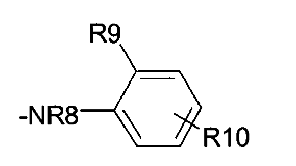 steht.

9. Verbindungen nach einem der vorhergehenden Ansprüche, wobei R4 für H steht.

10. Verbindungen nach einem der vorhergehenden Ansprüche, wobei R6 und R7 für H stehen.

11. Verbindungen nach einem der vorhergehenden Ansprüche der Formel

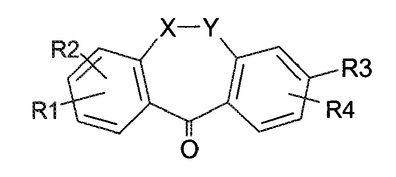

12. Verbindungen nach Anspruch 2 der Formel laa 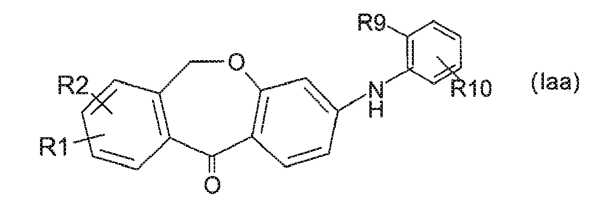 worin R1, R2, R9 und R10 die in Anspruch 1 oder 7 angegebenen Bedeutungen besitzen.

13. Verbindungen nach Anspruch 4 der Formel lca: 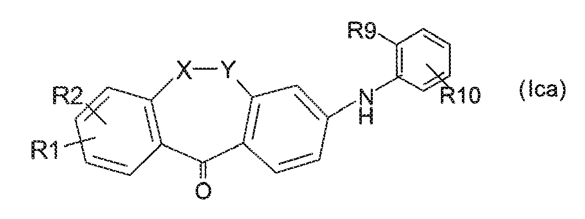 worin
-X-Y- für -CH₂-CH₂- oder -CH=CH- steht und R1, R2, R9 und R10 die in Anspruch 1 oder 7 angegebenen Bedeutungen besitzen.

14. Verbindungen nach Anspruch 1:
(1) 2-Nitro-7-methoxydibenzosuberenon
(2) 2-Chlor-7-methoxysuberenon
(3) 2-Acetamido-7-methoxydibenzosuberon
(4) 2-Chlor-7-methoxydibenzodibenzosuberon
(5) 2-Amino-7-methoxydibenzosuberon
(6) 2-Chlor-7-hydroxydibenzosuberon
(7) 2-Chlor-7-(S-1,2-isopropylidenglycer-3-yl)-10,11-dihydro-dibenzo[a,d]cyclohepten-5-on
(8) 2-Chlor-7-(R-1,2-isopropylidenglycer-3-yl)-10,11-dihydro-dibenzo[a,d]cyclohepten-5-on
(9) 2-Chlor-7-(3-acetoxypropoxy)-10,11-dihydro-dibenzo[a,d]-cyclohepten-5-on
(10) 2-Chlor-7-(3-acetoxyethoxy)-10,11-dihydro-dibenzo[a,d]cyclohepten-5-on
(11) 2-Chloro-7-(2-morpholin-4-yl-ethoxy)-10,11-dihydro-dibenzo[a,d]-cyclohepten-5-on
(12) 2-Chloro-7-(2-tetrahydropyran-4-yl-oxy)-10,11-dihydro-dibenzo[a,d]-cyclohepten-5-on
(13) 2-(2-Aminoanilino)-7-methoxydibenzosuberon
(14) 2-(2-Amino-4-fluoranilino)-7-methoxydibenzosuberon
(15) 2-(2,4-Difluoranilino)-7-methoxydibenzosuberon
(16) 2-(2-Chlor-4-fluoranilino)-7-methoxydibenzosuberon
(17) 2-(2,4,5-Trifluoranilino)-7-methoxydibenzosuberon
(18) 2-(2-Trifluormethylanilino)-7-methoxydibenzosuberon
(19) 2-(Anilino)-7-methoxydibenzosuberon
(20) 2-(2-Methoxyanilino)-7-methoxydibenzosuberon
(21) 2-(3-Methyl-4-fluoranilino)-7-methoxydibenzosuberon
(22) 2-(2-Amino-4-trifluormethylanilino)-7-methoxydibenzosuberon
(23) 2-(Phenyl)-7-methoxydibenzosuberon
(24) 2-(2,4-Difluoranilino)-7-methoxydibenzosuberenon
(25) 2-(2,4-Difluoranilino)-7-(S-1,2-isopropylidenglycer-3-yl)-10,11-dihydro-dibenzo[a,d]-cyclohepten-5-on
(26) 2-(2,4-Difluoranilino)-7-(R-1,2-isopropylidenglycer-3-yl)-10,11-dihydro-dibenzo[a,d]-cyclohepten-5-on
(27) 2-(2-Aminoanilino)-7-(S-1,2-isopropylidenglycer-3-yl)-10,11-dihydro-dibenzo[a,d]-cyclohepten-5-on
(28) 2-(2-Aminoanilino)-7-(R-1,2-isopropylidenglycer-3-yl)-10,11-dihydro-dibenzo[a,d]-cyclohepten-5-on
(29) 2-(2,4-Difluoranilino)-7-[2R-,3-dihydroxypropoxy]-10,11-dihydro-dibenzo[a,d]-cyclohepten-5-on
(30) 2-(2,4-Difluoranilino)-7-[2S-,3-dihydroxypropoxy]-10,11-dihydro-dibenzo[a,d]-cyclohepten-5-on
(31) 2-(2-Aminoanilino-7-[2R-,3-dihydroxypropoxy]-10,11-dihydro-dibenzo[a,d]-cyclohepten-5-on
(32) 2-(2-Aminoanilino-7-[2S-,3-dihydroxypropoxy]-10,11-dihydro-dibenzo[a,d]-cyclohepten-5-on
(33) 2-(2,4-Difluoranilino)-7-(2-hydroxy-ethoxy)-10,11-dihydro-dibenzo[a,d]-cyclohepten-5-on
(34) 2-(2,4-Difluoranilino)-7-(3-hydroxy-propoxy)-10,11-dihydro-dibenzo[a,d]-cyclohepten-5-on
(35) 2-(2,4-Difluoranilino)-7-(2-morpholin-4-yl-ethoxy)-10,11-dihydro-dibenzo[a,d]-cyclohepten-5-on
(36) 2-(2-Aminoanilino)-7-(2-morpholin-4-yl-ethoxy)-10,11-dihydro-dibenzo[a,d]-cyclohepten-5-on
(37) 2-(2,4-Difluoranilino)-7-(2-tetrahydropyran-4-yl-oxy)-10,11-dihydro-dibenzo[a,d]-cyclohepten-5-on
(38) 2-Chlor-8-methoxy-10,11-dihydrodibenzo[a,d]cyclohepten-5-on
(39) 2-Chlor-8-hydroxy-10,11-dihydrodibenzo[a,d]cyclohepten-5-on
(40) (S)-2-Chlor-8-(2,2-dimethyl-[1,3]dioxolan-4-ylmethoxy)-10,11-dihydrodibenzo[a,d]cyclohepten-5-on
(41) (S)-2-(2,4-Difluorophenylamino)-8-(2,2-dimethyl-[1,3]dioxolan-4-ylmethoxy)-10,11-dihydro-dibenzo[a,d]cyclohepten-5-on
(42) (R)-2-(2,4-Difluorophenylamino)-8-(2,3-dihydroxypropoxy)-10,11-dihydrodibenzo[a,d]cyclohepten-5-on
(43) (S)-2-(2-Aminophenylamino)-8-(2,2-dimethyl-[1,3]dioxolan-4-ylmethoxy)-10,11-dihydrodibenzo[a,d]cyclohepten-5-on
(44) (R)-2-(2-Aminophenylamino)-8-(2,3-dihydroxypropoxy)-10,11-dihydrodibenzo[a,d]cyclohepten-5-on
(45) 2-Chlor-8-(2-morpholin-4-ylethoxy)-10,11-dihydrodibenzo[a,d]cyclohepten-5-on
(46) 2-(2,4-Difluorphenylamino)-8-(2-morpholin-4-yl-ethoxy)-10,11-dihydrodibenzo[a,d]cyclohepten-5-on
(47) 8-Chlor-1-methoxy-10,11-dihydrodibenzo[a,d]cyclohepten-5-on
(48) 8-Chlor-1-hydroxy-10,11-dihydrodibenzo[a,d]cyclohepten-5-on
(49) 8-(2,4-Difluorphenylamino)-1-hydroxy-10,11-dihydro-dibenzo[a,d]cyclohepten-5-on
(50) 8-(2,4-Difluorphenylamino)-1-methoxy-10,11-dihydrodibenzo[a,d]cyclohepten-5-on
(51) 8-(2-Aminophenylamino)-1-methoxy-10,11-dihydrodibenzo[a,d]cyclohepten-5-on
(52) (S)-8-Chlor-1-(2,2-dimethyl-[1,3]dioxolan-4-ylmethoxy)-10,11-dihydrodibenzo-[a,d]cyclohepten-5-on
(53) (S)-8-(2,4-Difluorphenylamino)-1-(2,2-dimethyl-[1,3]dioxolan-4-ylmethoxy)-10,11-dihydrodibenzo[a,d]cyclohepten-5-on
(54) (R)-8-(2,4-Difluorphenylamino)-1-(2,3-dihydroxypropoxy)-10,11-dihydrodibenzo-[a,d]cyclohepten-5-on
(55) (S)-8-(2-Aminophenylamino)-1-(2,2-dimethyl-[1,3]dioxolan-4-ylmethoxy)-10,11-dihydrodibenzo[a,d]cyclohepten-5-on
(56) (R)-8-(2-Aminophenylamino)-1-(2,3-dihydroxypropoxy)-10,11-dihydrodibenzo-[a,d]cyclo-hepten-5-on
(57) 8-Chlor-1-(tetrahydropyran-4-yloxy)-10,11-dihydrodibenzo[a,d]cyclohepten-5-on
(58) 8-(2,4-Difluorphenylamino)-1-(tetrahydropyran-4-yloxy)-10,11-dihydrodibenzo[a,d]cyclohepten-5-on
(59) 8-Chlor-1-(2-morpholin-4-yl-ethoxy)-10,11-dihydrodibenzo[a,d]cyclohepten-5-on
(60) 8-(2,4-Difluorphenylamino)-1-(2-morpholin-4-yl-ethoxy)-10,11-dihydrodibenzo-[a,d]cyclo-hepten-5-on
(61) Trifluormethansulfonsäure-8-chlor-5-oxo-10,11-dihydro-5H-dibenzo[a,d]cyclohepten-1-ylester
(62) 3-Acetamido-8-nitro-6H-dibenzo[b,e]oxepin-11-on
(63) 3-Amino-8-nitro-6H-dibenzo[b,e]oxepin-11-on
(64) 3-(2,4-Difluorphenylamino)-8-nitro-6H-dibenzo[b,e]oxepin-11-on
(65) 3-(2,4-Difluorphenylamino)-8-amino-6H-dibenzo[b,e]oxepin-11-on
(66) 3-(2-Nitrophenylamino)-8-nitro-6H-dibenzo[b,e]oxepin-11-on
(67) 3-(2-Aminophenylamino)-8-amino-6H-dibenzo[b,e]oxepin-11-on
(68) 3-(4-Fluor-2-nitrophenylamino)-8-nitro-6H-dibenzo[b,e]oxepin-11-on
(69) 3-(2-Amino-4-fluorphenylamino)-8-nitro-6H-dibenzo[b,e]oxepin-11-on
(70) 3-(2-Methoxyphenylamino)-8-nitro-6H-dibenzo[b,e]oxepin-11-on
(71) 8-Amino-3-(2-methoxyphenylamino)-6H-dibenzo□b,e□oxepin-11-on
(72) 8-Nitro-3-(4-fluor-2-methoxyphenylamino)-6H-dibenzo[b,e]oxepin-11-on
(73) 8-Amino-3-(4-fluor-2-methoxyphenylamino)-6H-dibenzo□b,e□-oxepin-11-on
(74) 8-Nitro-3-(2-nitro-4-trifluormethylphenylamino)-6H-dibenzo[b,e]oxepin-11-on
(75) 8-Amino-3-(2-amino-4-trifluormethylphenylamino)-6H-dibenzo[b,e]oxepin-11-on
(76) 3-(Tetrazol-1-yl)-8-nitro-6H-dibenzo[b,e]oxepin-11-on
(77) 8-Amino-3-(tetrazol-1-yl)-6H-dibenzo[b,e]oxepin-11-on
(78) 3-(2,4-Difluorphenylamino)-8-tetrazol-1-yl-6H-dibenzo[b,e]oxepin-11-on
(79) 2-(2-Methyl-4-Fluoranilino)-7-methoxydibenzosuberon
(80) 2-(2-Chloranilino)-7-methoxydibenzosuberon
(81) 2-(2-Amino-4-fluoranilino)-7-hydroxy-10,11-dihydro-dibenzo[a,d]-cyclohepten-5-on
(82) 2-(2, 4-Difluoranilino)-7-hydroxy-10,11-dihydro-dibenzo[a,d]-cyclohepten-5-on
(83) 2-(2-Chlor-4-fluoranilino)-7-hydroxy-10,11-dihydro-dibenzo[a,d]-cyclohepten-5-on
(84) 2-(2-Chloranilino)-7-hydroxy-10,11-dihydro-dibenzo[a,d]-cyclohepten-5-on
(85) 2-(Anilino)-7-hydroxy-10,11-dihydro-dibenzo[a,d]-cyclohepten-5-on
(86) 2-(2,4-Difluoranilino)-7-hydroxy-dibenzo[a,d]-cyclohepten-5-on
(87) 2-(2,4-Difluoranilino)-7-[3-(4-Hydroxypiperidin-4-yl-propoxy)]-10,11-dihydro-dibenzo[a,d]-cyclohepten-5-on
(88) 3-(2-Amino-4-fluorphenylamino)-8-nitro-6H-dibenzo[b,e]oxepin-11-on
(89) Morpholin-4-carbonsäure[3-(2,4-difluorphenylamino)-1-oxo-6,11-dihydro-dibenzo[b,e]oxepin-8-yl]amid.

15. Pharmazeutisches Mittel, enthaltend wenigstens eine Verbindung der Formel I gemäß einem der Ansprüche 1 bis 13, gegebenenfalls zusammen mit physiologisch verträglichen Exzipienten.

16. Verwendung wenigstens einer Verbindung der Formel I nach einem der Ansprüche 1 bis 14, zur Herstellung eines pharmazeutischen Mittels zur Immunmodulierung und/oder zur Inhibierung der Freisetzung von IL-1β und/oder TNF-α.
